# FASCICULE DE BREVET EUROPEEN

(11) **EP 0 953 334 B1**
(45) Date de publication et mention de la délivrance du brevet: **20.10.2004**
(21) Numéro de dépôt: 99400711.0
(22) Date de dépôt: 23.03.1999
(51) Int. Cl.: A61K 7/13

(54) **Composition de teinture pour fibres kératiniques avec un colorant direct cationique et un polymère substantif**
Zusammensetzung zum Färben von Keratin-Fasern mit einem directen kationischen Färbungsmittel und einem substantiven Polymer
Composition for dying keratinous fibres with a direct cationic dye and a substantive polymer

(30) Priorité: 06.04.1998 FR 9804234
(43) Date de publication de la demande: 03.11.1999
(73) Titulaire: L'OREAL, 75008 Paris (FR)
(72) Inventeur: Rondeau, Christine, 78500 Sartrouville (FR)
(74) Mandataire: Miszputen, Laurent

(56) Documents cités:
- EP-A- 0 132 960
- EP-A- 0 850 638
- WO-A-95/15144
- DE-A- 4 421 031
- DE-U- 29 512 302
- FR-A- 2 282 860

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un polymère substantif cationique ou amphotère particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes, à la fois sur le plan de l'homogénéité de la couleur répartie le long de la fibre (« unisson »), on dit alors que la coloration est trop sélective, que sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations moins sélectives et résistant bien aux diverses agressions que peuvent subir les cheveux, en associant au moins un polymère substantif cationique ou amphotère particulier à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, **(i)**au moins un colorant direct cationique dont la structure répond aux formules suivantes, caractérisée par le fait qu'elle contient en outre **(ii)**au moins un polymère substantif cationique ou amphotère particulier.
**(i)** Le colorant direct cationique utilisable selon la présente invention est un composé choisi parmi ceux de formules (I), (II), (III), (III') suivantes :
   **a) les composés de formule (I) suivante :** 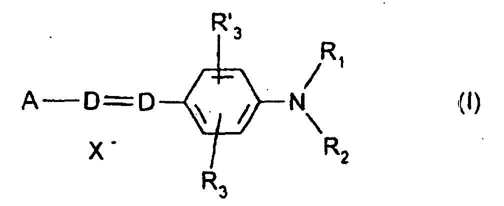 dans laquelle:
      D représente un atome d'azote ou le groupement -CH,
      R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
      X⁻ représente un anion de préférence choisi parmi le chlorure, le méthyl sulfate et l'acétate,
      A représente un groupement choisi par les structures A1 à A18 suivantes : 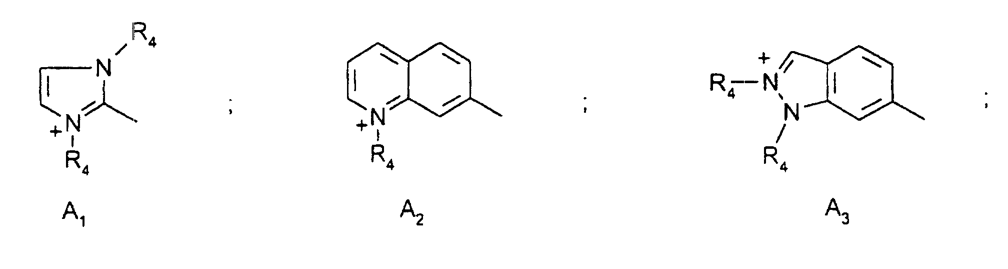 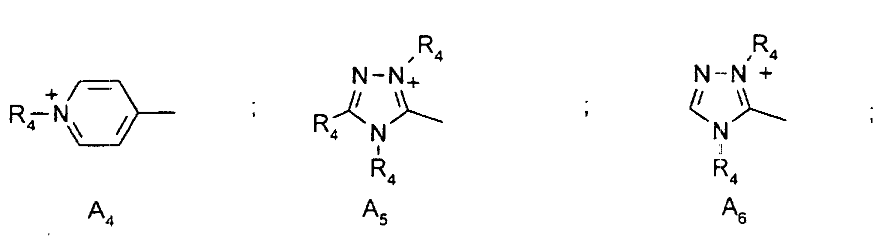 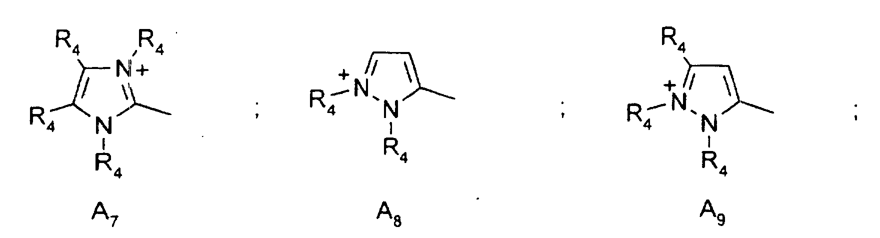 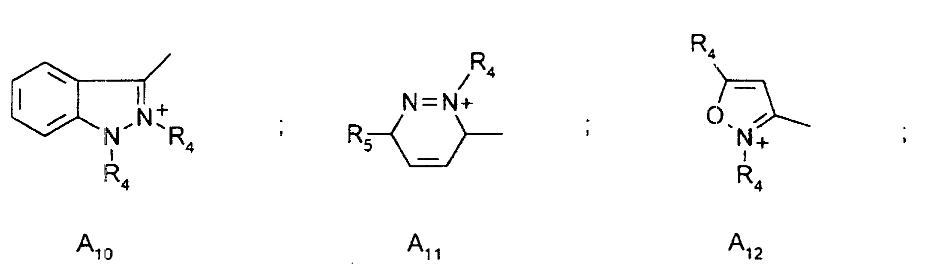 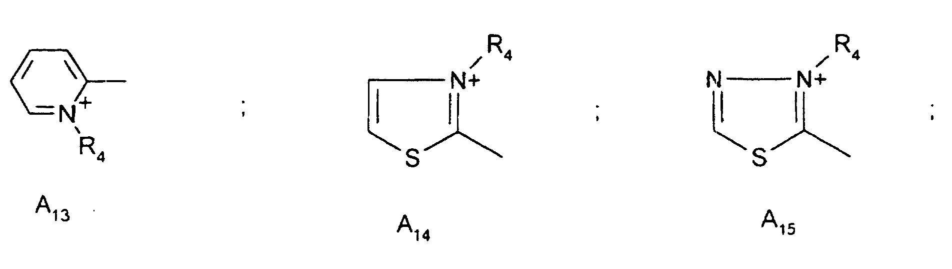 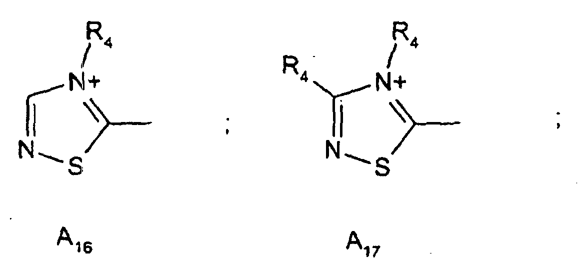 et 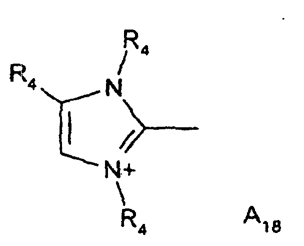 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
         ainsi que les composés suivants de formule (I): 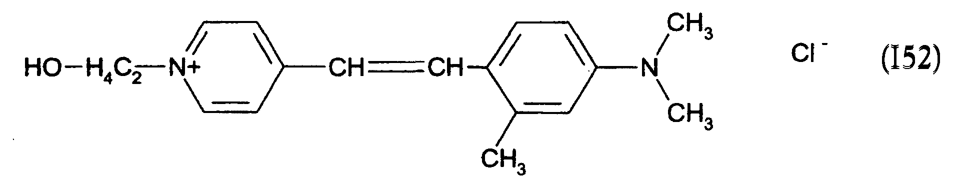 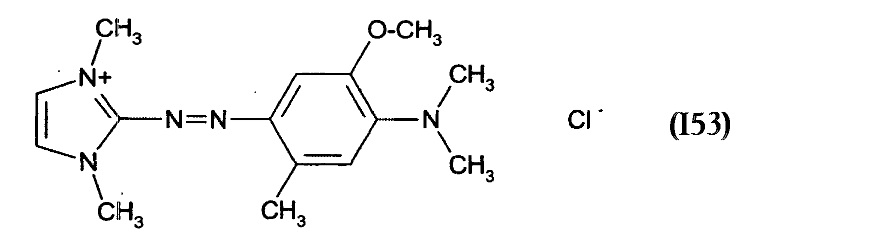 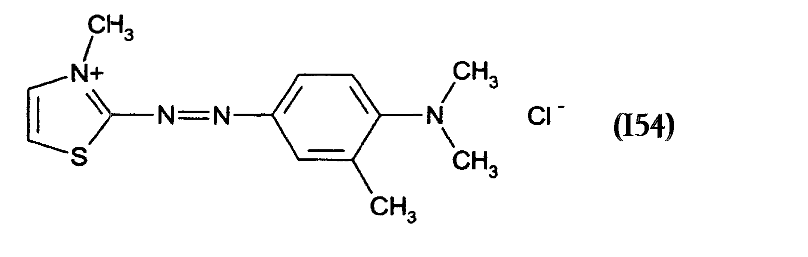
   **b) les composés de formule (II) suivante :** 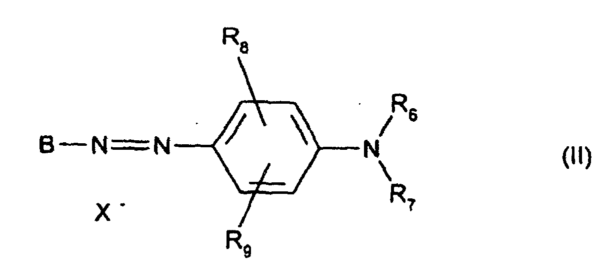 dans laquelle :
      R₆ représente un atome d'hydrogène ou un radical alkyle en C₁-C₄,
      R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
      R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
      X⁻ représente un anion de préférence choisi parmi le chlorure, le méthyl sulfate et l'acétate,
      B représente un groupement choisi par les structures B1 à B6 suivantes : 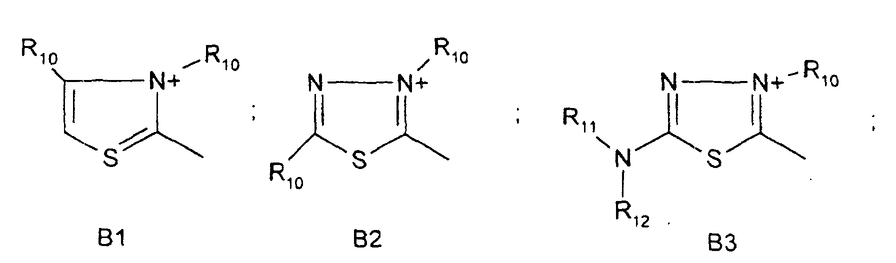 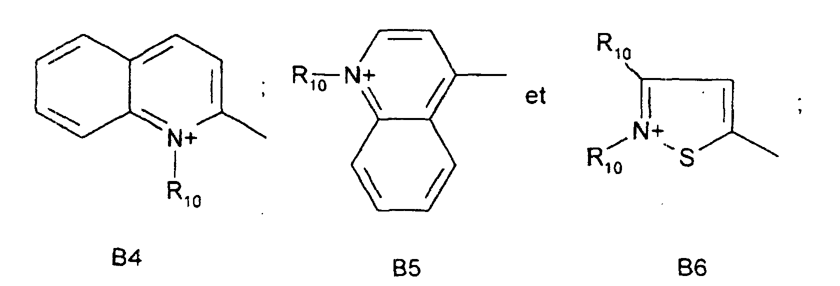 dans lesquelles R₁₀ représente un radical alkyle en C₁-C₄, R₁₁ et R₁₂, identiques ou différents, représentent un atome d'hydrogène ou un radical alkyle en C₁-C₄ ;
   **c) les composés de formules (III) et (III') suivantes :** 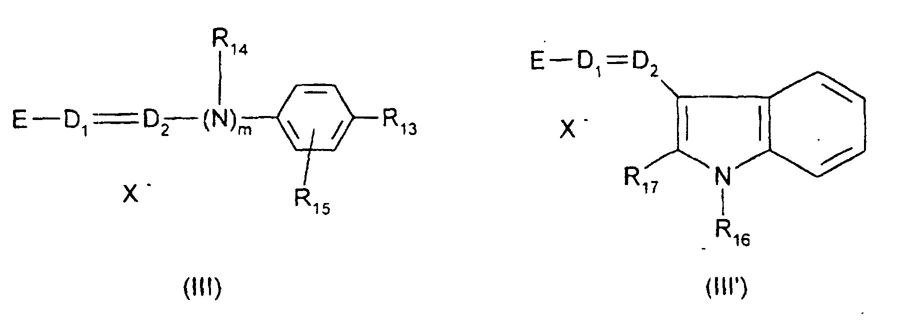 dans lesquelles :
      R₁₃ représente un atome d'hydrogène, un radical alcoxy en C₁-C₄, un atome d'halogène tel que le brome, le chlore, l'iode ou le fluor ou un radical amino,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1,
      étant entendu que lorsque R₁₃ représente un groupement amino non substitué, alors D₁ et D₂ représentent simultanément un groupement -CH et m = 0,
      X⁻ représente un anion de préférence choisi parmi le chlorure, le méthyl sulfate et l'acétate,
      E représente un groupement choisi par les structures E1 à E8 suivantes : 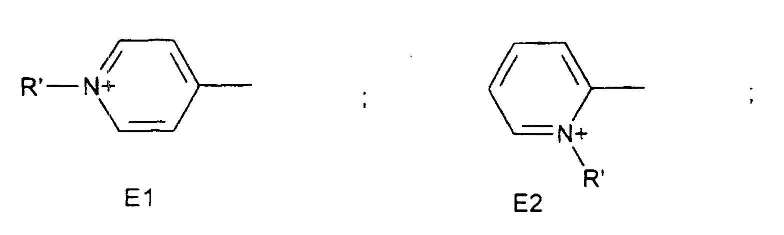 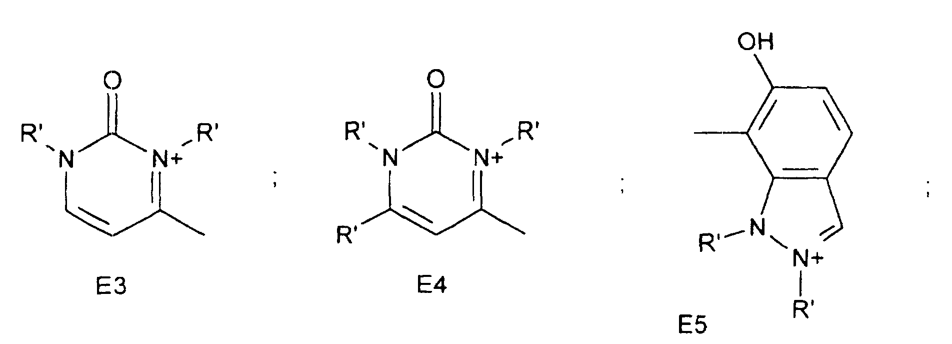 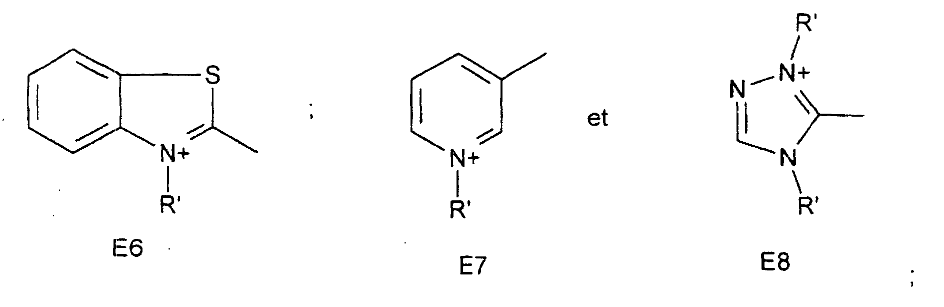 dans lesquelles R' représente un radical alkyle en C₁-C₄ ;
      lorsque m = 0 et que D₁ représente un atome d'azote, alors E peut également désigner un groupement de structure E9 suivante : 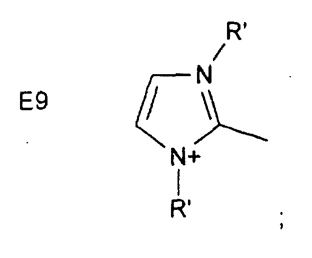 dans laquelle R' représente un radical alkyle en C₁-C₄.

   Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954.
   Parmi les colorants directs cationiques de formule (I) utilisables dans les compositions tinctoriales conformes à l'invention, on peut plus particulièrement citer les composés répondant aux structures (I1) à (I52) suivantes : 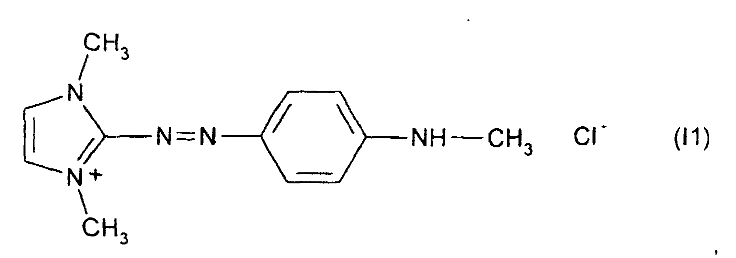 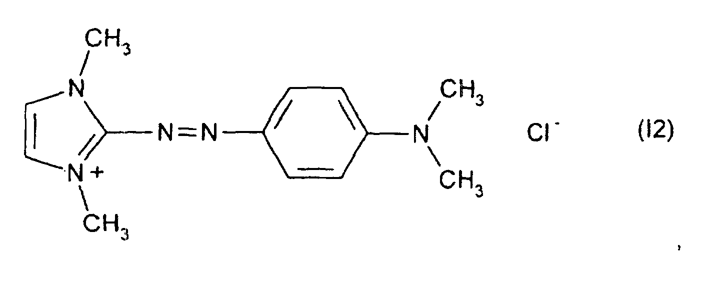 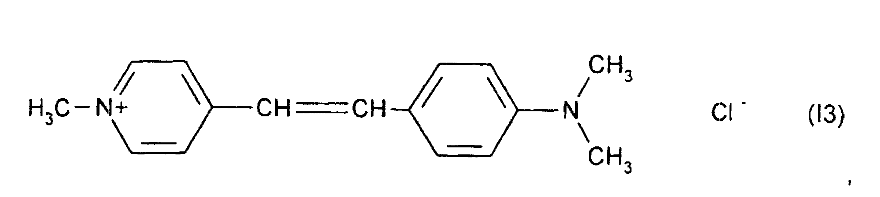 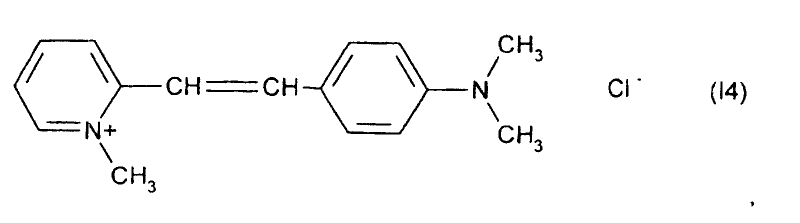 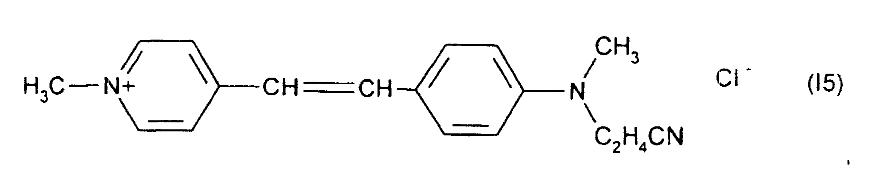 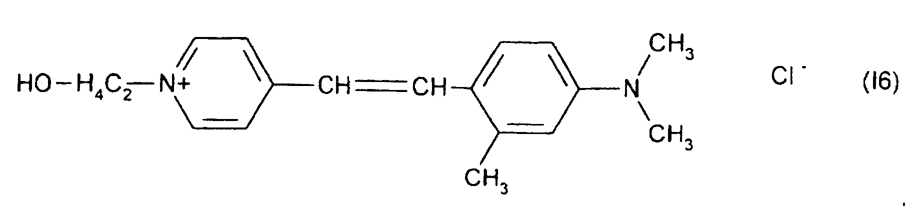 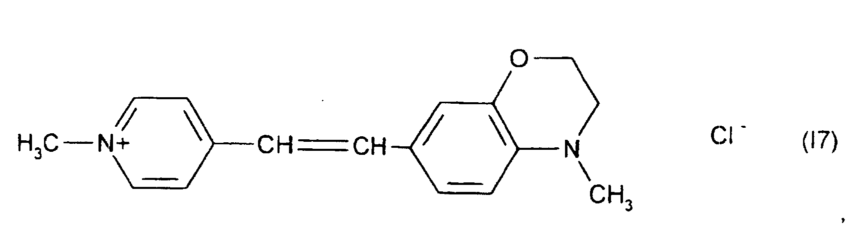 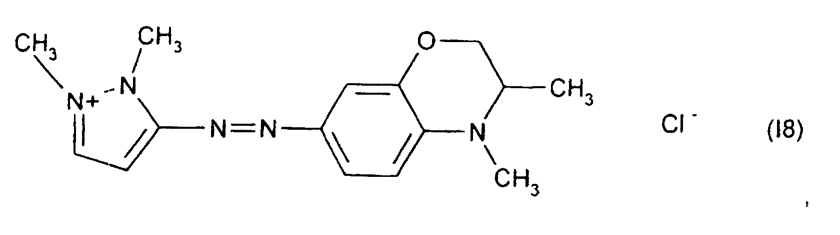 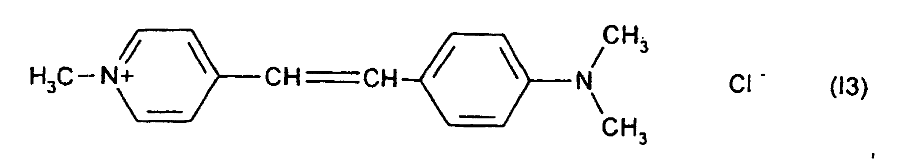 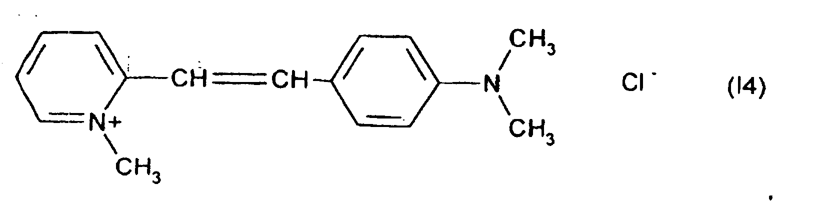 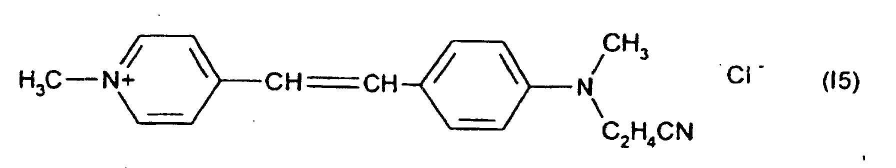 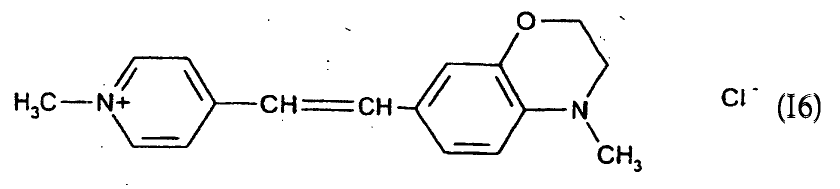 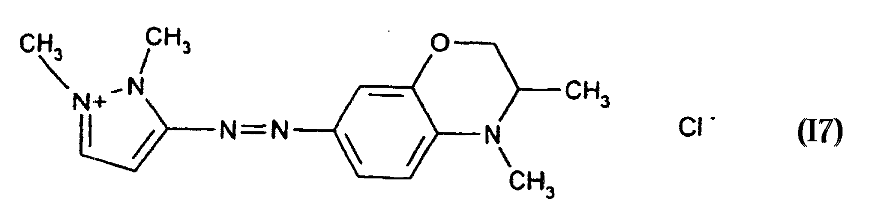 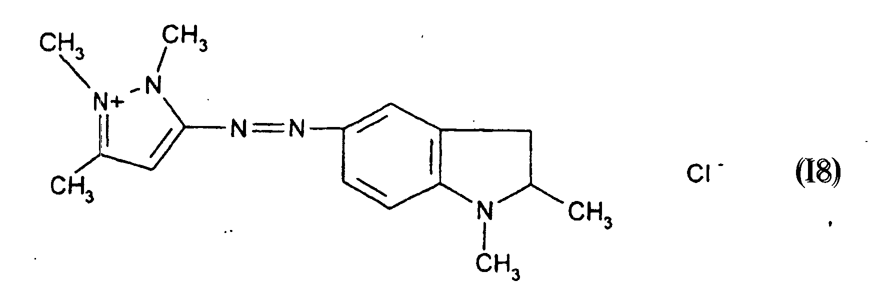 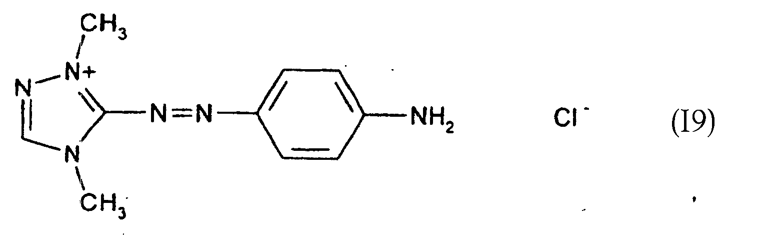 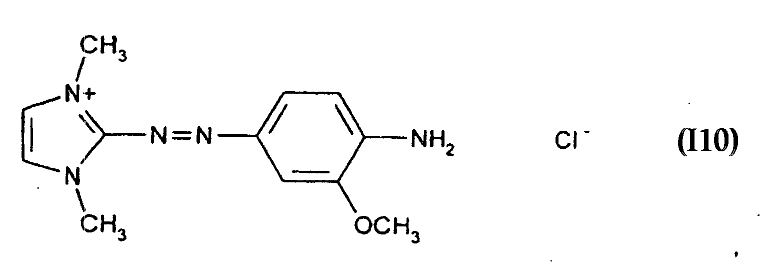 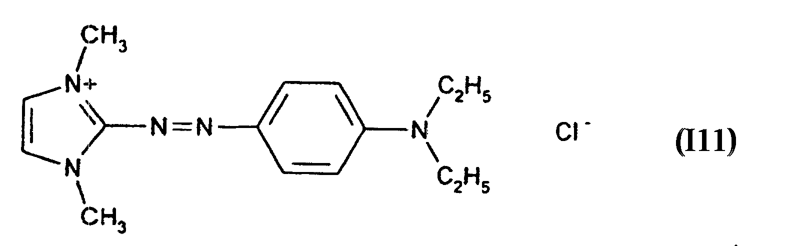 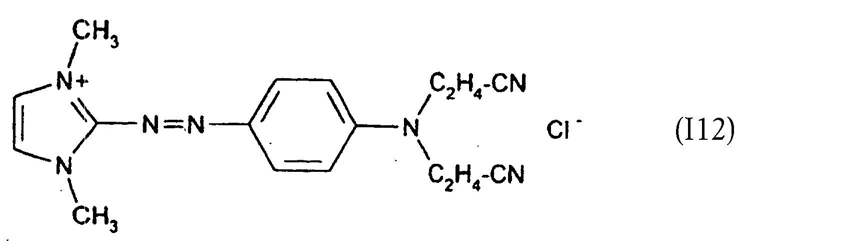 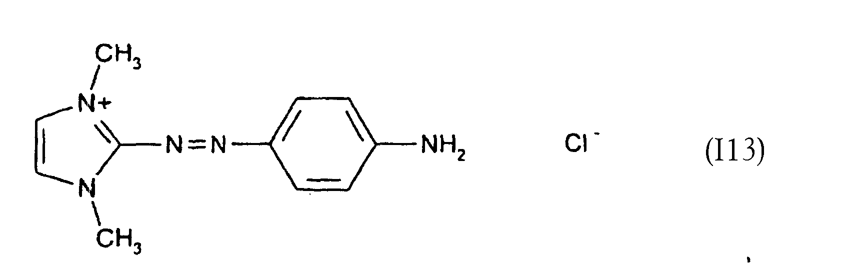 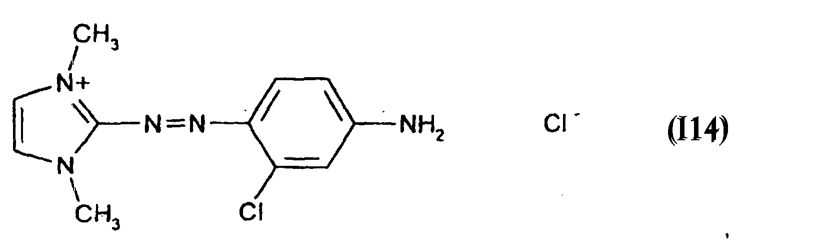 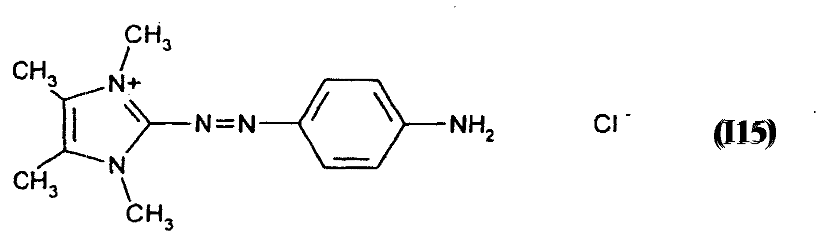 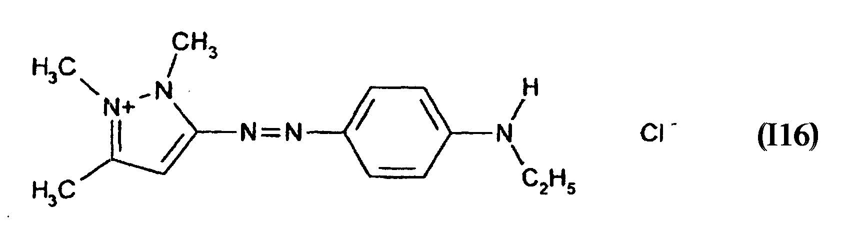 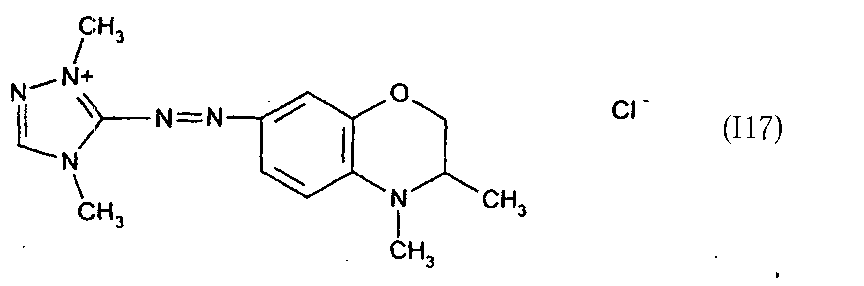 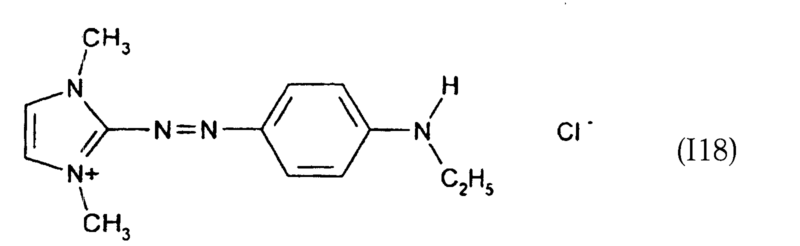 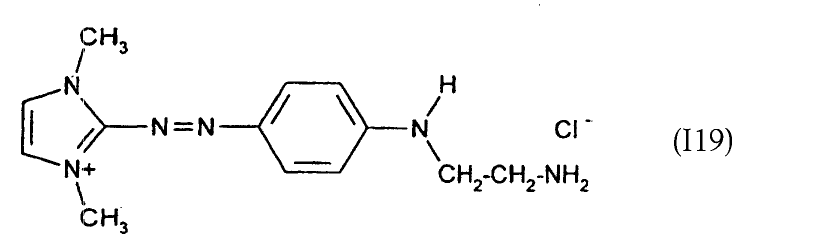 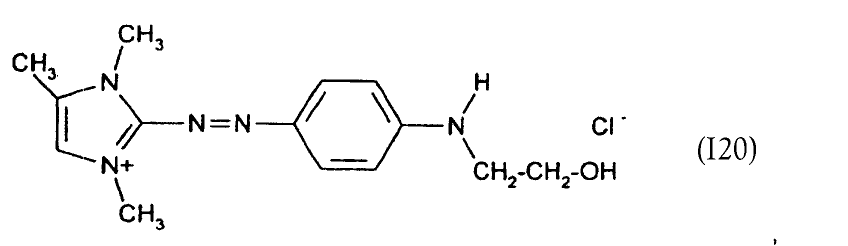 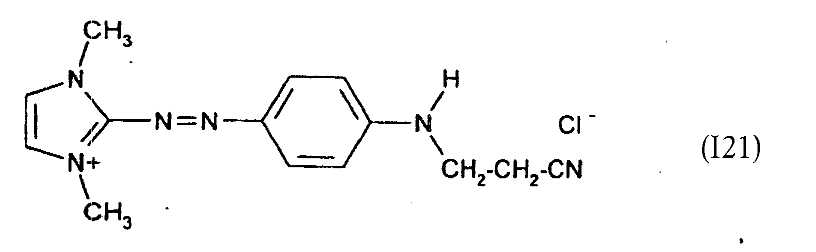 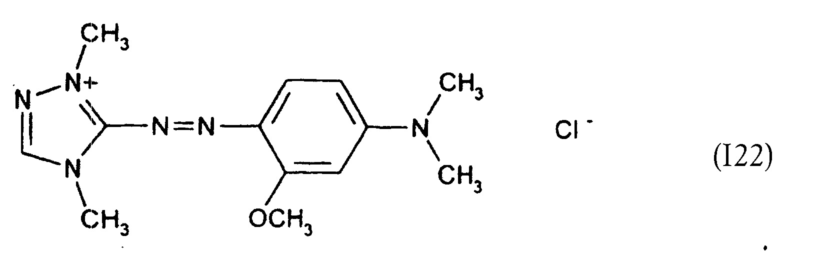 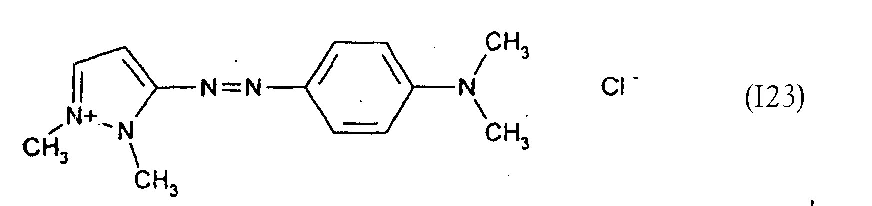 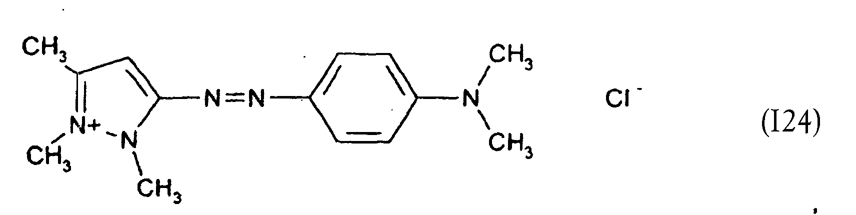 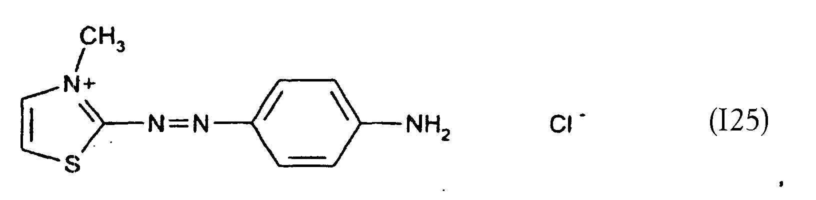 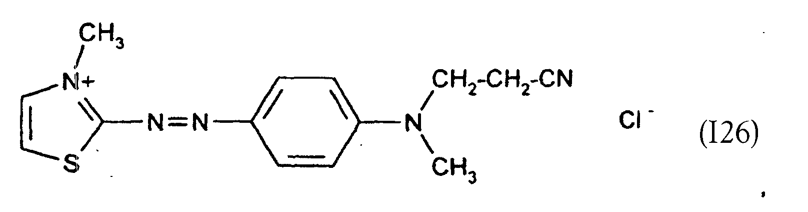 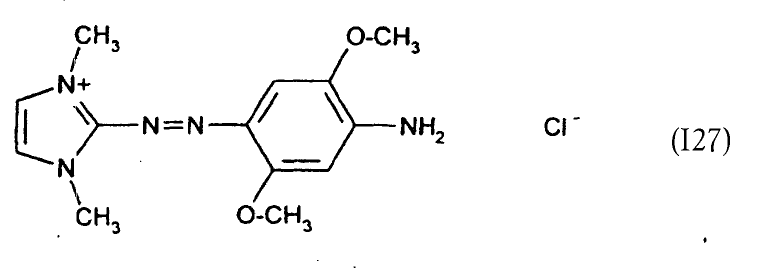 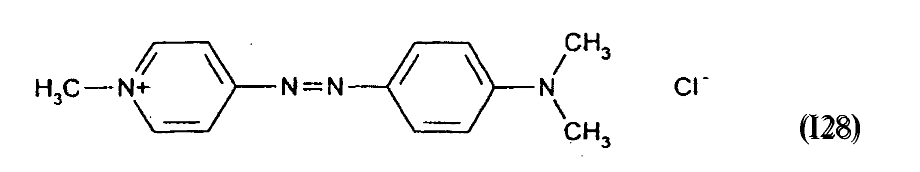 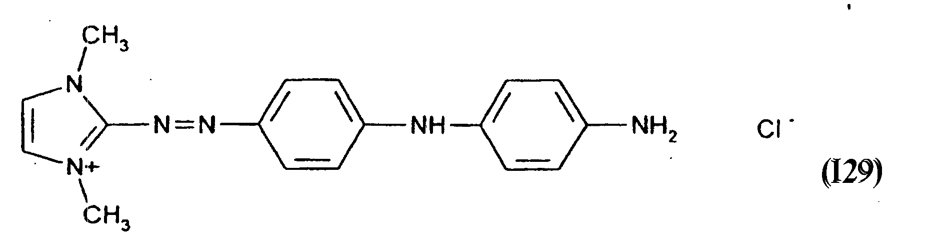 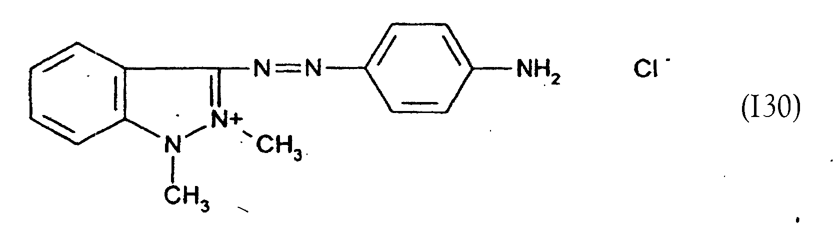 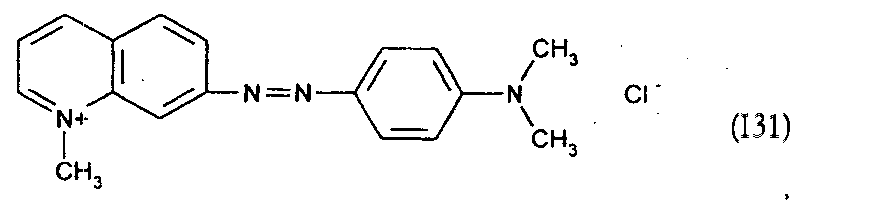 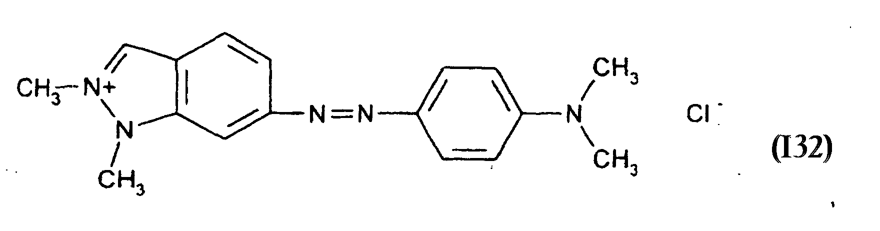 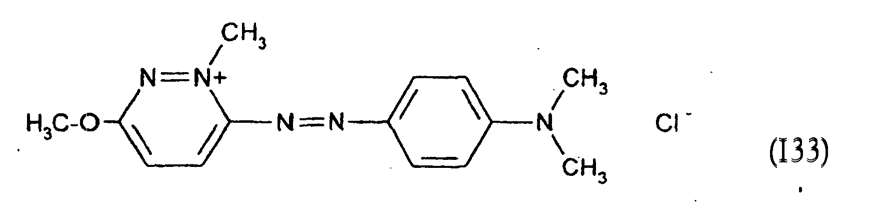 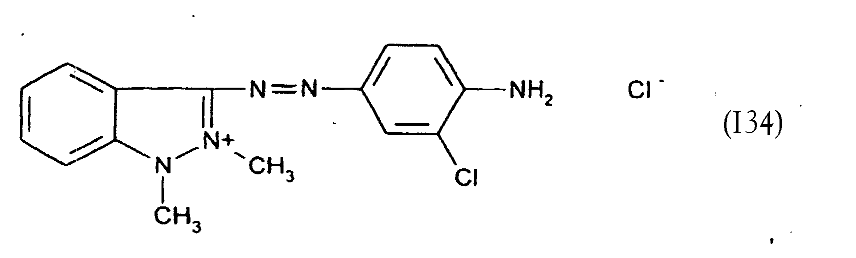 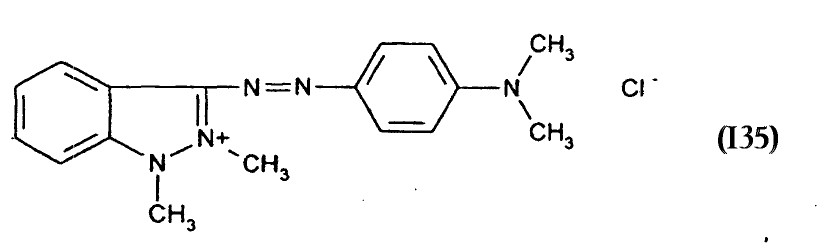 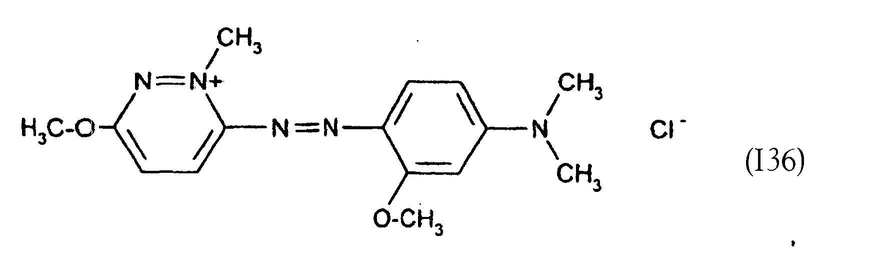 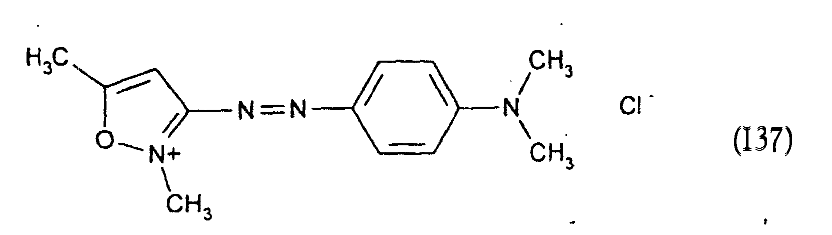 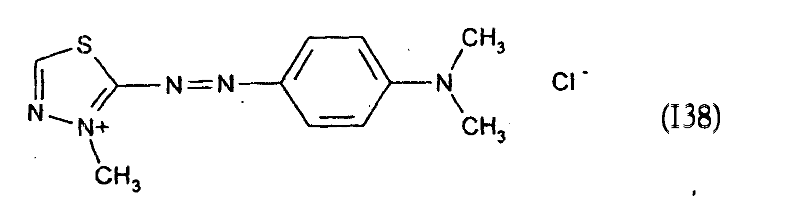 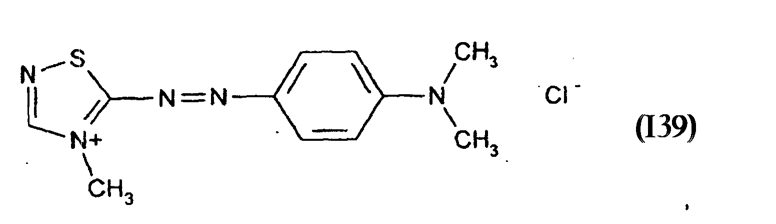 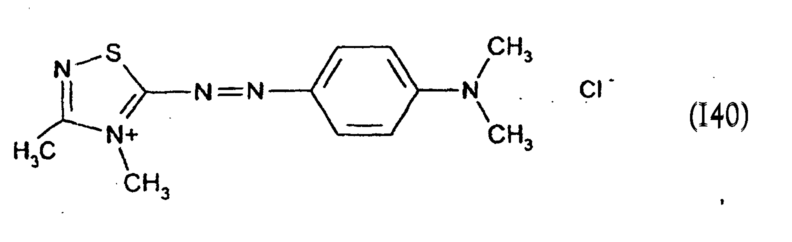 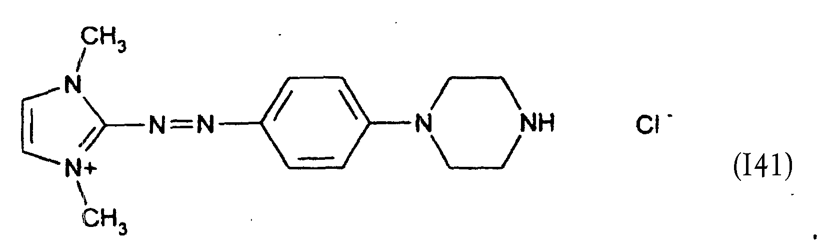 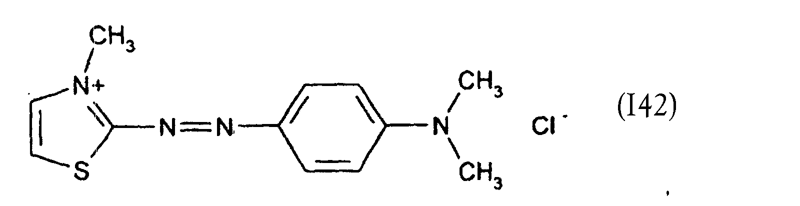 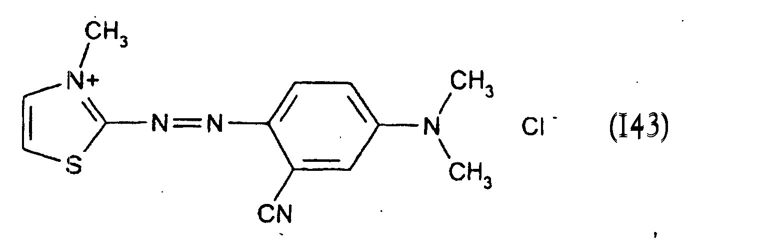 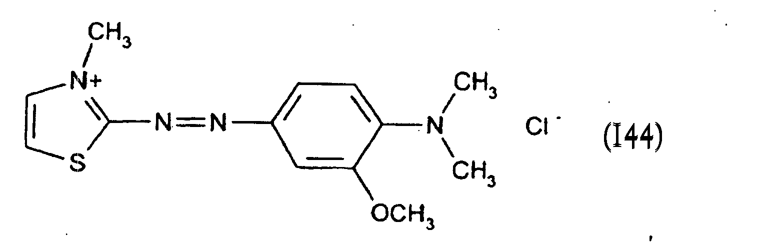 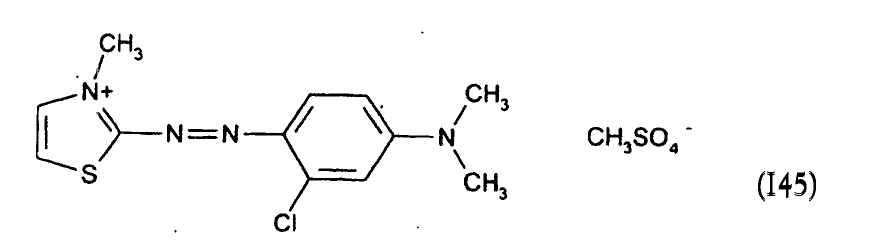 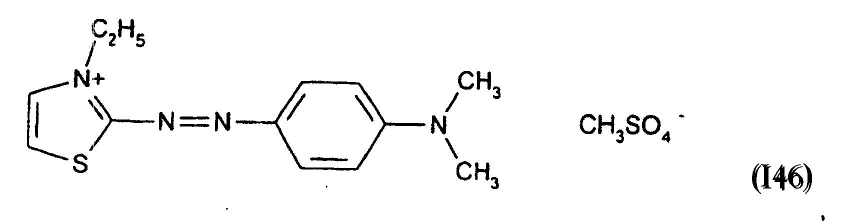 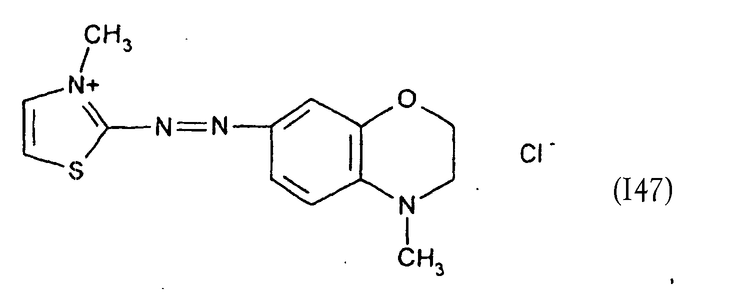 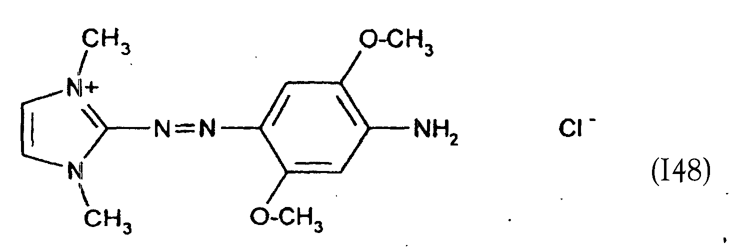 et 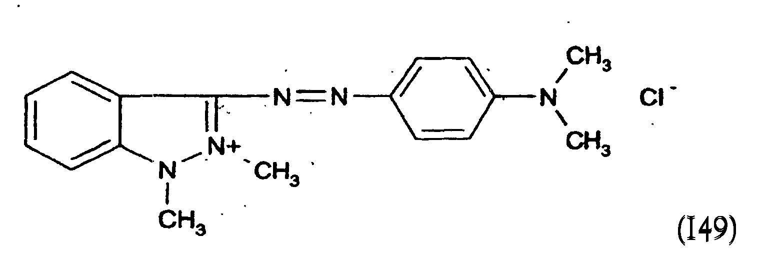 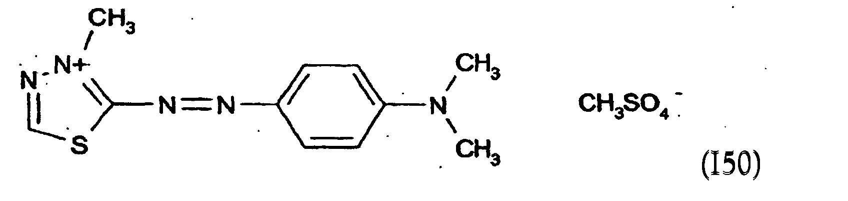 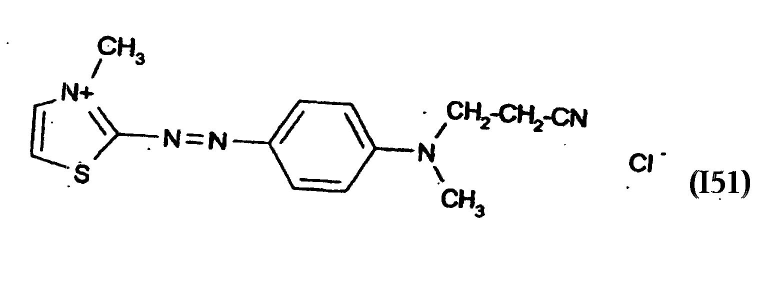
   Parmi les composés de structures (I1) à (I54) décrits ci-dessus, on préfère tout particulièrement les composés répondant aux structures (I1), (I2), (I13) et (I29)
   Parmi les colorants directs cationiques de formule (II) utilisables dans les compositions tinctoriales conformes à l'invention, on peut plus particulièrement citer les composés répondant aux structures (II1) à (II9) suivantes : 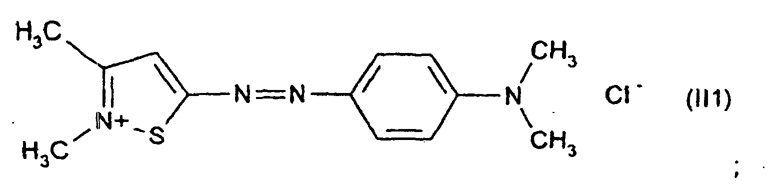 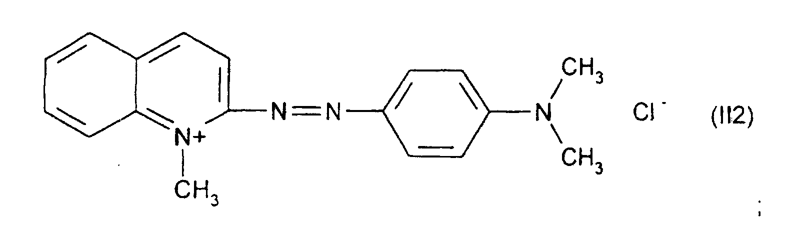 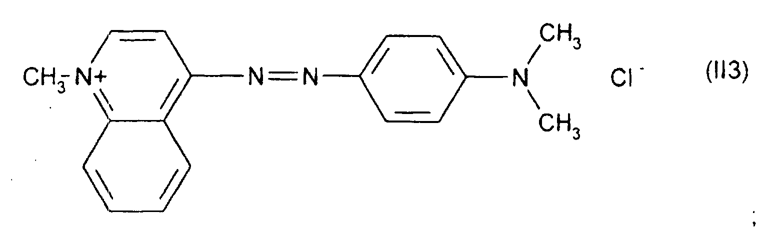 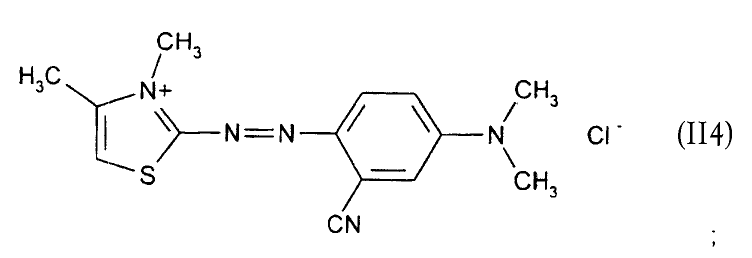 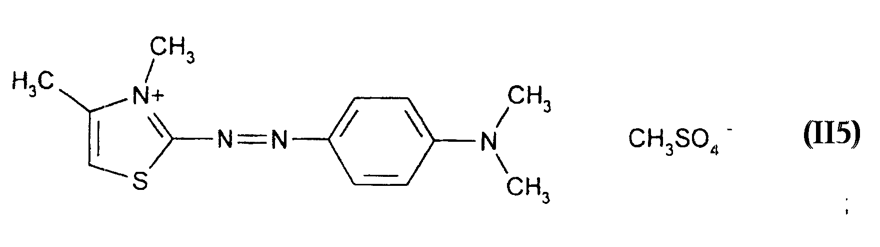 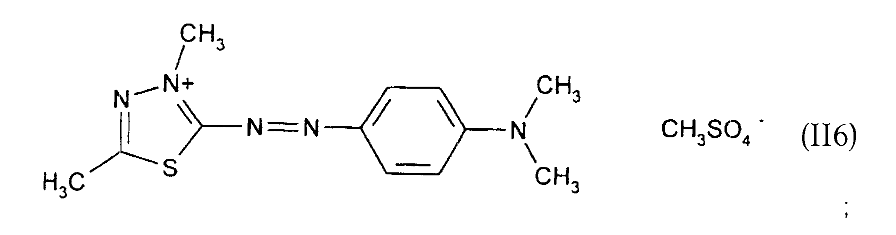 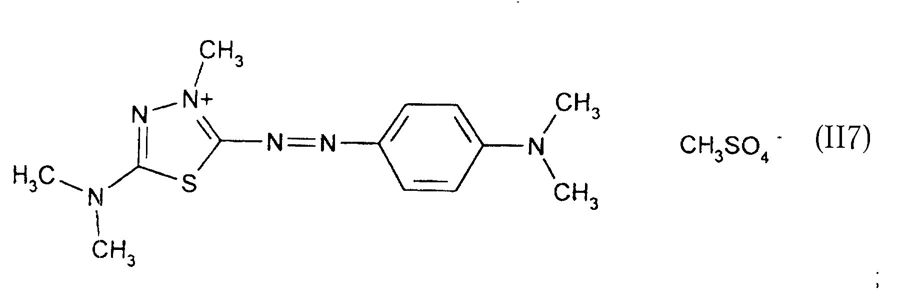 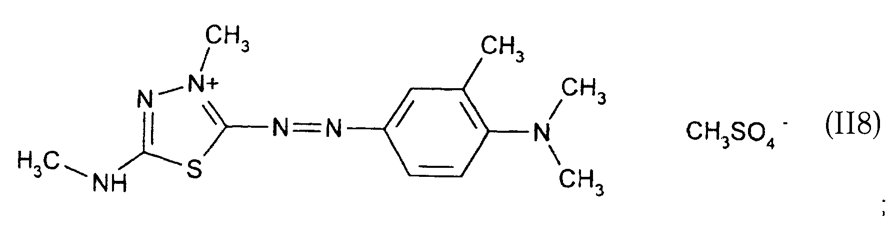 et 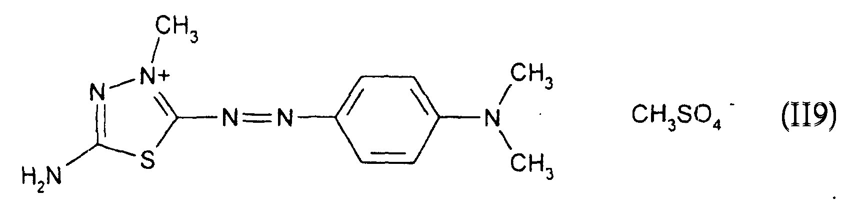
   Parmi les colorants directs cationiques de formule (III), utilisables dans les compositions tinctoriales conformes à l'invention, on peut plus particulièrement citer les composés répondant aux structures (III1) à (III18) suivantes : 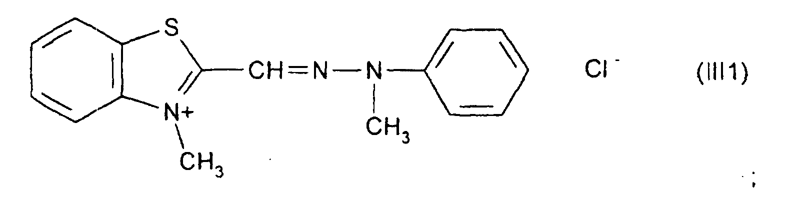 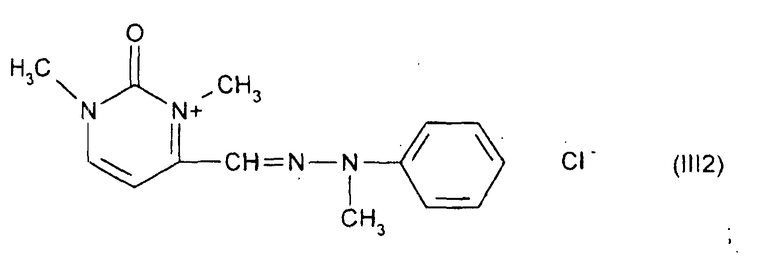 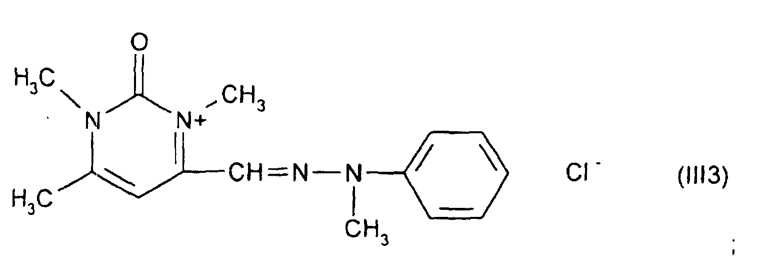 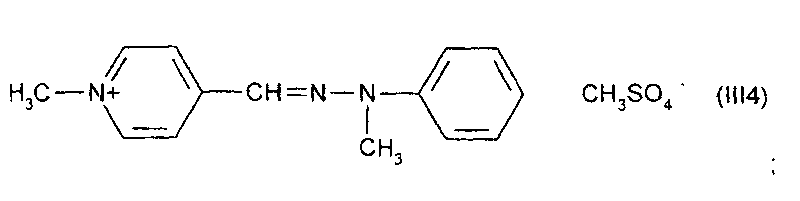 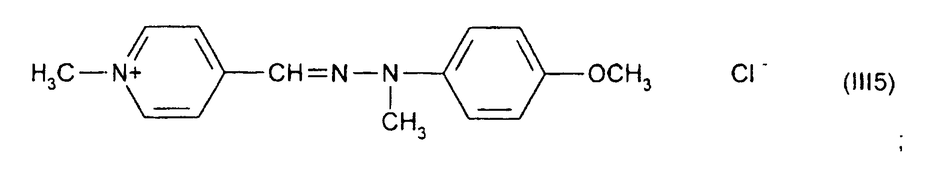 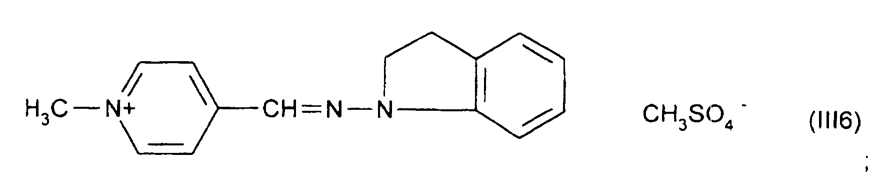 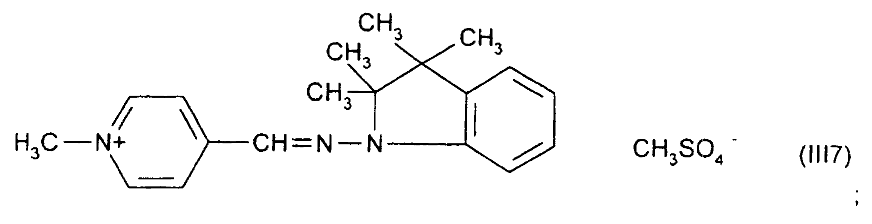 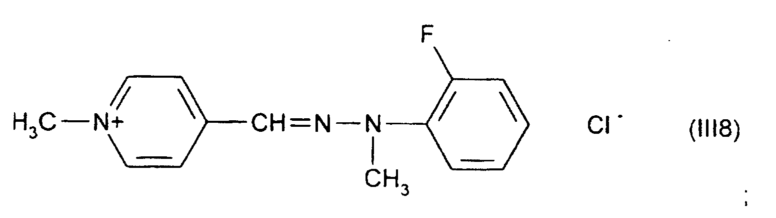 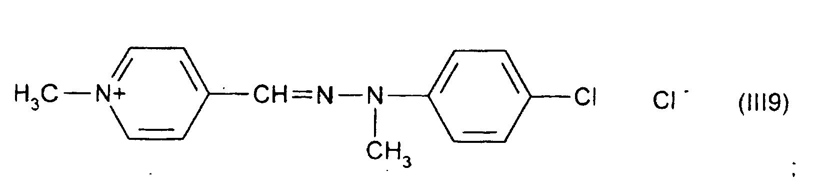 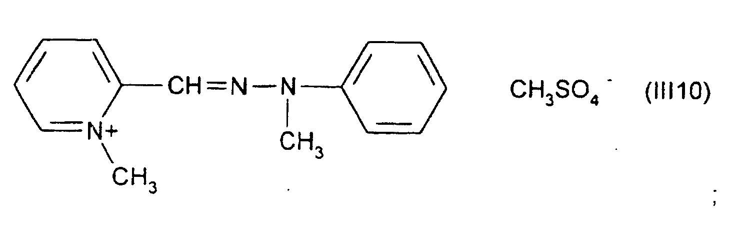 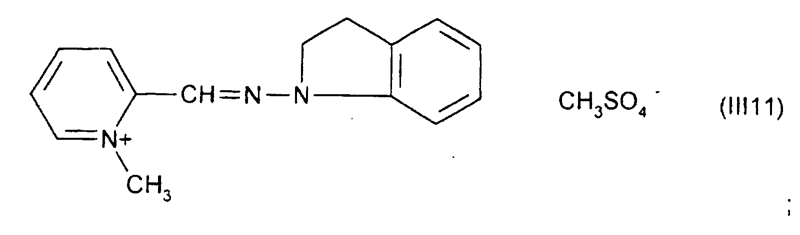 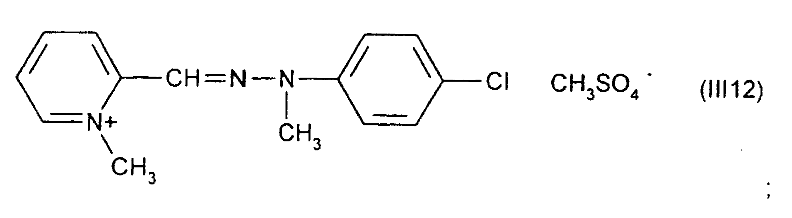 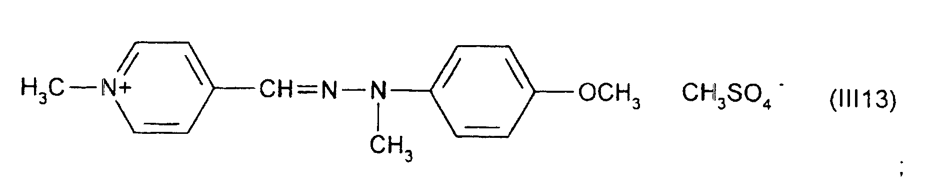 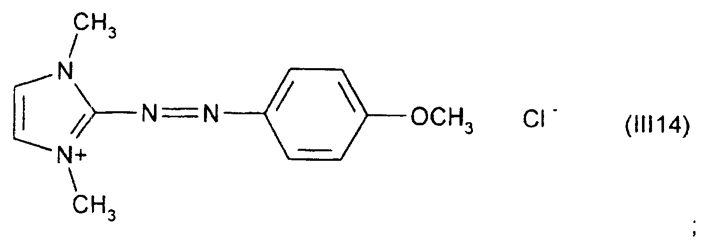 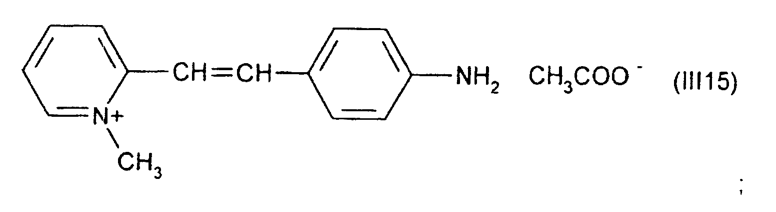 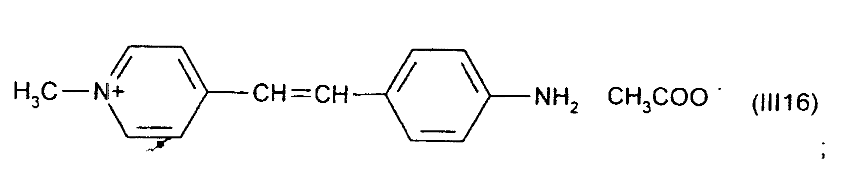 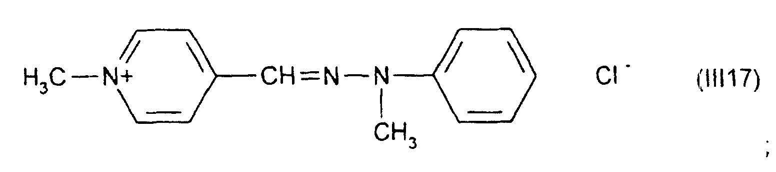 et 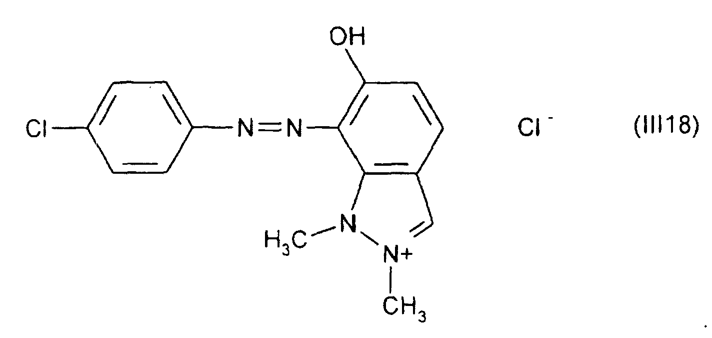
   Parmi les composés particuliers de structures (III1) à (III18) décrits ci-dessus, on préfère tout particulièrement les composés répondant aux structures (III4), (III5) et (III13).
   Parmi les colorants directs cationiques de formule (III'), utilisables dans les compositions tinctoriales conformes à l'invention, on peut plus particulièrement citer les composés répondant aux structures (III'1) à (III'3) suivantes : 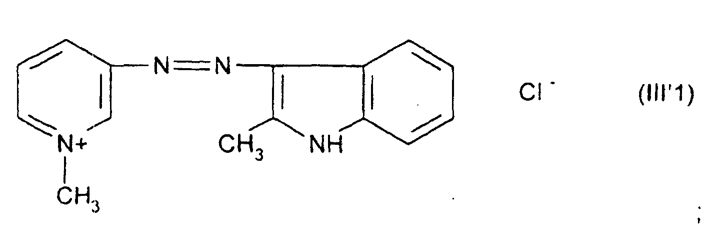 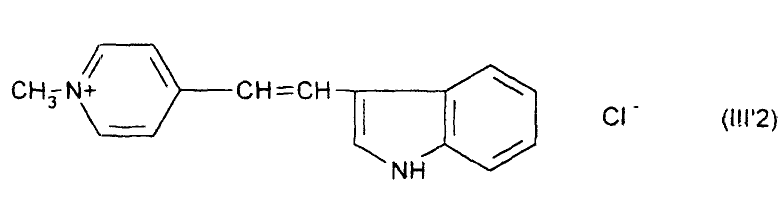 et 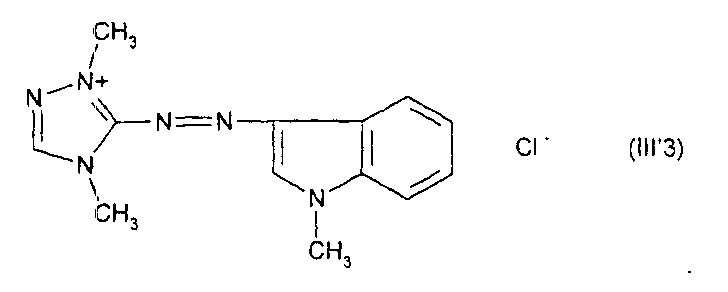
   Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.
**(ii)** Le polymère substantif cationique ou amphotère utilisable selon la présente invention est choisi dans le groupe constitué par :
   1/- les dérivés cationiques cellulosiques à l'exception du Polyquaternium 10;
   2/- les copolymères d'halogénure de diméthyldiallylammonium et d'acide (méth)acrylique ;
   3/- les homopolymères et copolymères d'halogénure de méthacryloyloxyéthyltriméthylammonium ;
   4/- les polymères polyammonium quaternaire choisis parmi :
      - les polymères constitués de motifs récurrents répondant à la formule (IV) suivante : 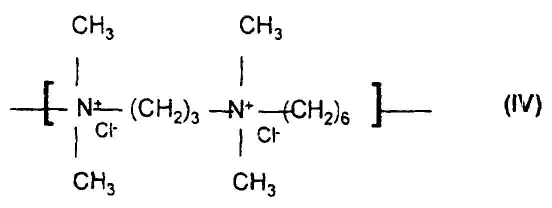
      - les polymères constitués de motifs récurrents répondant à la formule (V) suivante : 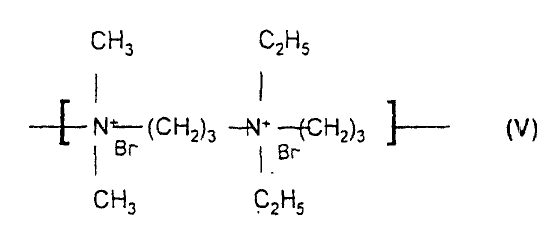
      - les polymères constitués de motifs récurrents répondant à la formule (VI) suivante : 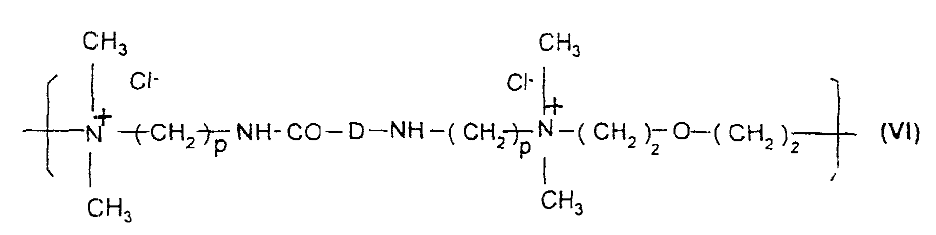
      dans laquelle p désigne un nombre entier variant de 1 à 6 environ, D peut être nul ou peut représenter un groupement -(CH₂)ᵣ₋CO- dans lequel r désigne un nombre égal à 4 ou à 7;
5/- les copolymères de vinylpyrrolidone à motifs cationiques ;
6/- leurs mélanges.

Le caractère substantif (c'est à dire l'aptitude au dépôt sur les cheveux) des polymères utilisés conformément à l'invention est classiquement déterminé au moyen du test décrit par Richard J. Crawford, Journal of the Society of Cosmetic Chemists, 1980, 31 - (5) - pages 273 à 278 (révélation par colorant acide Red 80).

Ces polymères substantifs peuvent être choisis parmi ceux antérieurement décrits dans la littérature, en particulier dans la demande de brevet EP-A-0 557 203, de la page 4, ligne 19, à la page 12, ligne 14.

On peut citer, parmi les dérivés cellulosiques cationiques, les dérivés d'éthers de cellulose quaternisés tels que ceux décrits dans la demande EP-A- 0 189 935, et en particulier le polymère commercialisé sous la dénomination "Quatrisoft LM 200" par la société Union Carbide ; ces polymères sont également définis dans le dictionnaire CTFA (5ème édition, 1993) comme des ammonium quaternaires d'hydroxyéthylcellulose ayant réagi avec un époxyde substitué par un groupement lauryldiméthylammonium et y sont répertoriés sous l'appellation de "Polyquaternium 24".

Parmi les polymères substantifs du type homopolymère et copolymère d'halogénure de méthacryloyloxyéthyltriméthylammonium utilisables selon l'invention, on peut citer en particulier les produits qui sont dénommés dans le dictionnaire CTFA (5ème édition, 1993) "Polyquaternium 37" , "Polyquaternium 32" et "Polyquaternium 35" , qui correspondent respectivement, en ce qui concerne le "Polyquaternium 37", à l'homopolymère poly(chlorure de méthacryloyloxyéthyltriméthylammonium) réticulé, en dispersion à 50% dans de l'huile minérale, et vendu sous la dénomination Salcare SC95 par la société Allied Colloids, en ce qui concerne le "Polyquaternium 32", au copolymère réticulé de l'acrylamide et du chlorure de méthacryloyloxyéthyltriméthylammonium (20/80 en poids), en dispersion à 50% dans de l'huile minérale, et vendu sous la dénomination Salcare SC92 par la société Allied Colloids, et en ce qui concerne le "Polyquaternium 35", au méthosulfate du copolymère de méthacryloyloxyéthyltriméthylammonium et de méthacryloyloxyéthyldiméthylacétylammonium, vendu sous la dénomination Plex 7525L par la société Rohm GmbH.

Parmi les polymères substantifs du type copolymère d'halogénure de diméthyldiallylammonium et d'acide (méth)acrylique utilisables selon l'invention, on peut citer en particulier les copolymères de chlorure de diallyldiméthylammonium et d'acide acrylique comme celui de proportions (80/20 en poids) vendu sous la dénomination Merquat 280 par la société Calgon.

Parmi les polymères substantifs du type polyammonium quaternaire utilisables selon l'invention, on peut citer en particulier:
- les polymères préparés et décrits dans le brevet français 2 270 846, constitués de motifs récurrents répondant à la formule (IV) suivante : 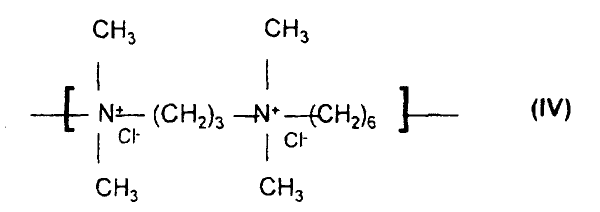 et notamment ceux dont le poids moléculaire, déterminé par chromatographie par perméation de gel, est compris entre 9500 et 9900 ;
- les polymères préparés et décrits dans le brevet français 2 270 846, constitués de motifs récurrents répondant à la formule (V) suivante : 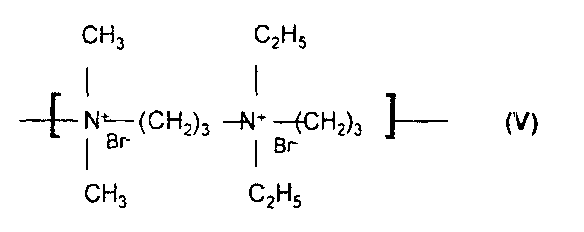 et notamment ceux dont le poids moléculaire, déterminé par chromatographie par perméation de gel, est d'environ 1200 ;
- les polymères décrits et préparés dans les brevets US 4 157 388, 4 390 689, 4 702 906, 4 719 282, et constitués de motifs récurrents répondant à la formule (VI) suivante : 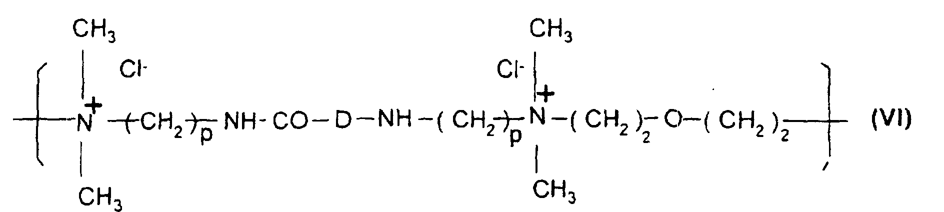 dans laquelle p désigne un nombre entier variant de 1 à 6 environ, D peut être nul ou peut représenter un groupement -(CH₂)ᵣCO- dans lequel r désigne un nombre égal à 4 ou à 7, la masse moléculaire desdits polymères étant de préférence inférieure à 100 000, et plus préférentiellement encore inférieure ou égale à 50 000 ; de tels polymères sont notamment vendus par la société Miranol sous les dénominations "Mirapol A15", "Mirapol AD1", "Mirapol AZ1" et "Mirapol 175" ;

Parmi les polymères de Vinylpyrrolidone (PVP) à motifs cationiques utilisables conformément à l'invention , on peut citer en particulier:
a) les polymères de Vinylpyrrolidone comportant des motifs Méthacrylate de diméthylaminoéthyle ; on peut citer parmi ceux-ci :
   - le copolymère Vinylpyrrolidone / Méthacrylate de diméthylaminoéthyle (20/80 en poids) vendu sous la dénomination commerciale COPOLYMER 845 par la société I.S.P.
   - les copolymères Vinylpyrrolidone / Méthacrylate de diméthylaminoéthyle quaternisés par du sulfate de diéthyle, vendus sous les dénominations GAFQUAT 734, 755, 755 S et 755 L par la société I.S.P.
   - les PVP / Méthacrylate de diméthylaminoéthyle / Polyuréthane hydrophile, vendus sous la dénomination commerciale PECOGEL GC-310 par la société U.C.I.B. ou encore sous les dénominations AQUAMERE C 1031 et C 1511 par la société BLAGDEN CHEMICALS,
   - les PVP Méthacrylate de diméthylaminoéthyle / Oléfine en C8 à C16, quaternisés ou non quarternisés, vendus sous les dénominations GANEX ACP 1050 à 1057, 1062 à 1069, 1079 à 1086, par la société I.S.P.
   - le PVP / Méthacrylate de diméthylaminoéthyle / Vinylcaprolactame, vendu sous la dénomination GAFFIX VC 713 par la société I.S.P.
b) les polymères de Vinylpyrrolidone comportant des motifs Méthacrylamidopropyltriméthylammonium ( M.A.P.T.A.C. ), parmi lesquels on peut citer notamment :
   - les copolymères Vinylpyrrolidone / M.A.P.T.A.C., vendus sous les dénominations commerciales GAFQUAT ACP 1011 et GAFQUAT HS 100 par la société I.S.P.
c) les polymères de Vinylpyrrolidone comportant des motifs Méthylvinylimidazolium, et parmi lesquels on peut citer plus particulièrement :
   - les PVP / Chlorure de méthylvinylimidazolium, vendus sous les dénominations LUVIQUAT FC 370, FC 550, FC 905, HM 552 par la société B.A.S.F.
   - le PVP / Chlorure de méthylvinylimidazolium / Vinylimidazole, vendu sous la dénomination LUVIQUAT 8155 par la société B.A.S.F.
   - le PVP / Méthosulfate de méthylvinylimidazolium, vendu sous la dénomination LUVIQUAT MS 370 par la société B.A.S.F.

La concentration en polymère substantif (**ii**) dans la composition de teinture selon l'invention peut varier entre 0,01 et 10 % environ par rapport au poids total de la composition de teinture, et de préférence entre 0,1 et 5 %.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique ou le phénoxyéthanol, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 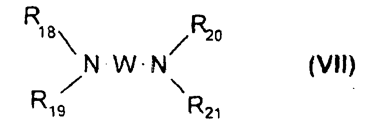 dans laquelle W est un reste propylène éventuellement substitué par un groupement hydroxyle ou un radical alkyle en C₁-C₆ ; R₁₈, R₁₉, R₂₀ et R₂₁, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques. Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et du polymère substantif (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon claissique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents tensioactifs, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. La présentation sous forme de shampooing est particulièrement préférée.

Lorsque l'association du colorant direct cationique (i) et du polymère substantif (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydoréductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère substantif cationique ou amphotère (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère substantif cationique ou amphotère tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLE 1 :

On a préparé la composition de teinture suivante :

| | |
|---|---|
| Colorant direct cationique de formule I(2) | 0,125 g |
| Paraaminophénol | 0,120 g |
| 5-N-β-hydroxyéthylamino-2-méthyl-phénol | 0,125 g |
| Polymère substantif: copolymère de chlorure de | |
| diallyldiméthylammonium et d'acide acrylique (80/20 en poids) | |
| vendu sous la dénomination Merquat 280 par la société | |
| CALGON | 1,0 g M.A.* |
| Alcool oléique polyglycérolé à 2 moles de glycérol | 4,0 g |
| Alcool oléique polyglycérolé à 4 moles de glycérol, à 78 % de | |
| matières actives (M.A.) | 5,69 g M.A.* |
| Acide oléique | 3,0 g |
| Amine oléique à 2 moles d'oxyde d'éthylène vendue sous la dénomination commerciale ETHOMEEN 012 par la société AKZO | 7,0 g |
| Laurylamino succinamate de diéthylaminopropyle, sel de | 3,0 g M.A.* |
| sodium, à55% de M.A | |
| Alcool oléique | 5,0 g |
| Diéthanolamide d'acide oléique | 12,0 g |
| Propylèneglycol | 3,5 g |
| Dipropylèneglycol | 0,5 g |
| Monométhyléther de propylèneglycol | 9,0 g |
| Ethanol | 7,0 g |
| Métabisulfite de sodium en solution aqueuse, à 35 % de M.A | 0,455 g M.A.* |
| Acétate d'ammonium | 0,8 g |
| Antioxydant, séquestrant | q.s. |
| Parfum, conservateur | q.s. |
| Ammoniaque à 20 % de NH₃ | 10,0 g |

| | |
|---|---|
| M.A.*: Matière Active | |

Au moment de l'emploi, on a mélangé cette composition avec une quantité égale d'une solution aqueuse de peroxyde d'hydrogène à 20 volumes (6 % en poids).

La composition résultante a été appliquée pendant 30 minutes sur des mèches de cheveux gris naturels à 90 % de blancs. Les mèches de cheveux ont ensuite été rincées, lavées avec un shampooing standard puis séchées.

Elles ont été teintes dans une nuance blond clair à reflet rouge intense.

### EXEMPLE 2 :

On a préparé la composition de teinture suivante :

| | |
|---|---|
| Colorant direct cationique de formule I(14) | 0,09 g |
| Polymère substantif de type polyammonium quaternaire | |
| de formule(lV) | 1,0 g M.A.* |
| Nonyl phénol à 9 moles d'oxyde d'éthylène | 8,0 g |
| 2-amino-2-méthyl-propanol q.s | pH 9 |
| Eau déminéralisée q.s.p | 100 g |

| | |
|---|---|
| M.A.* : Matière Active | |

La composition ci-dessus a été appliquée pendant 30 minutes sur des mèches de cheveux gris naturels à 90 % de blancs. Les mèches de cheveux ont ensuite été rincées, lavées avec un shampooing standard puis séchées.

Elles ont été teintes dans une nuance cuivrée intense.

## Revendications

1. Composition pour la teinture des fibres kératiniques et en particulier des fibres kératiniques humaines telles que les cheveux, renfermant dans un milieu approprié pour la teinture, **(i)** au moins un colorant direct cationique de formules suivantes (I), (II), (III), (III'): 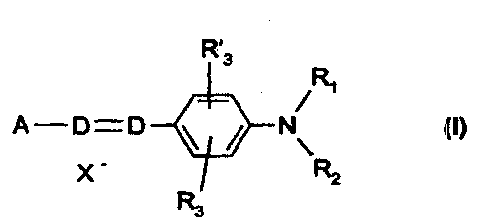 **formule (I) dans laquelle :**
**D** représente un atome d'azote ou le groupement -CH,
**R**_{**1**} et **R**_{**2**}, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
**R**_{**3**} et **R'**_{**3**}, identiques ou différents, représentent un atome d'hydrogène ou d'halogène choisi parmi le chlore, le brome, l'iode et le fluor, un radical cyano, alcoxy en C₁-C₄ ou acétyloxy,
**X** représente un anion de préférence choisi parmi le chlorure, le méthyl sulfate et l'acétate,
**A** représente un groupement choisi par les structures A1 à A18 suivantes : 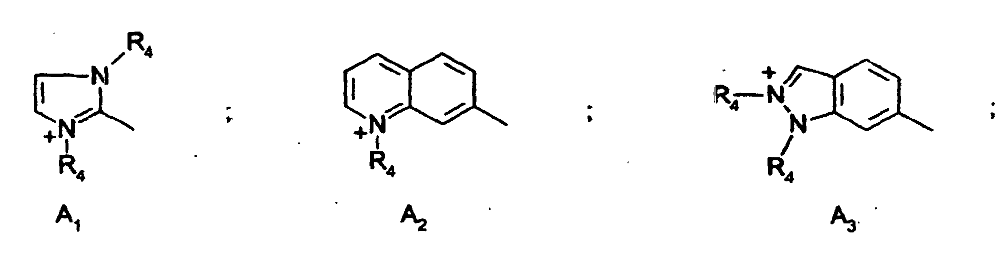 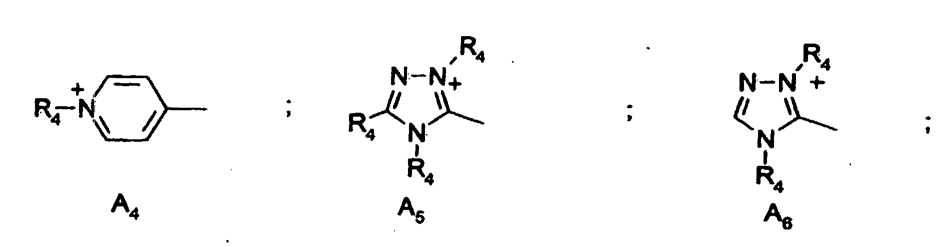 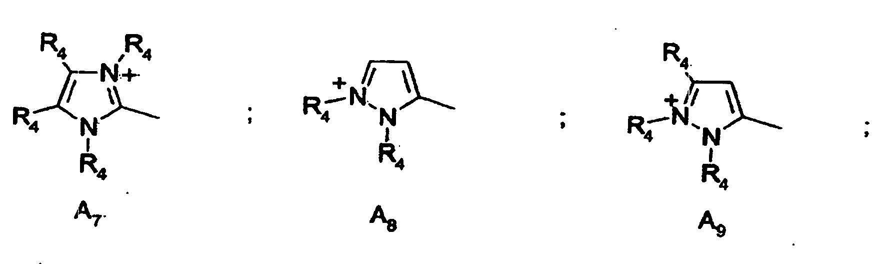 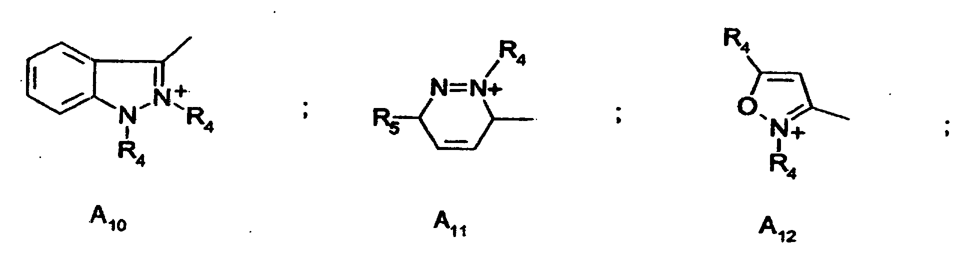 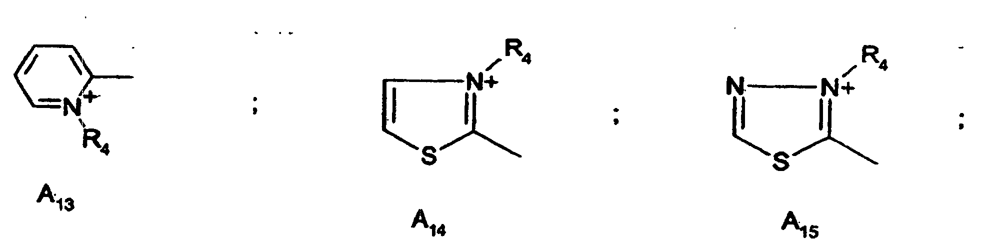 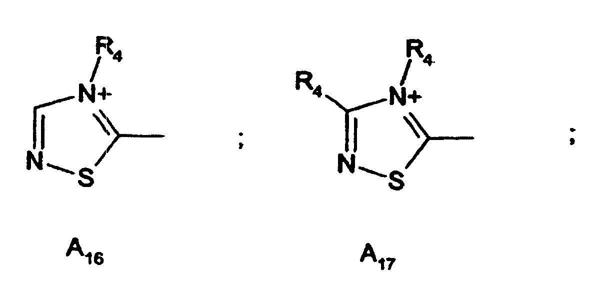 et 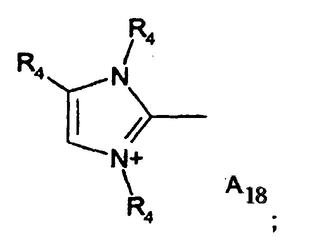 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
ainsi que les composés suivants de formule (I) : 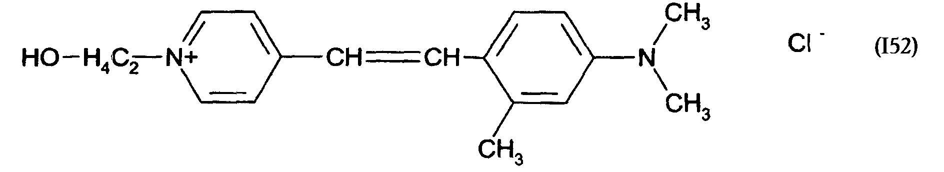 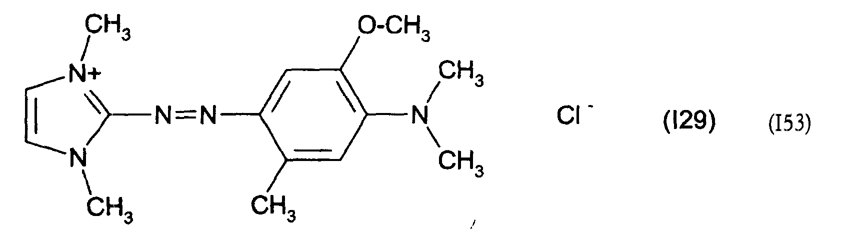 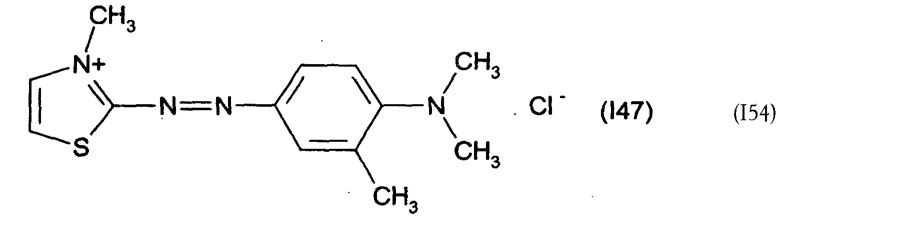 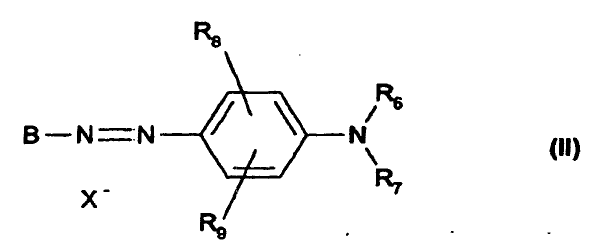
**formule (II) dans laquelle :**
**R**_{**6**} représente un atome d'hydrogène ou un radical alkyle en C₁-C₄,
**R**_{**7**} représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
**R**_{**8**} et **R**_{**9**}**,** identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
**X** représente un anion de préférence choisi parmi le chlorure, le méthyl sulfate et l'acétate,
**B** représente un groupement choisi par les structures B1 à B6 suivantes : 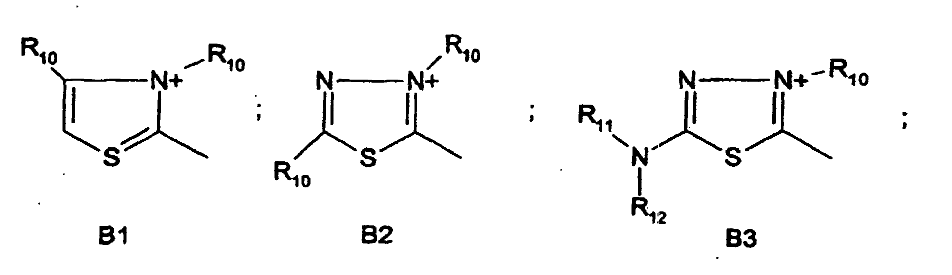 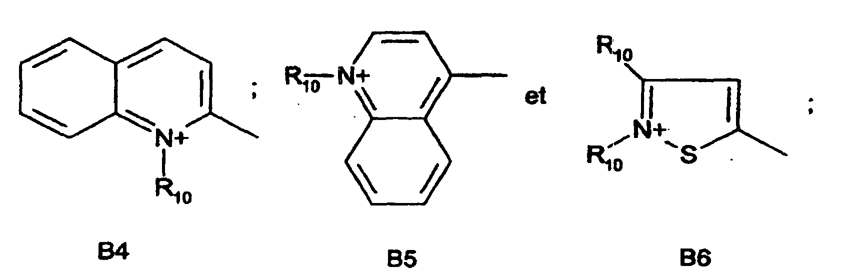 dans lesquelles R₁₀ représente un radical alkyle en C₁-C₄, R₁₁ et R₁₂, identiques ou différents, représentent un atome d'hydrogène ou un radical alkyle en C₁-C₄ ; 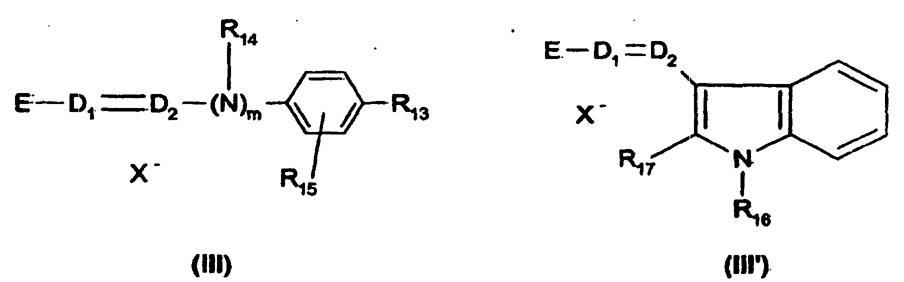
**formules (III) et (III') dans lesquelles :**
**R**_{**13**} représente un atome d'hydrogène, un radical alcoxy en C₁-C₄, un atome d'halogène tel que le brome, le chlore, l'iode ou le fluor ou un radical amino,
**R**_{**14**} représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
**R**_{**15**} représente un atome d'hydrogène ou d'halogène tel que le brome, le chlore, l'iode ou le fluor,
**R**_{**16**} et **R**_{**17**}**,** identiques ou différents, représentent un atome d'hydrogène ou un radical alkyle en C₁-C₄,
**D**_{**1**} et **D**_{**2**}, identiques ou différents, représentent un atome d'azote ou le groupement -CH,
**m** = 0 ou 1,
étant entendu que lorsque R₁₃ représente un groupement amino non substitué, alors D₁ et D₂ représentent simultanément un groupement -CH et m = 0,
**X**⁻ représente un anion de préférence choisi parmi le chlorure, le méthyl sulfate et l'acétate,
**E** représente un groupement choisi par les structures E1 à E8 suivantes : 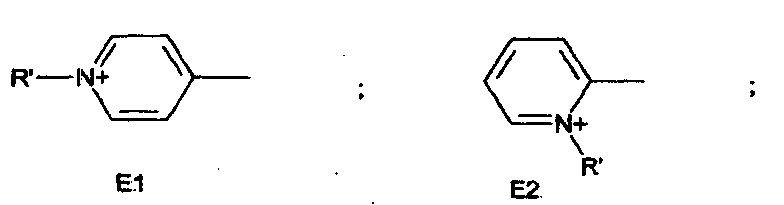 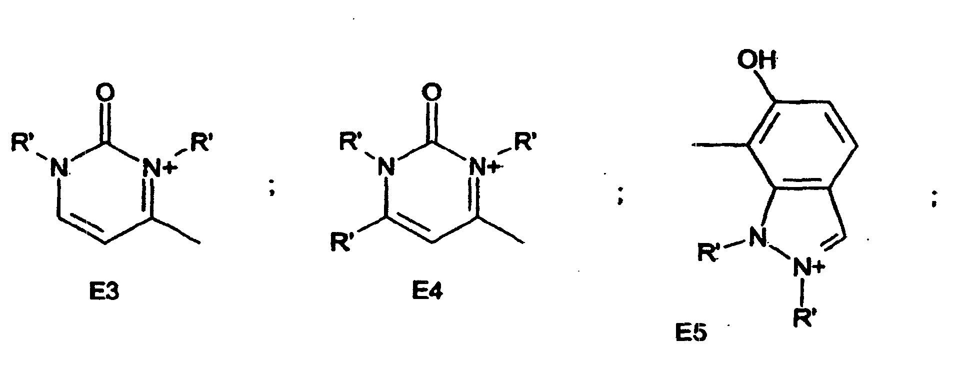 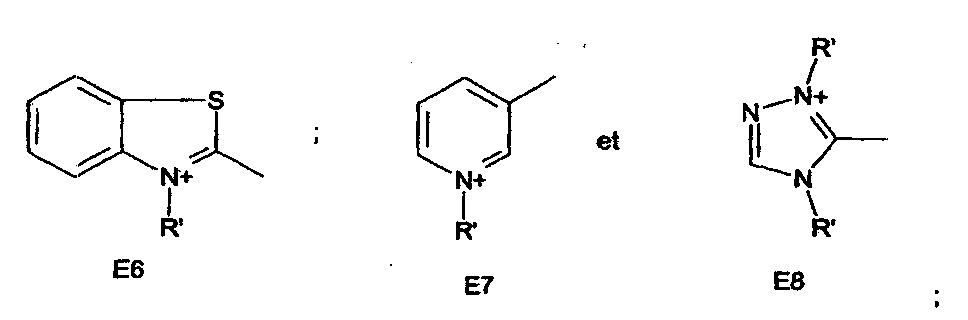 dans lesquelles R' représente un radical alkyle en C₁-C₄ ;
lorsque m = 0 et que D₁ représente un atome d'azote, alors E peut également désigner un groupement de structure E9 suivante : 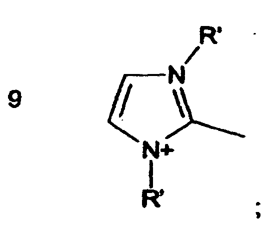 dans laquelle R' représente un radical alkyle en C₁-C₄ ;
ladite composition étant **caractérisée par le fait qu'**elle contient en outre (ii) au moins un polymère substantif cationique ou amphotère choisi dans le groupe formé par :
**1/-** les dérivés cationiques cellulosiques à l'exception du Polyquaternium 10 ;
**2/-** les copolymères d'halogénure de diméthyldiallylammonium et d'acide (méth)acrylique ;
**3/-** les homopolymères et copolymères d'halogénure deméthacryloyloxyéthyl triméthylammonium ;
**4/-** les polymères polyammonium quaternaire choisis parmi :
- les polymères constitués de motifs récurrents répondant à la formule (IV) suivante: 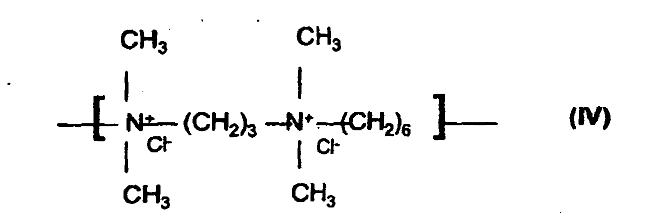
- les polymères constitués de motifs récurrents répondant à la formule (V) suivante : 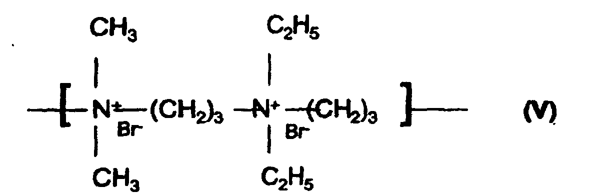
- les polymères constitués de motifs récurrents répondant à la formule (VI) suivante : 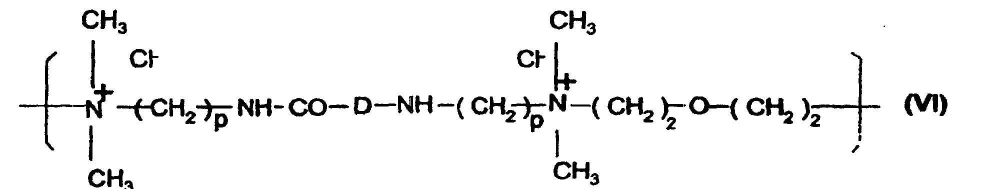 dans laquelle p désigne un nombre entier variant de 1 à 6 environ, D peut être nul ou peut représenter un groupement -(CH₂)ᵣ₋CO- dans lequel r désigne un nombre égal à 4 ou à 7 ;
**5/-** les copolymères de vinylpyrrolidone à motifs cationiques ;
**6/-** leurs mélanges.

2. Composition selon la revendication 1, **caractérisée par le fait que** les colorants directs cationiques de formule (I) sont choisis parmi les composés répondant aux structures (I1) à (I51) suivantes :

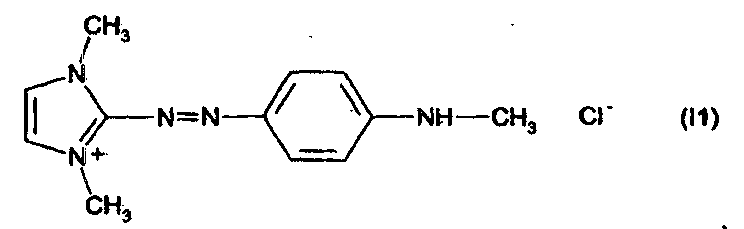

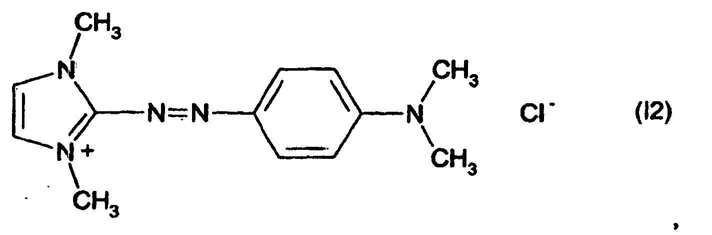

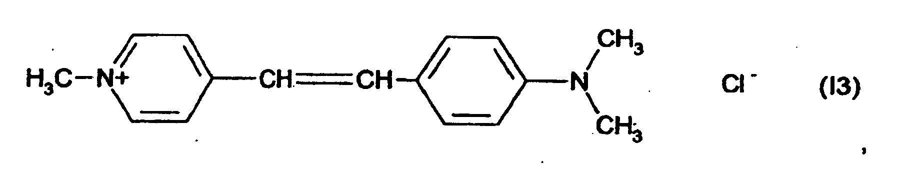

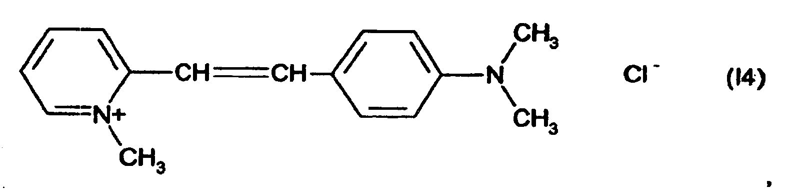

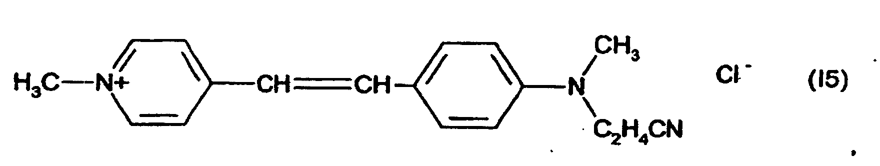

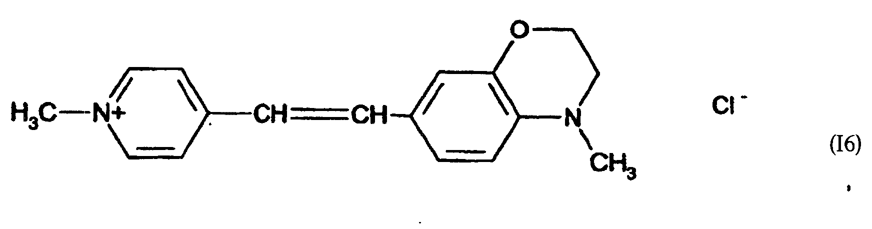

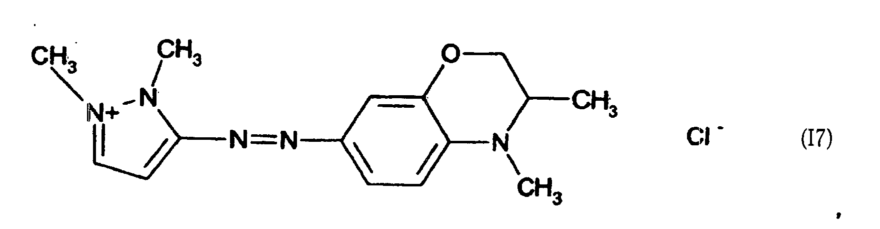

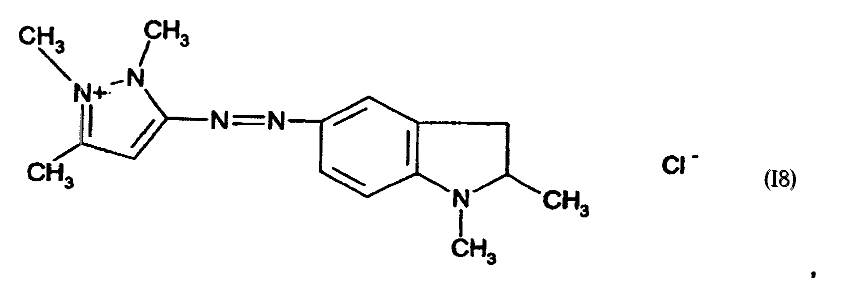

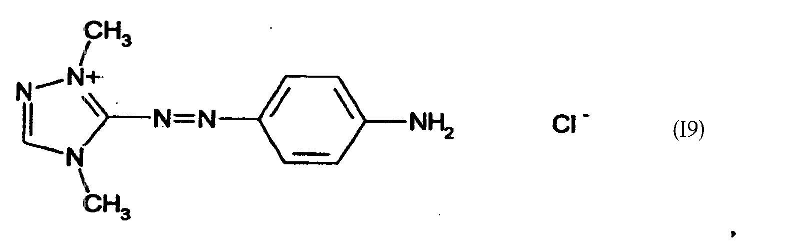

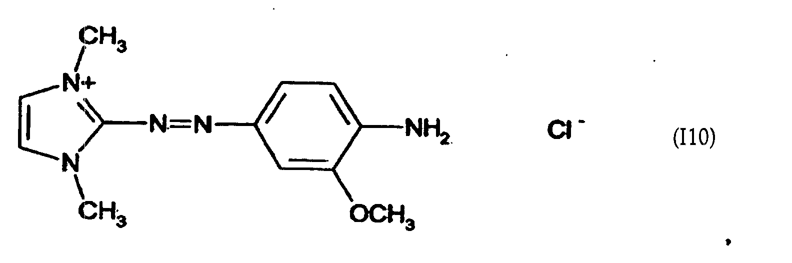

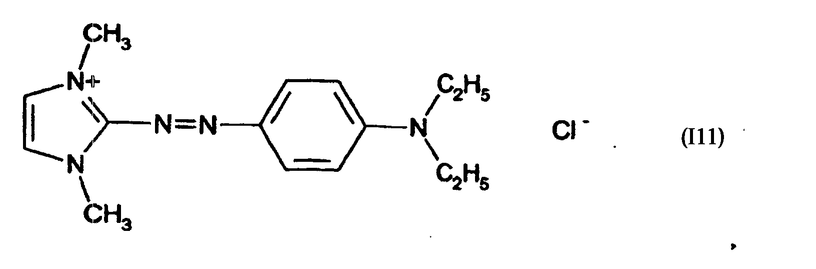

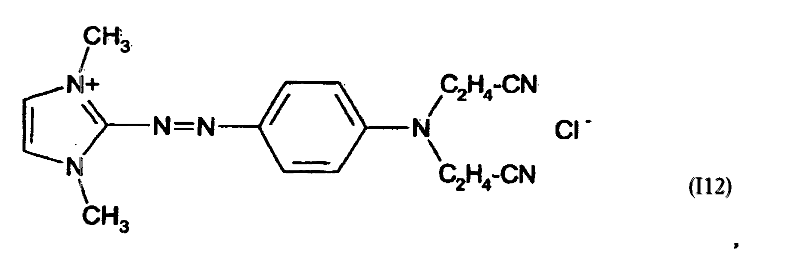

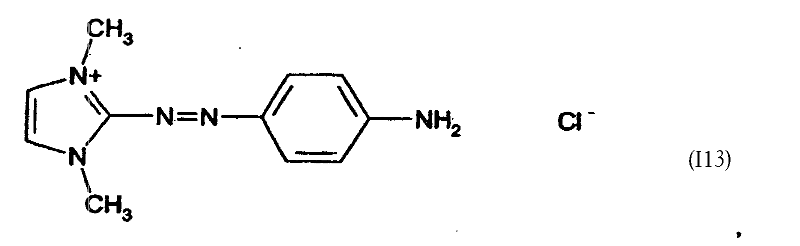

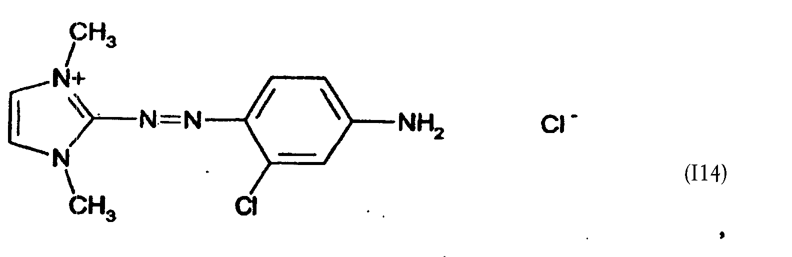

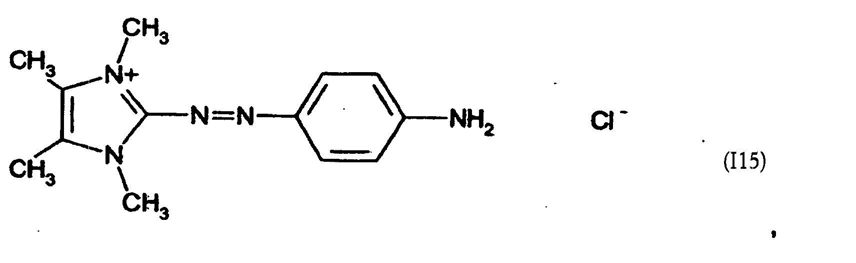

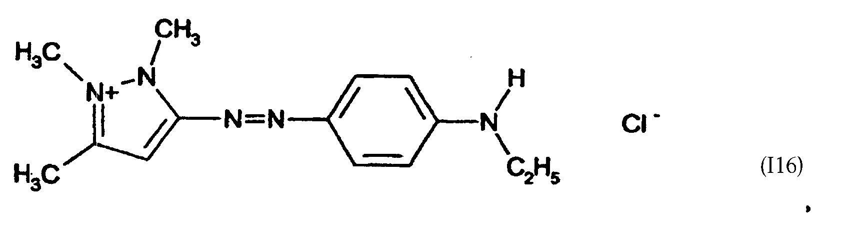

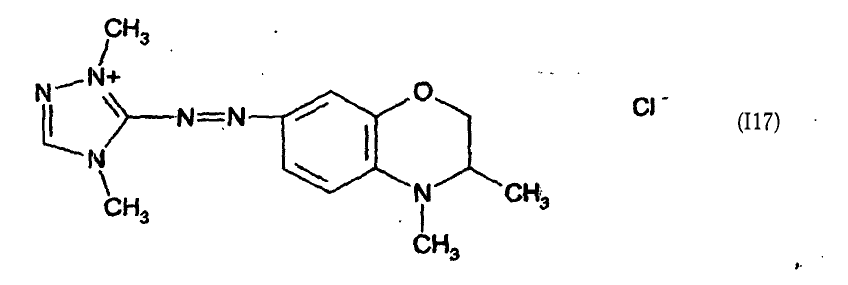

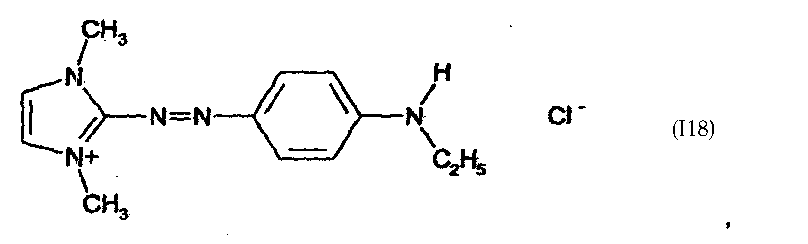

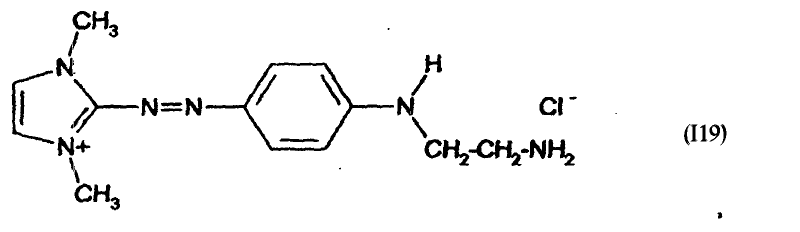

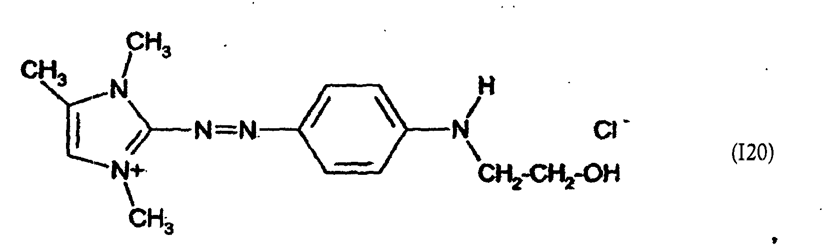

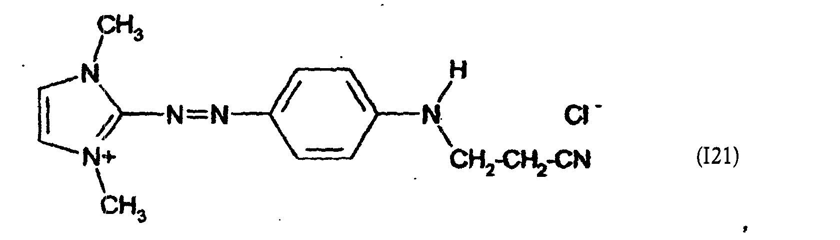

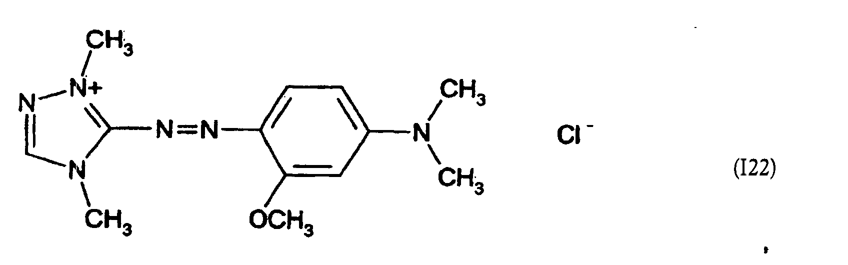

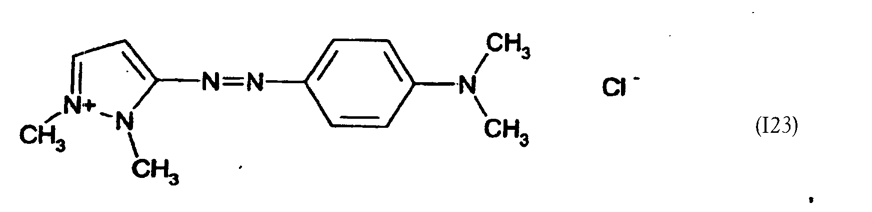

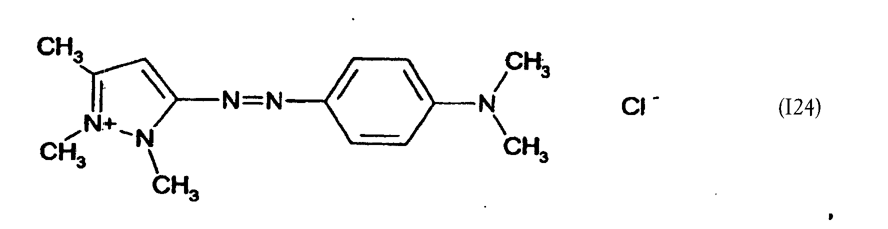

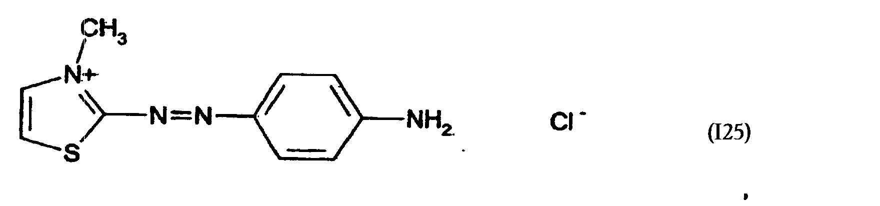

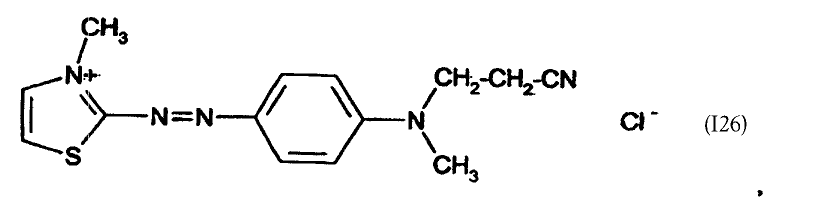

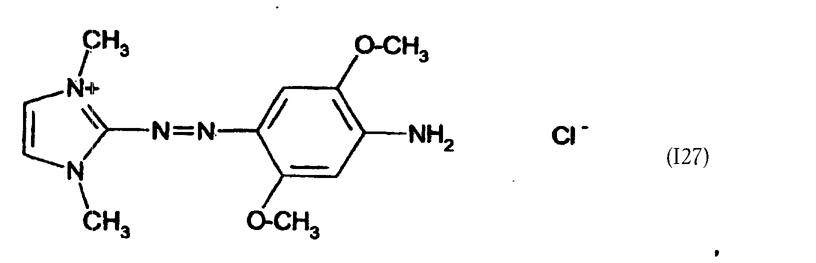

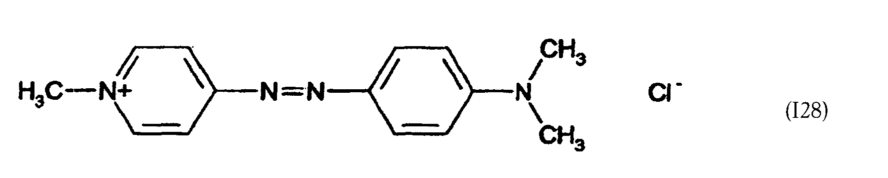

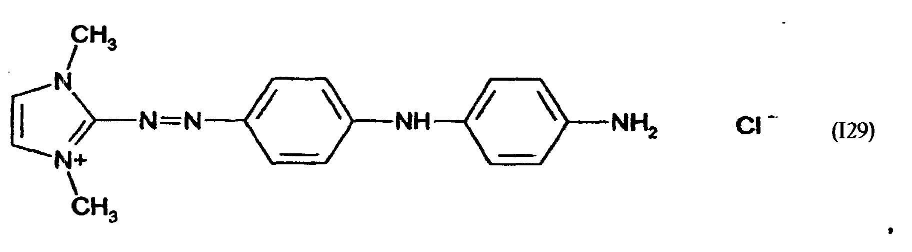

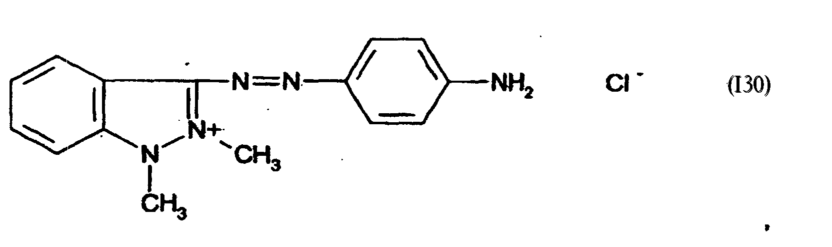

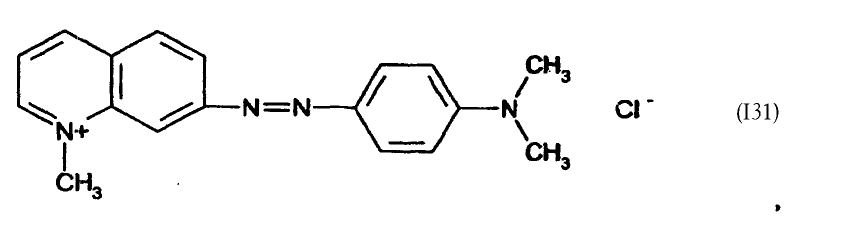

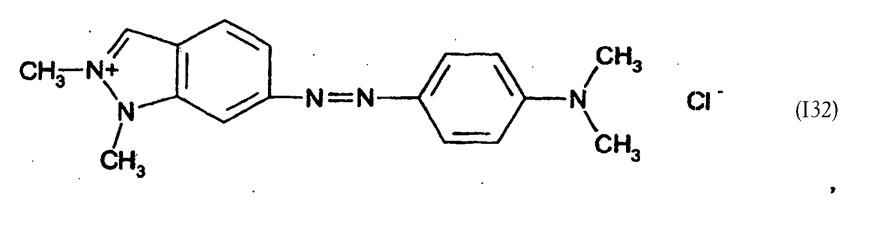

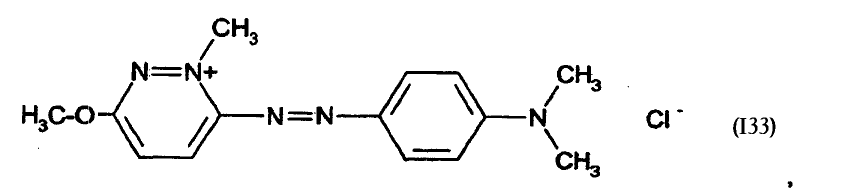

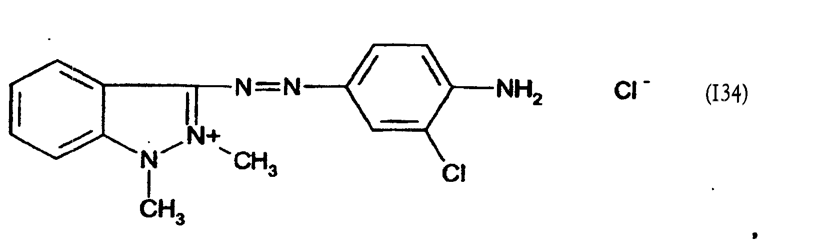

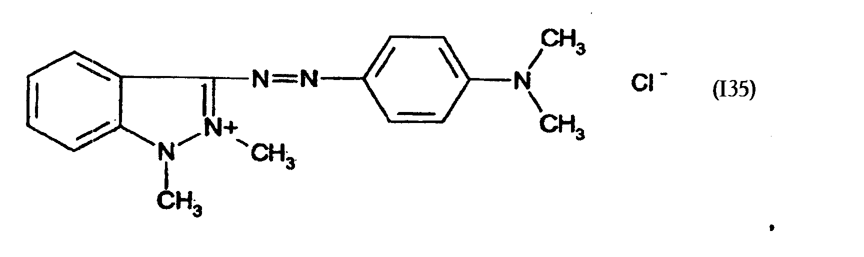

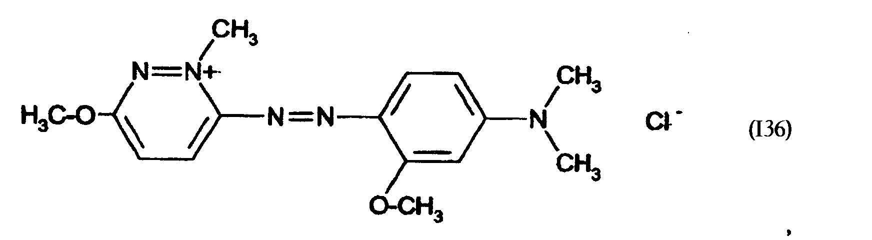

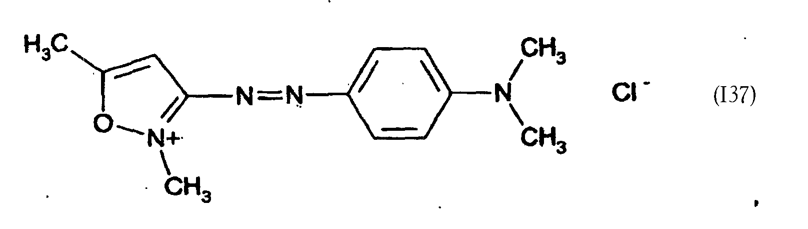

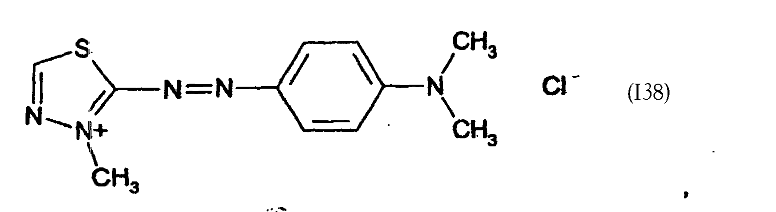

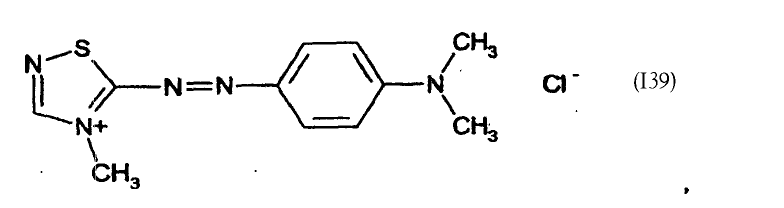

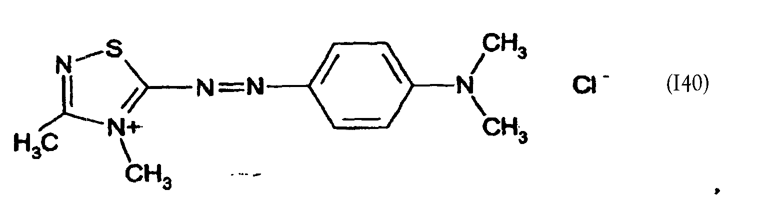

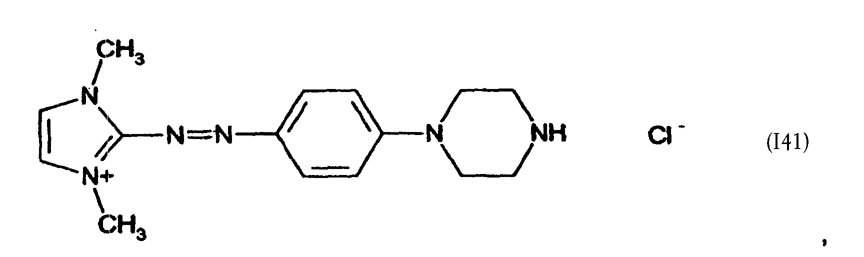

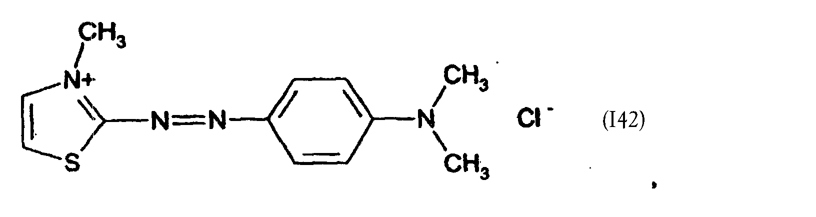

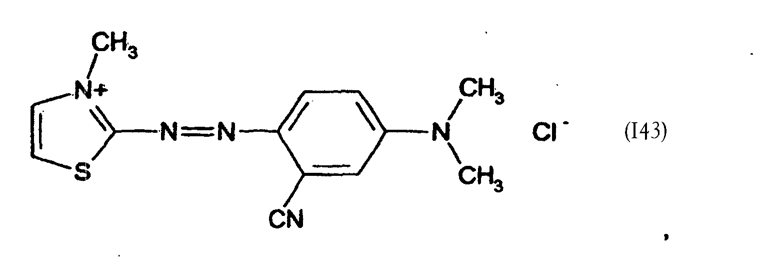

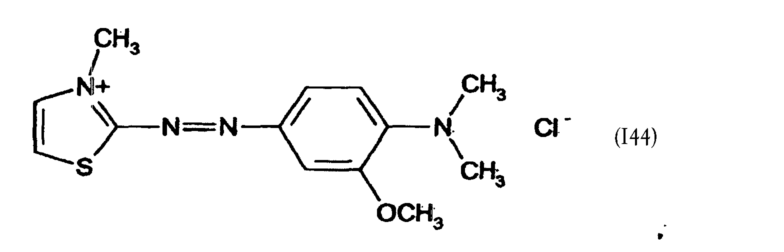

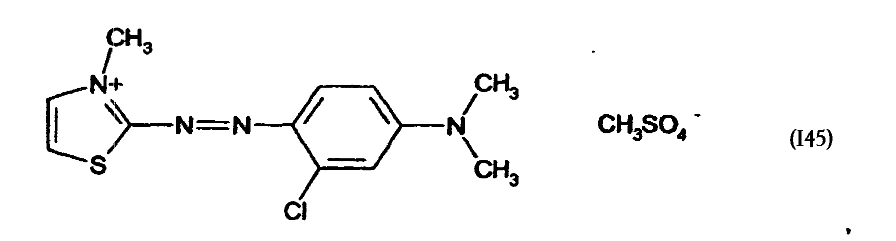

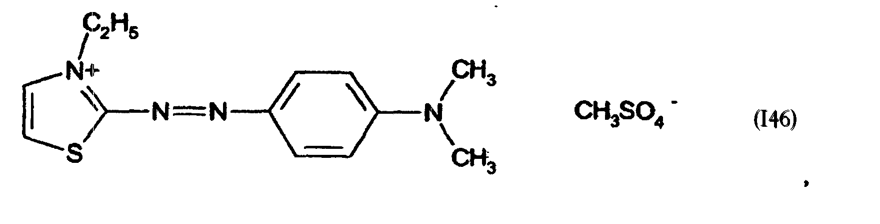

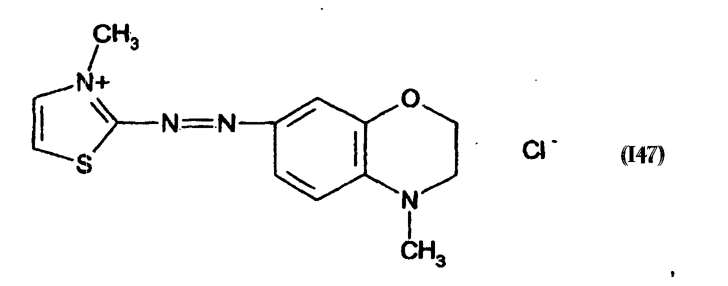

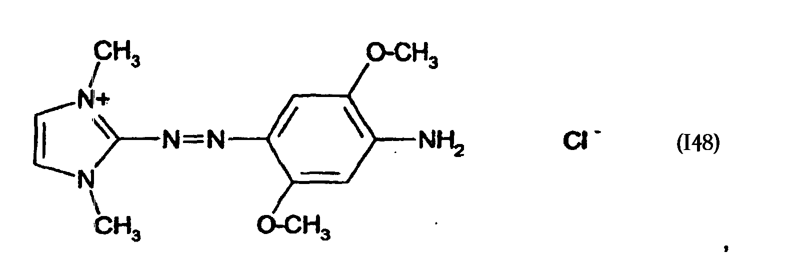

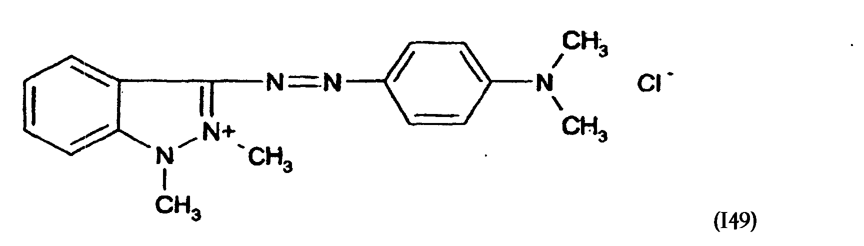

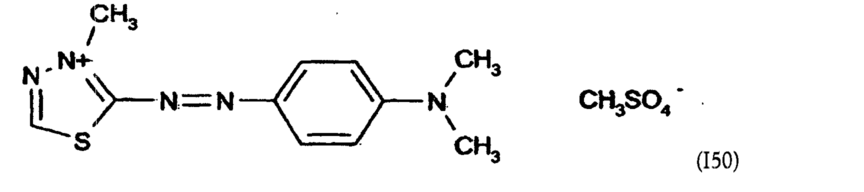

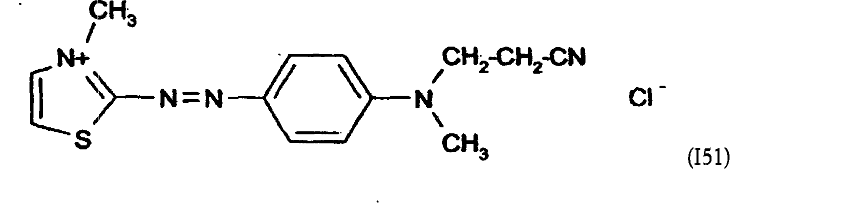

3. Composition selon la revendication 2, **caractérisée par le fait que** les colorants directs cationiques répondent aux structures (I1), (I2), (I13) et (I29).

4. Composition selon la revendication 1, **caractérisée par le fait que** les colorants directs cationiques de formule (Il) sont choisis parmi les composés répondant aux structures (II1) à (II9) suivantes : 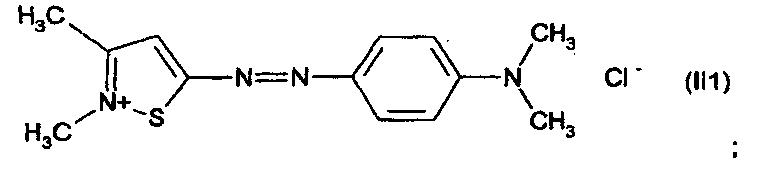 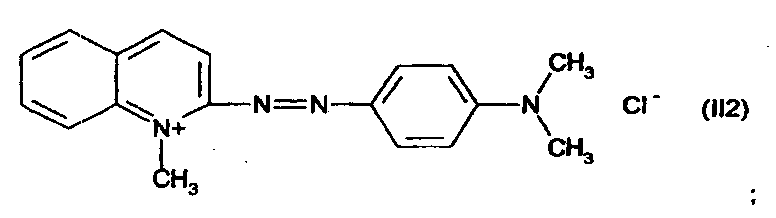 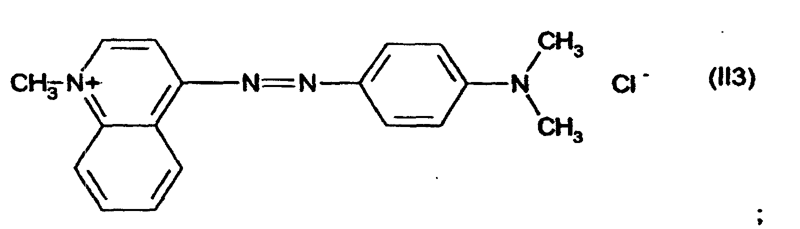 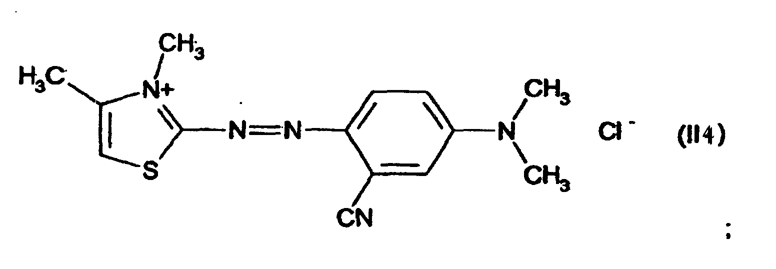 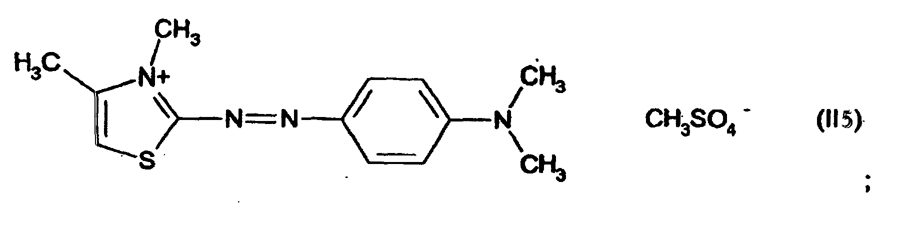 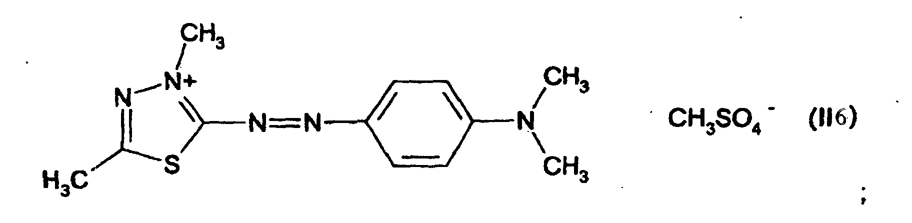 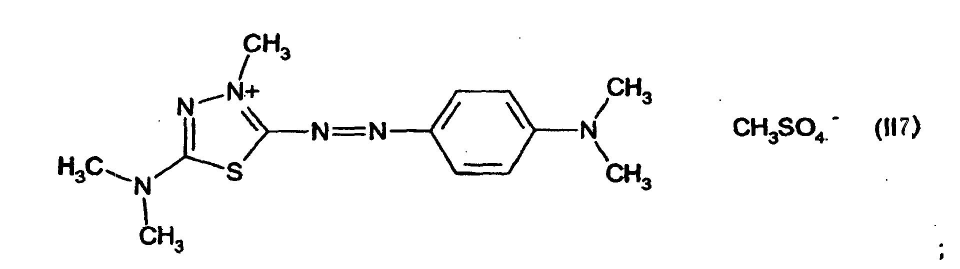 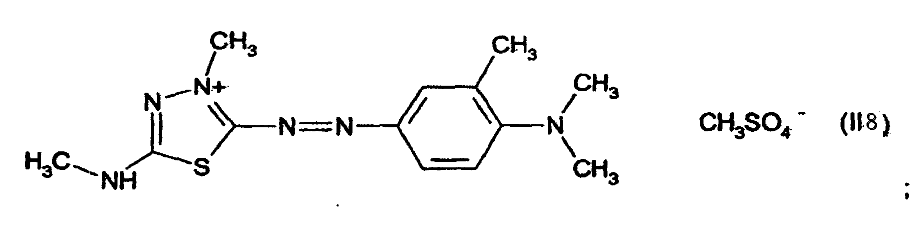 et

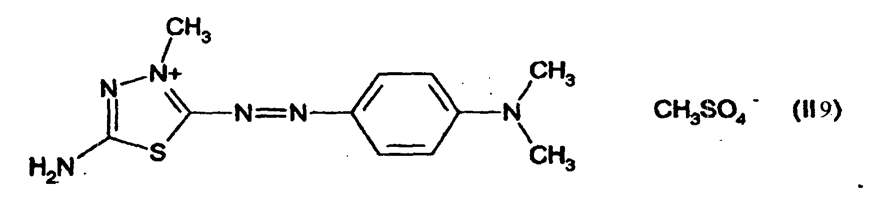

5. Composition selon la revendication 1, **caractérisée par le fait que** les colorants directs cationiques de formule (III) sont choisis parmi les composés répondant aux structures (III1) à (III18) suivantes : 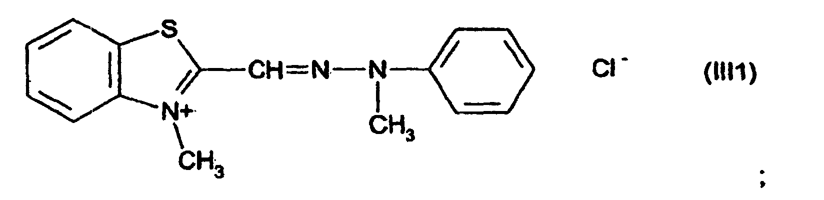 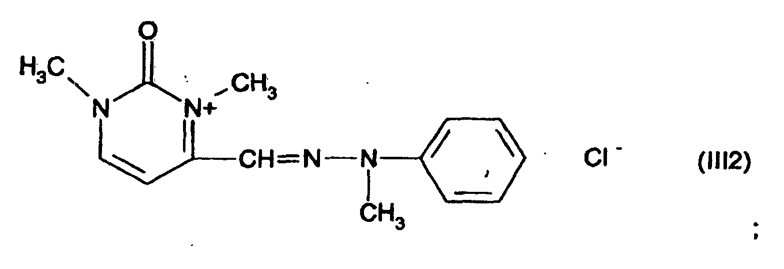 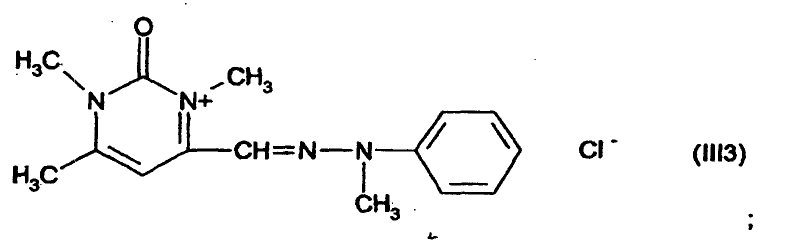 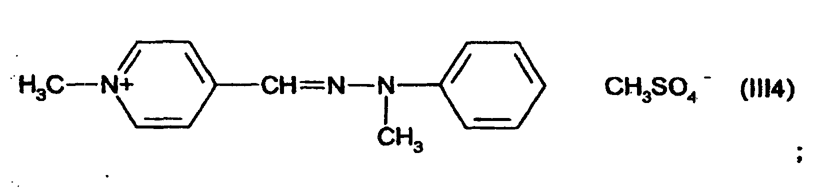 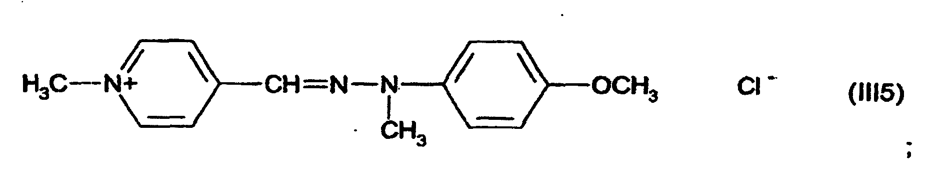 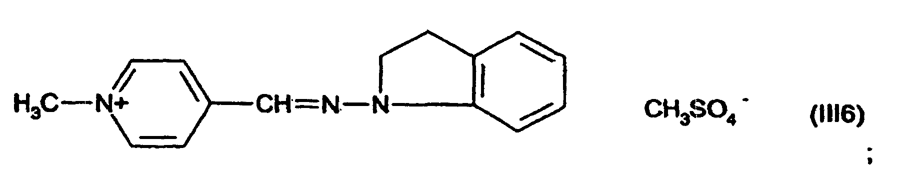 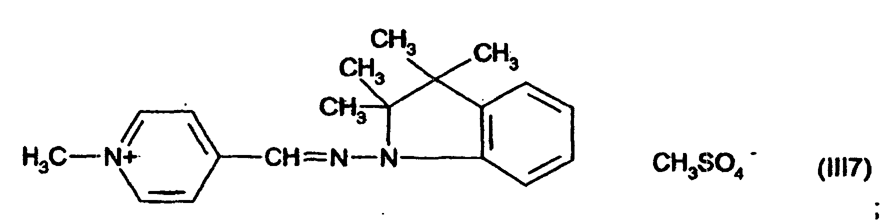 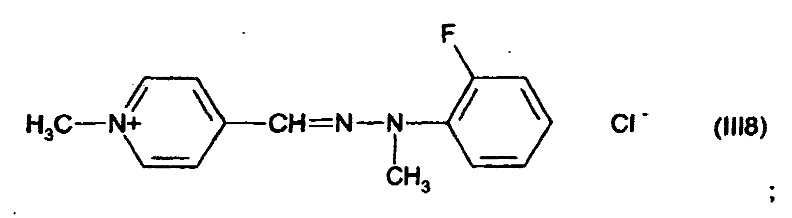 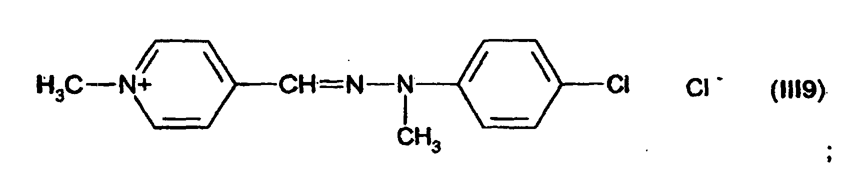 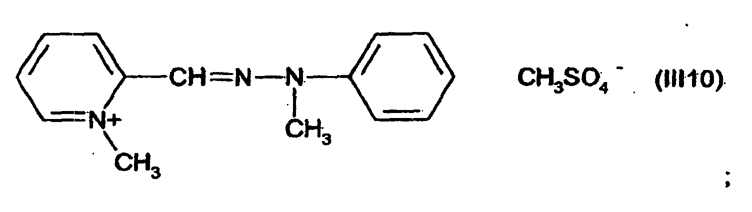 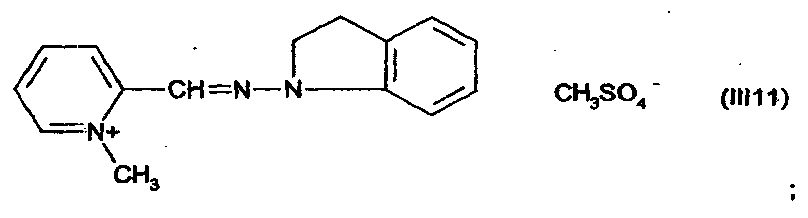 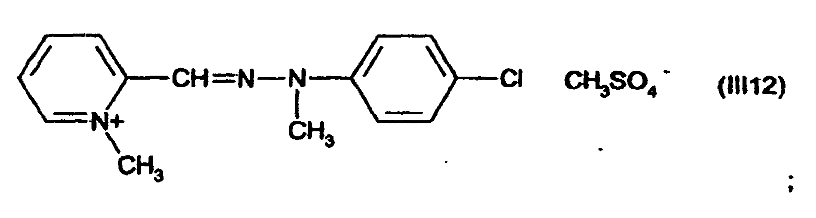 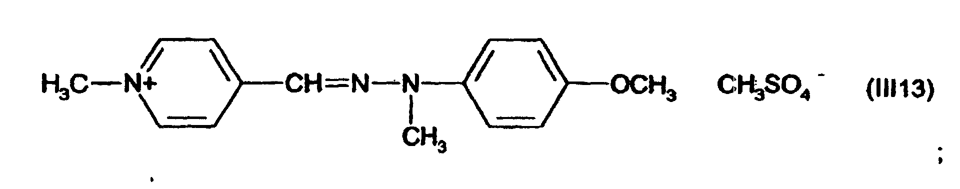 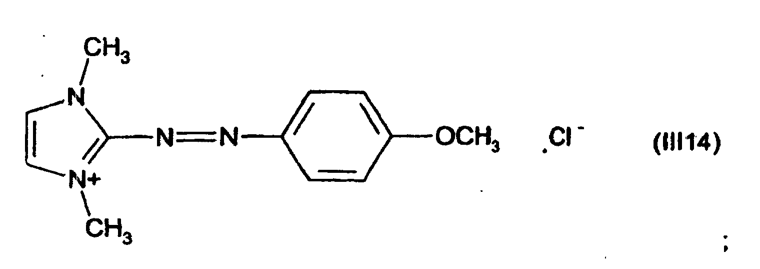 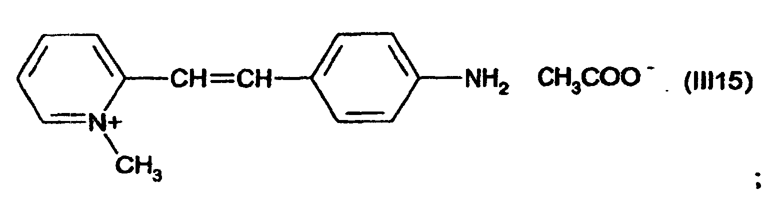 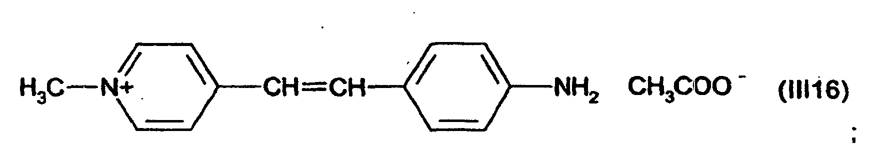 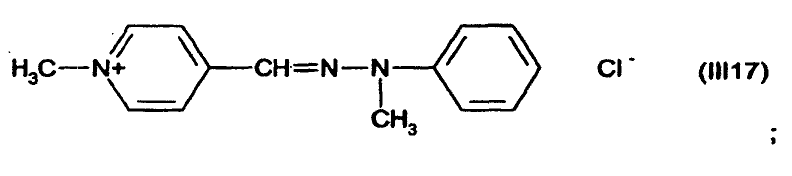 et

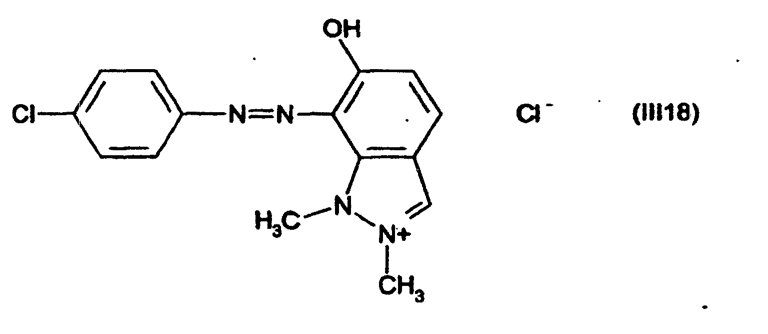

6. Composition selon la revendication 5, **caractérisée par le fait que** les colorants directs cationiques de formule (III) sont choisis parmi les composés répondant aux structures (III4), (III5) et (III13).

7. Composition selon la revendication 1, **caractérisée par le fait que** les colorants directs cationiques de formule (III') sont choisis parmi les composés répondant aux structures (III'1) à (III'3) suivantes : 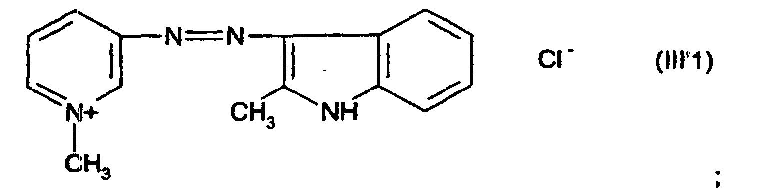 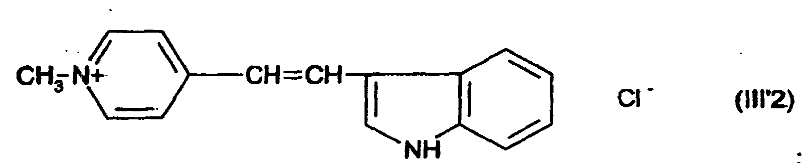 et

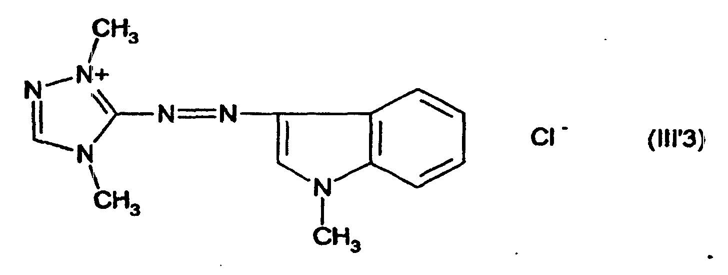

8. Composition selon l'une quelconque des revendications précédentes, **caractérisée par le fait que** le ou les colorants directs cationiques de formules (I), (II)_{,} (III) ou (III') représentent de 0,001 à 10 % en poids du poids total de la composition.

9. Composition selon la revendication 8, **caractérisée par le fait que** le ou les colorants directs cationiques de formules (I), (II), (III) ou (III') représentent de 0,005 à 5 % en poids du poids total de la composition.

10. Composition selon l'une quelconque des revendications précédentes, **caractérisée par le fait que** le polymère substantif cellulosique est un Polyquaternium 24.

11. Composition selon l'une quelconque des revendications 1 à 9, **caractérisée par le fait que** le polymère substantif du type copolymère d'halogénure de diméthyldiallylammonium et d'acide (méth)acrytique est un copolymère de chlorure de diméthyldiallylammonium et d'acide acrylique (80/20 en poids).

12. Composition selon l'une quelconque des revendications 1 à 9, **caractérisée par le fait que** les polymères substantifs du type homopolymère et copolymère d'halogénure de méthacryloyloxyéthyltriméthylammonium sont choisis parmi l'homopolymère poly(chlorure de méthacryloyloxyéthyltriméthylammonium) réticulé, en dispersion à 50% dans de l'huile minérale, le copolymère réticulé de l'acrylamide et du chlorure de méthacryloyloxyéthyltriméthylammonium (20/80 en poids), en dispersion à 50% dans de l'huile minérale, le méthosulfate du copolymère de méthacryloyloxyéthyltriméthylammonium et de méthacryloyl oxyéthyldiméthylacétylammonium.

13. Composition selon l'une quelconque des revendications 1 à 9, **caractérisée par le fait que** les polymères substantifs du type polymère de vinylpyrrolidone à motifs cationiques sont choisis parmi :
a) les polymères de vinylpyrrolidone comportant des motifs méthacrylate de diméthylaminoéthyle;
b) les polymères de vinylpyrrolidone comportant des motifs méthacrylamido propyltriméthylammonium;
c) les polymères de vinylpyrrolidone comportant des motifs méthylvinyl imidazolium.

14. Composition selon l'une quelconque des revendications 1 et 10 à 13, **caractérisée par le fait que** le ou les polymères substantifs (ii) représentent de 0,01 à 10 % en poids du poids total de la composition.

15. Composition selon la revendication 14, **caractérisée par le fait que** le ou les polymères substantifs (ii) représentent de 0,1 à 5% en poids du poids total de la composition.

16. Composition selon l'une quelconque des revendications précédentes, **caractérisée par le fait que** le milieu approprié pour la teinture (ou support) est constitué par de l'eau ou par un mélange d'eau et d'au moins un solvant organique.

17. Composition selon l'une quelconque des revendications précédentes, **caractérisée par le fait qu'**elle présente un pH compris entre 2 et 11, et de préférence entre 5 et 10.

18. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

19. Composition selon la revendication 18, **caractérisée par le fait que** la ou les bases d'oxydation représentent 0,0005 à 12 % en poids du poids total de la composition tinctoriale.

20. Composition selon la revendication 19, **caractérisée par le fait que** la ou les bases d'oxydation représentent 0,005 à 6 % en poids du poids total de la composition tinctoriale.

21. Composition selon l'une quelconque des revendications 18 à 20, **caractérisée par le fait qu'**elle renferme un ou plusieurs coupleurs choisis parmi les métaphénylènediamines, les méta-aminophénols, les nnétadiphénois et les coupleurs hétérocycliques.

22. Composition selon la revendication 21, **caractérisée par le fait que** le ou les coupleurs représentent de 0,0001 à 10 % en poids du poids total de la composition tinctoriale.

23. Composition selon la revendication 22, **caractérisée par le fait que** le ou les coupleurs représentent de 0,005 à 5 % en poids du poids total de la composition tinctoriale.

24. Composition selon l'une quelconque des revendications précédentes, **caractérisée par le fait qu'**elle est destinée à la teinture d'oxydation ou la teinture directe éclaircissante et qu'elle renferme au moins un agent oxydant.

25. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 24, pendant un temps suffisant pour développer la coloration désirée, après quoi on rince, on lave éventuellement au shampooing, on rince à nouveau et on sèche.

26. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 24, pendant un temps suffisant pour développer la coloration désirée, sans rinçage final.

27. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère substantif (ii) tel que défini dans les revendications précédentes.

28. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère substantif (ii) tel que défini dans les revendications précédentes.

29. Dispositif à plusieurs compartiments ou "kit" de teinture à plusieurs compartiments, **caractérisé par le fait qu'**un premier compartiment renferme la composition (A1) ou (A2) telle que définie à la revendication 27 ou 28 et un second compartiment renferme la composition (B1) ou (B2) telle que définie à la revendication 27 ou 28.

## Patentansprüche

1. Zusammensetzung zum Färben von Keratinfasern, insbesondere menschlichen Keratinfasern und ganz besonders des Haares, die in einem zum Färben geeigneten Medium enthält: **(i)** mindestens einen kationischen Direktfarbstoff der folgenden Formeln (I), (II), (III) oder (III'): 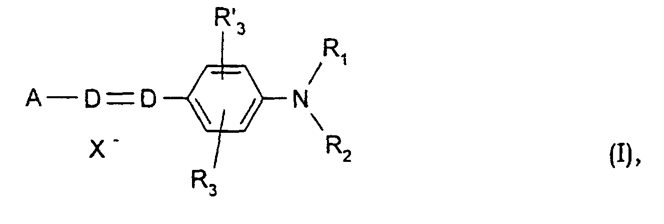 wobei in der Formel (I) bedeuten:
**D** ein Stickstoffatom oder die Gruppe -CH,
**R**_{**1**} und **R**_{**2**}**,** die gleich oder verschieden sind, ein Wasserstoffatom; eine C₁₋₄-Alkylgruppe, die mit einer Gruppe -CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁₋₄-Alkylgruppen substituiert sein kann; oder eine 4'-Aminophenylgruppe,
**R**_{**3**} und **R'**_{**3**}**,** die identisch oder voneinander verschieden sind, ein Wasserstoffatom, ein Halogenatom, das unter Chlor, Brom, Iod und Fluor ausgewählt ist, Cyano, C₁₋₄-Alkoxy oder Acetyloxy,
**X** ein Anion, das vorzugsweise unter Chlorid, Methylsulfat und Acetat ausgewählt ist,
**A** eine Gruppe, die unter den folgenden Strukturen A1 bis A18 ausgewählt ist: 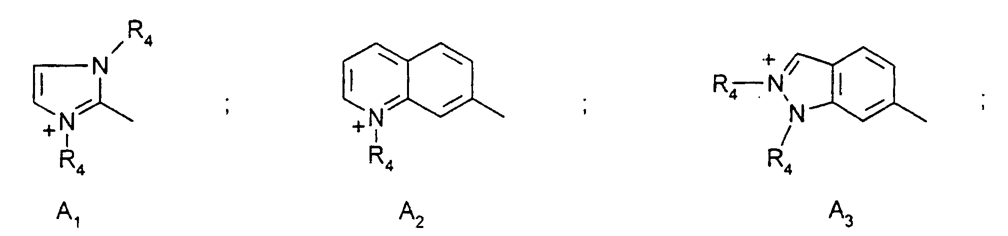 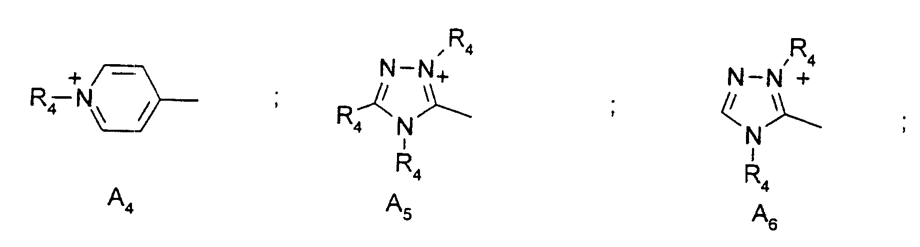 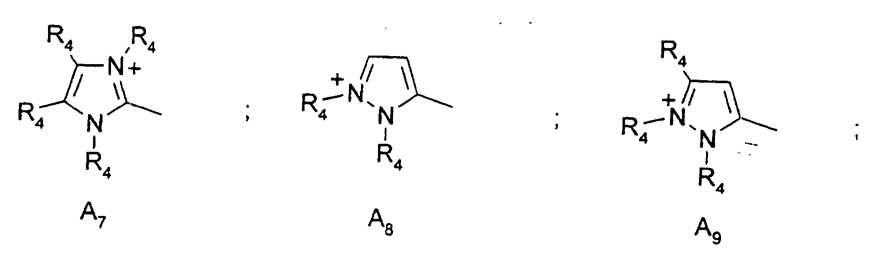 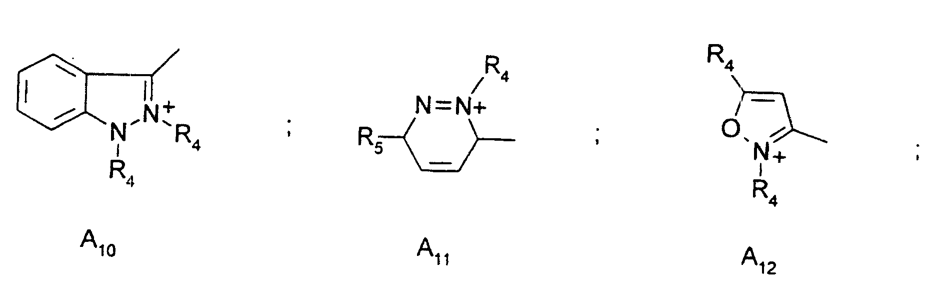 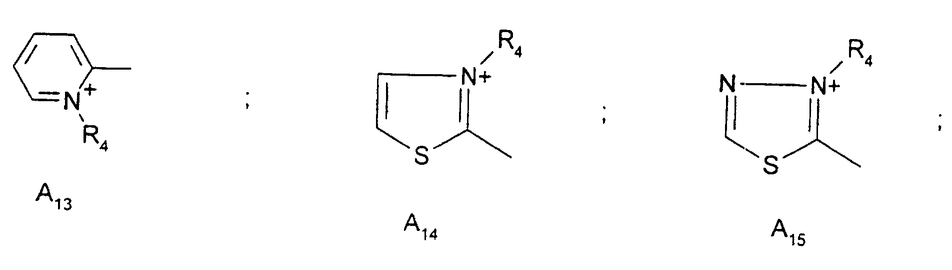 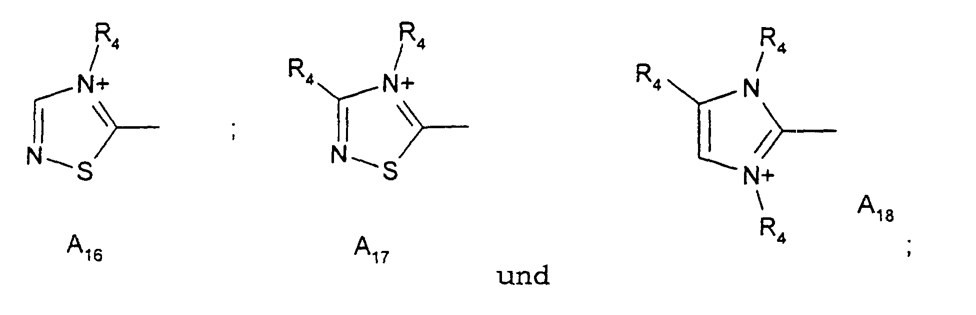 worin die Gruppe R₄ eine C₁₋₄-Alkylgruppe bedeutet, die mit einer Hydroxygruppe substituiert sein kann, und R₅ eine C₁₋₄-Alkoxygruppe bedeutet, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
sowie die folgenden Verbindungen der Formel (I): 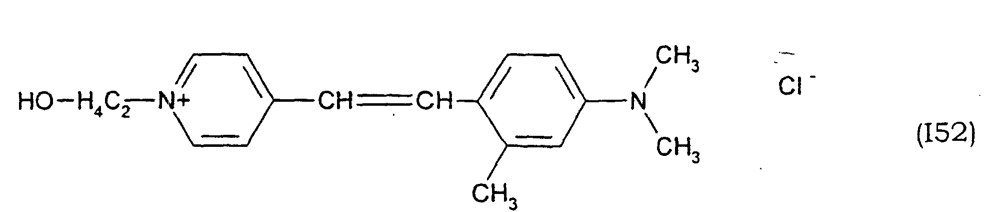 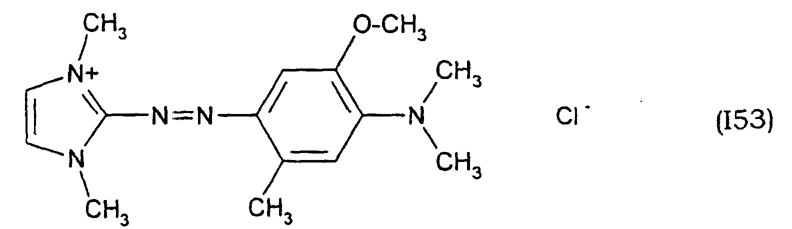 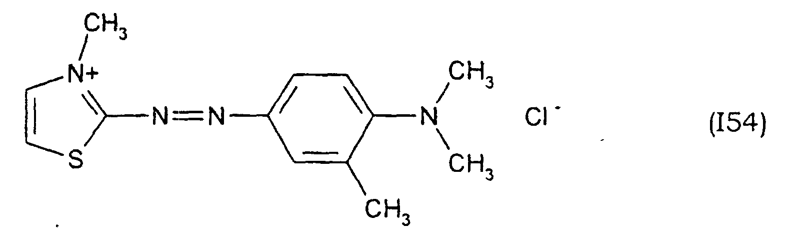 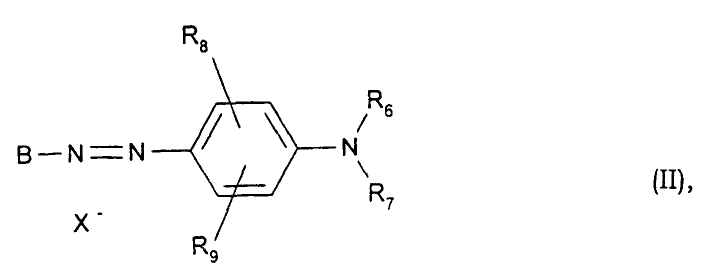 worin bedeuten:
**R**_{**6**} ein Wasserstoffatom oder eine C₁₋₄-Alkylgruppe,
**R**_{**7**} ein Wasserstoffatom, eine Alkylgruppe, die mit der Gruppe - CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
**R**_{**8**} und **R**_{**9**}**,** die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe - CN,
**X** ein Anion, das vorzugsweise unter Chlorid, Methylsulfat und Acetat ausgewählt ist,
**B** eine Gruppe, die unter den folgenden Strukturen B1 bis B6 ausgewählt ist: 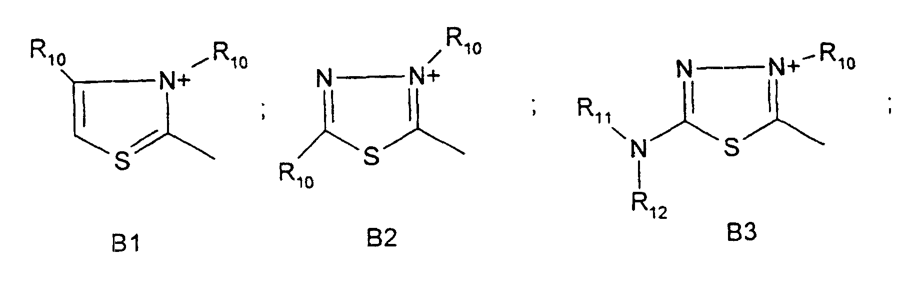 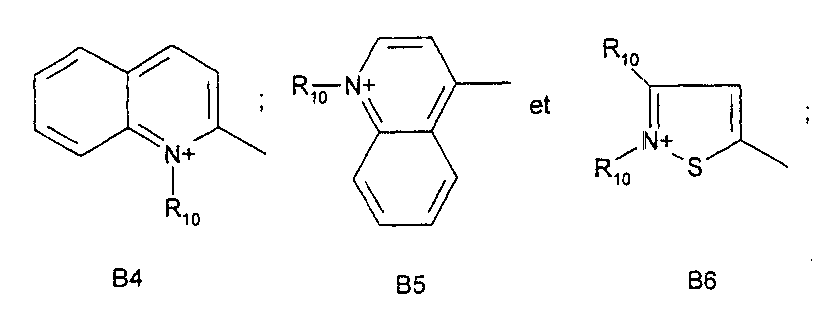 worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten; 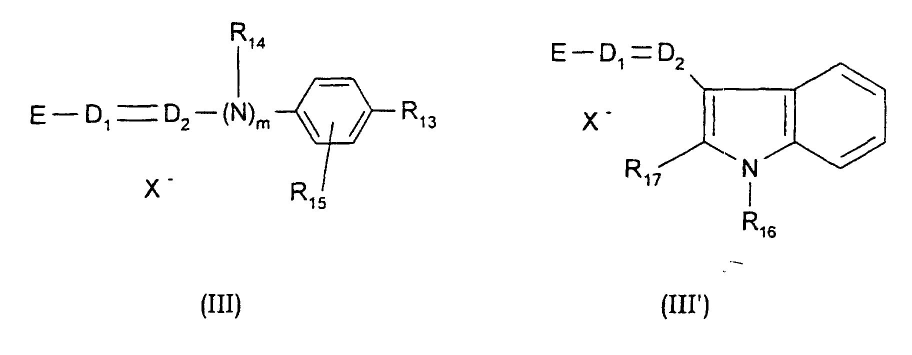
worin bedeuten:
**R**_{**13**} ein Wasserstoffatom, eine C₁₋₄-Alkoxygruppe, ein Halogenatom, wie Brom, Chlor, Iod oder Fluor, oder eine Aminogruppe, **R**_{**14**} ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig ist und/oder mit einer oder mehreren C₁₋₄-Alkylgruppen substituiert ist;
**R**_{**15**} ein Wasserstoffatom oder ein Halogenatom, wie Brom, Chlor, Iod oder Fluor,
**R**_{**16**} und **R**_{**17**}**,** die identisch oder voneinander verschieden sind, Wasserstoff oder C₁₋₄-Alkyl,
**D**_{**1**} und **D**_{**2**}**,** die gleich oder verschieden sind, ein Stickstoffatom oder die Gruppe -CH,
**m** = 0 oder 1, mit der Maßgabe, dass die Gruppen D₁ und D₂ gleichzeitig -CH bedeuten und m = 0, wenn R₁₃ eine unsubstituierte Aminogruppe ist,
**X**^{**-**} ein Anion, das vorzugsweise unter Chlorid, Methylsulfat und Acetat ausgewählt ist,
**E** eine Gruppe, die unter den folgenden Strukturen E1 bis E8 ausgewählt ist: 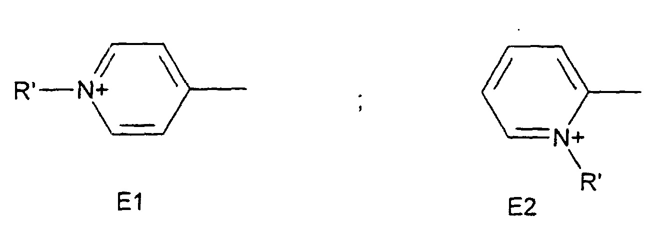 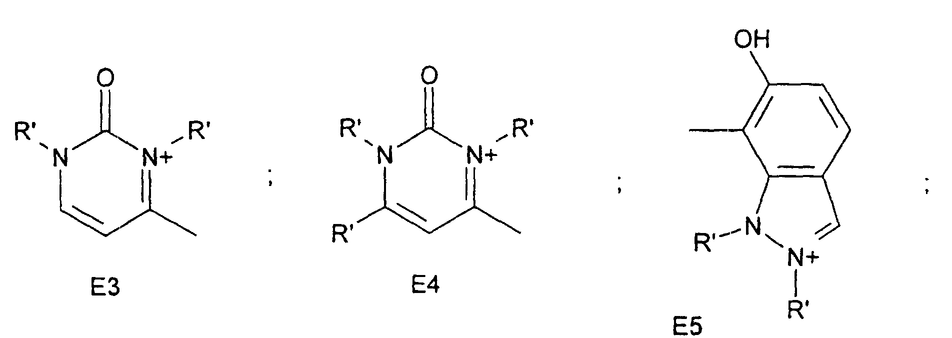 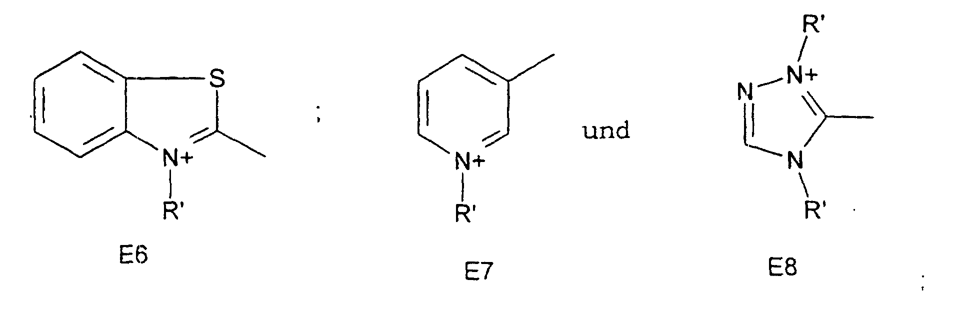 wobei R' eine C₁₋₄-Alkylgruppe bedeutet;
wenn. m = 0 und D₁ ein Stickstoffatom bedeutet, kann die Gruppe E auch eine Gruppe der folgenden Struktur E9 sein: 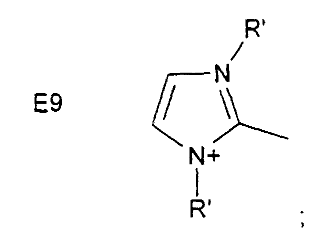 wobei R' eine C₁₋₄-Alkylgruppe bedeutet;
wobei die Zusammensetzung **dadurch gekennzeichnet ist, dass** sie ferner **(ii)** mindestens ein kationisches oder amphoteres substantives Polymer enthält, das unter den folgenden Polymeren ausgewählt ist:
1/ kationischen Cellulosederivaten, wobei Polyquaternium 10 ausgenommen ist;
2 / Dimethyldiallylammoniumhalogenid/(Meth)acrylsäure-Copolymeren;
3 / Methacryloyloxyethyltrimethylammoniumhalogenid-Homopolymeren und Methacryloyloxyethyltrimethylammoniumhalogenid-Copolymeren;
4/ quartären Polyammoniumpolymeren, die ausgewählt sind unter:
- Polymeren, die aus wiederkehrenden Einheiten der folgenden Formel (IV) bestehen: 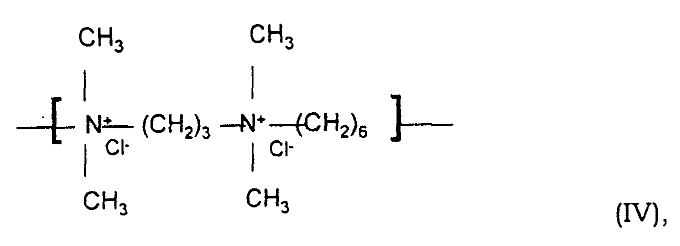
- Polymeren, die aus wiederkehrenden Einheiten der folgenden Formel (V) bestehen: 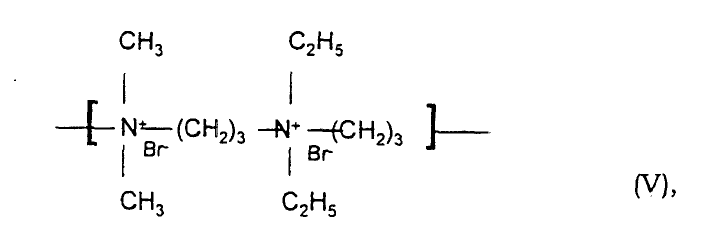
- Polymeren, die aus wiederkehrenden Einheiten der folgenden Formel (VI) bestehen: 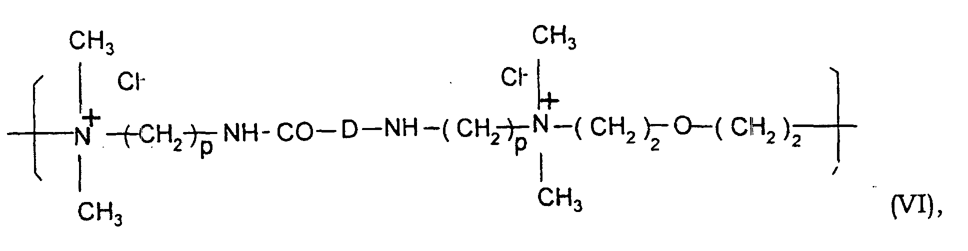 worin p eine ganze Zahl von etwa 1 bis 6 bedeutet und D nicht vorhanden sein kann oder einen Gruppe -(CH₂)ᵣ-COist, wobei r 4 oder 7 bedeutet,
5/ Vinylpyrrolidon-Copolymeren mit kationischen Einheiten; und
6/ deren Gemischen.

2. Zusammensetzung nach Anspruch 1, **dadurch gekennzeichnet, dass** die kationischen Direktfarbstoffe der Formel (I) unter den Verbindungen der folgenden Strukturen (I1) bis (I51) ausgewählt sind:

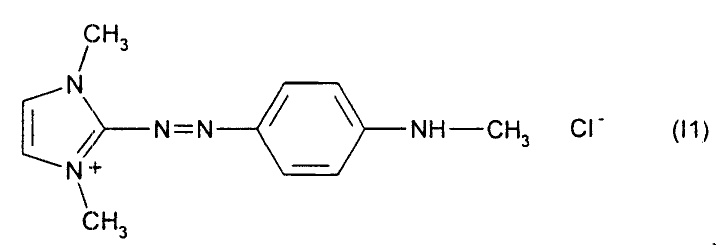

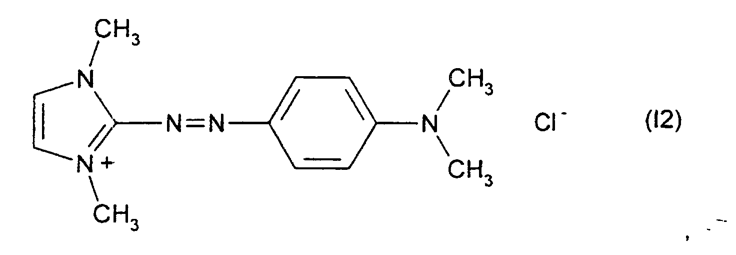

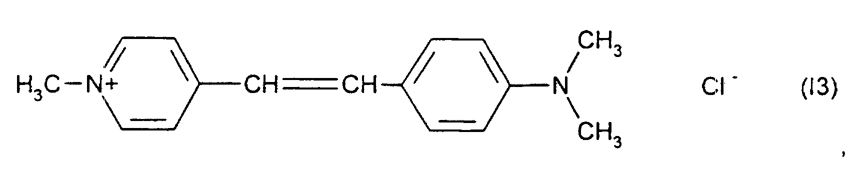

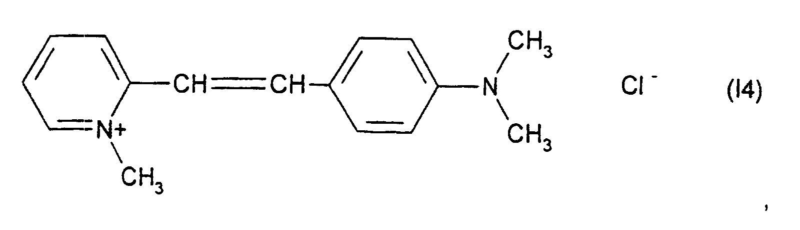

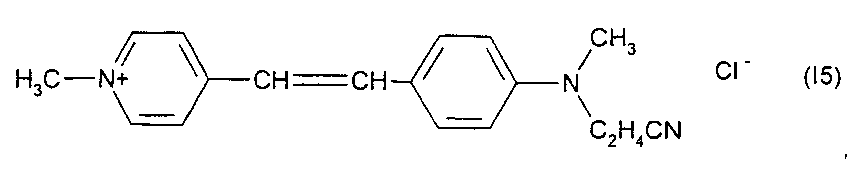

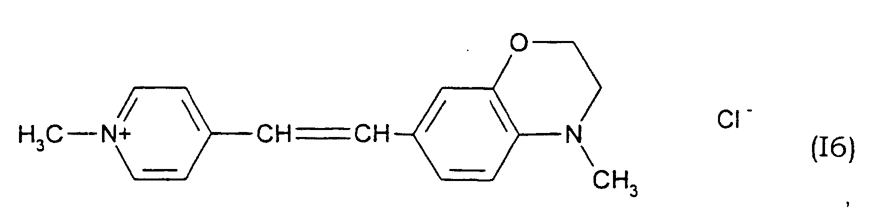

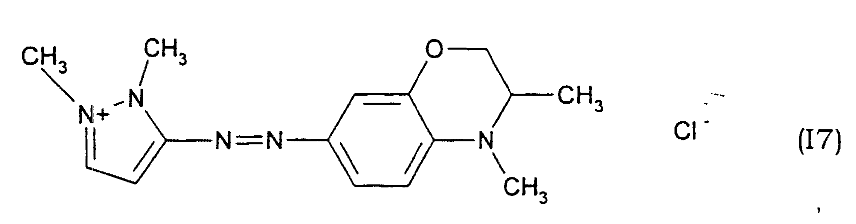

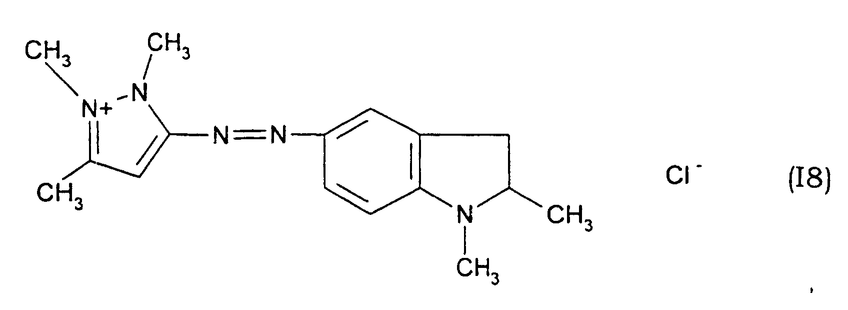

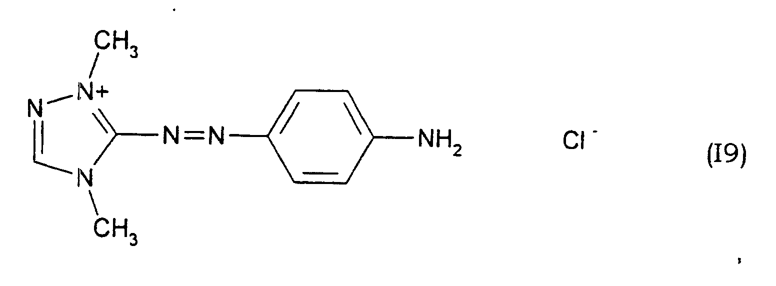

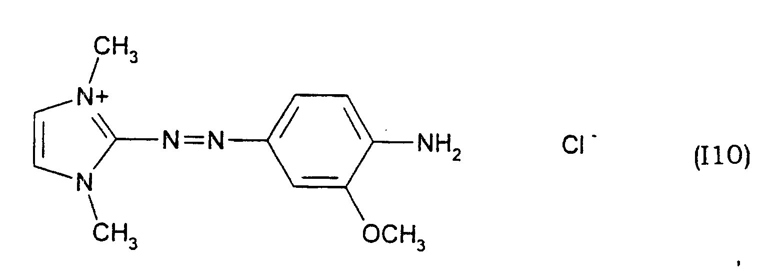

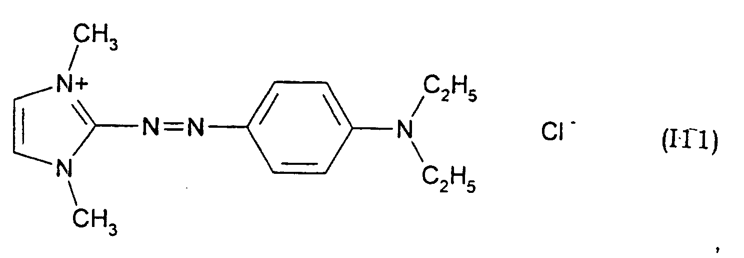

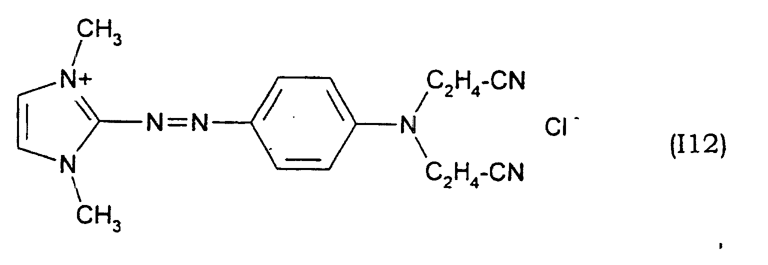

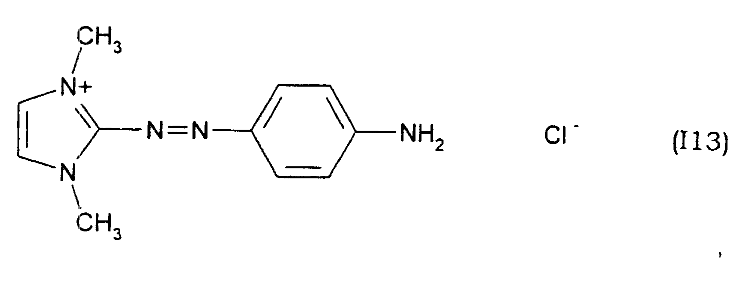

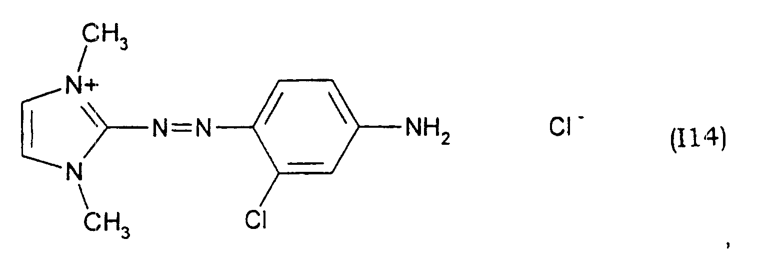

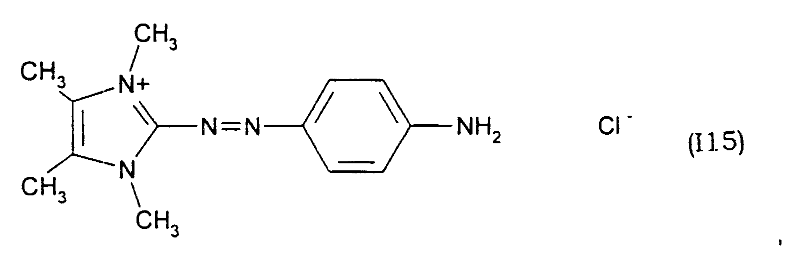

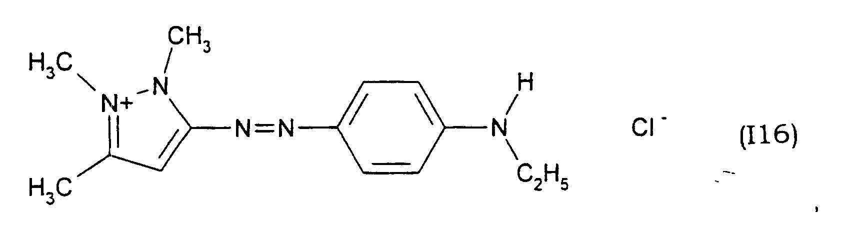

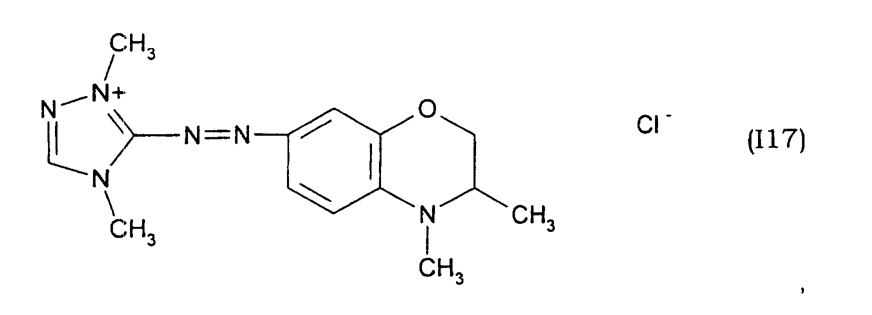

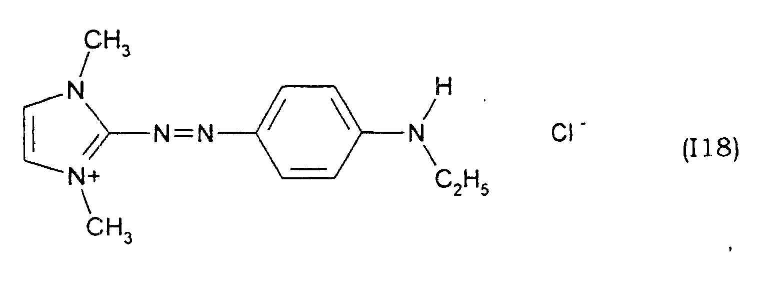

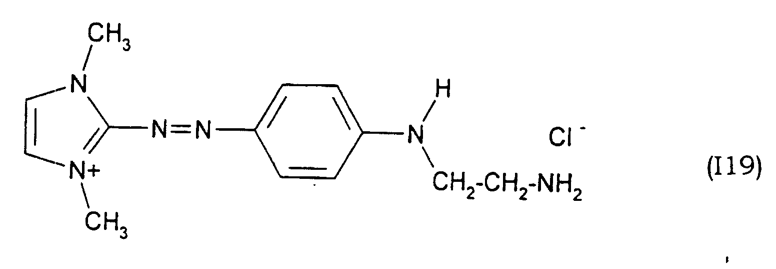

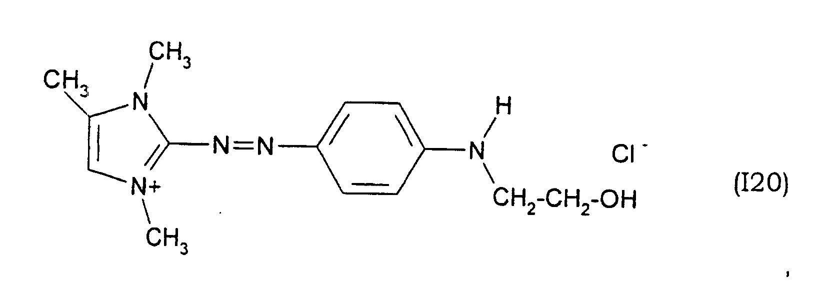

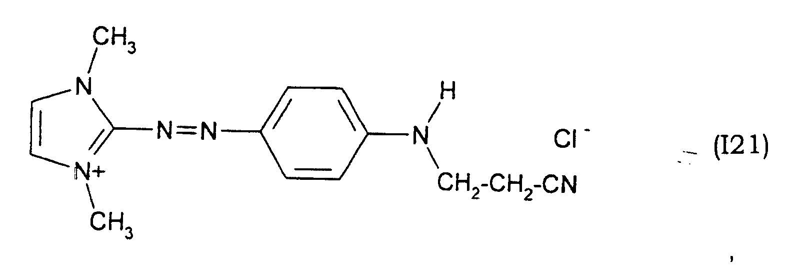

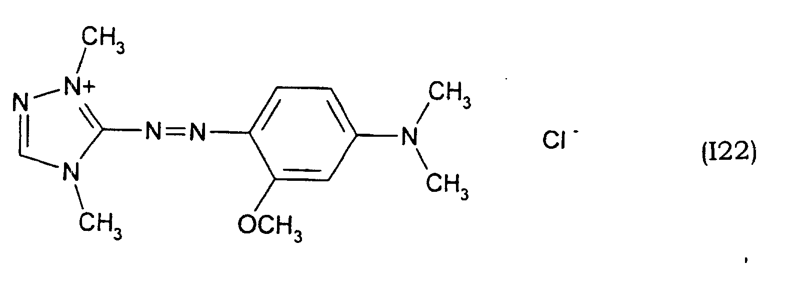

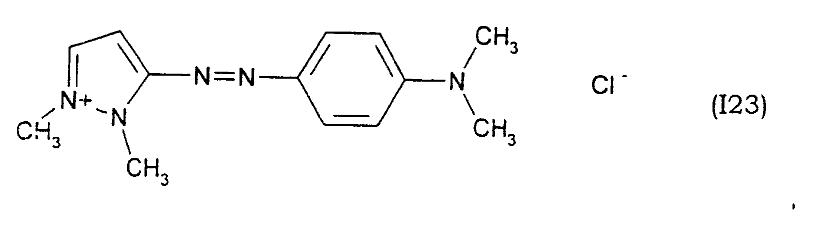

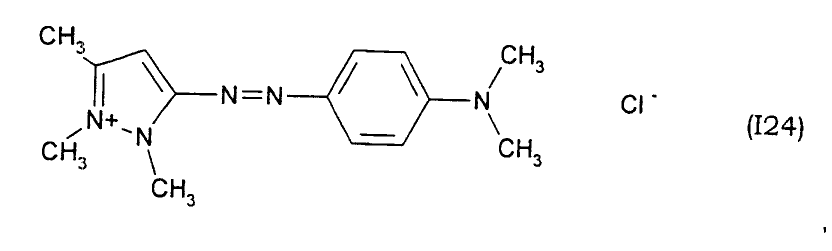

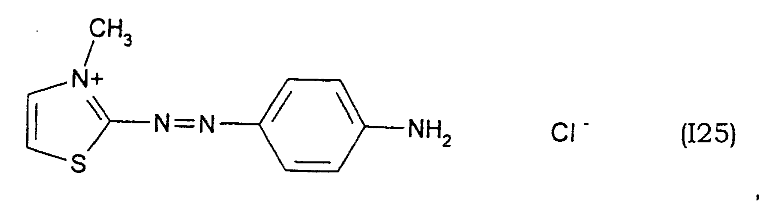

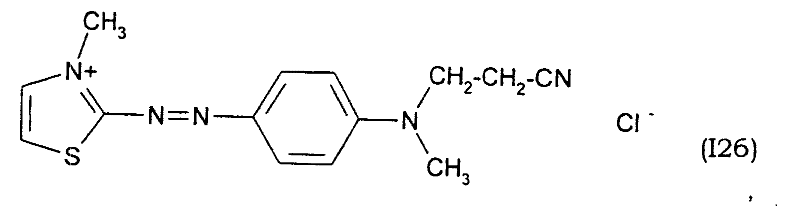

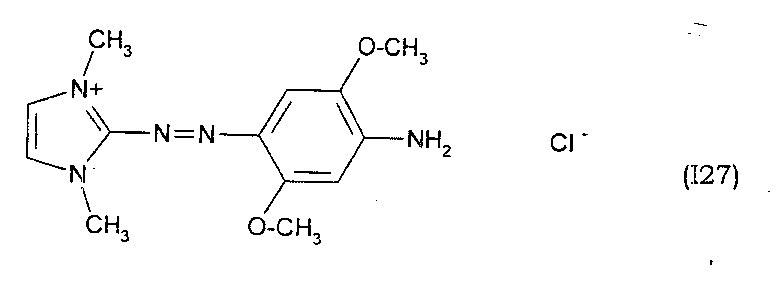

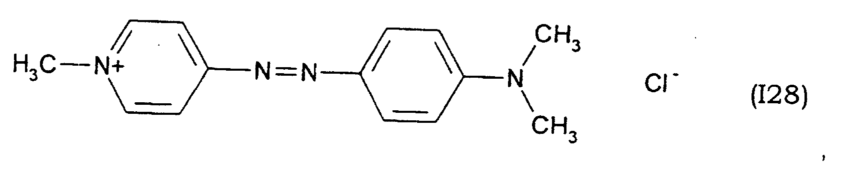

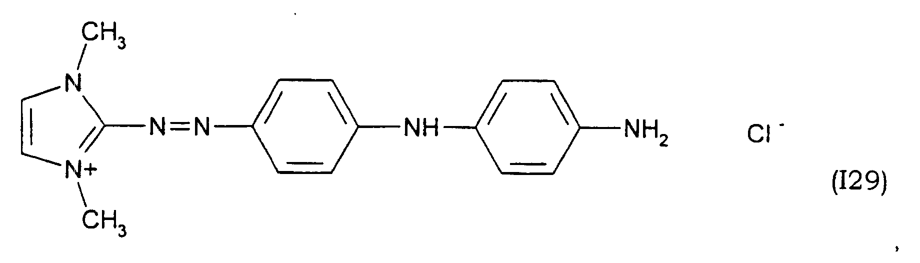

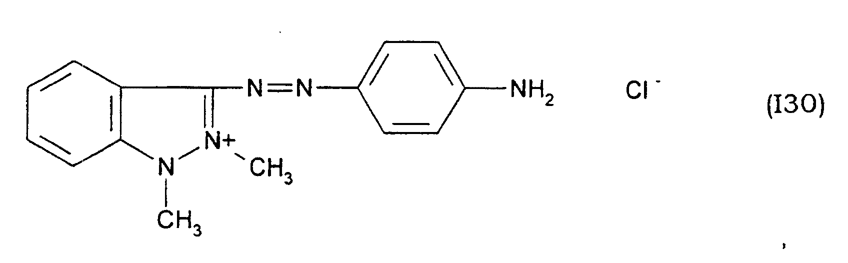

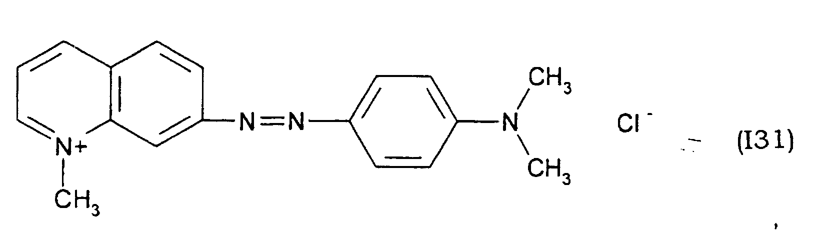

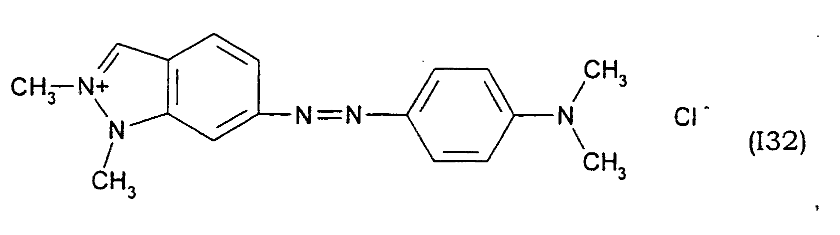

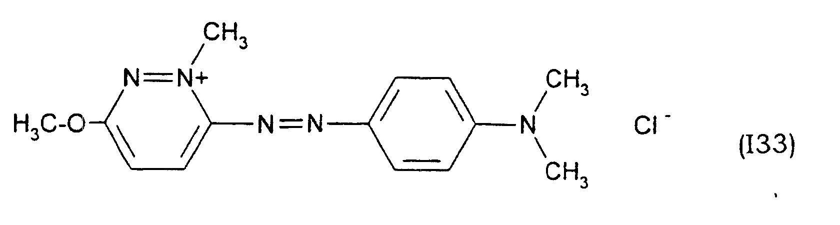

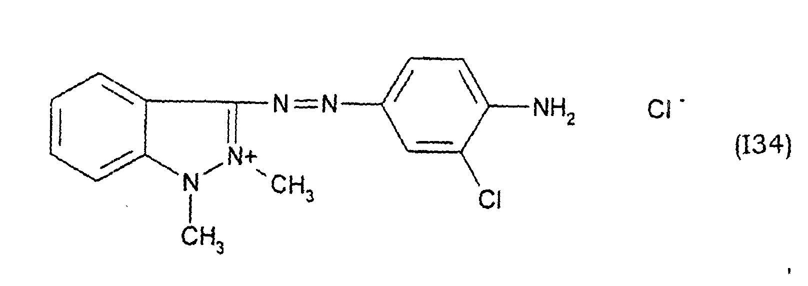

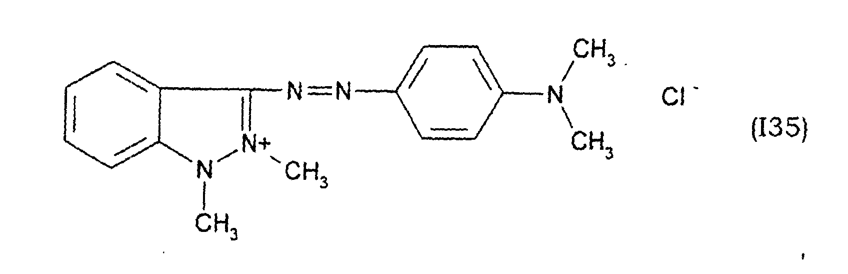

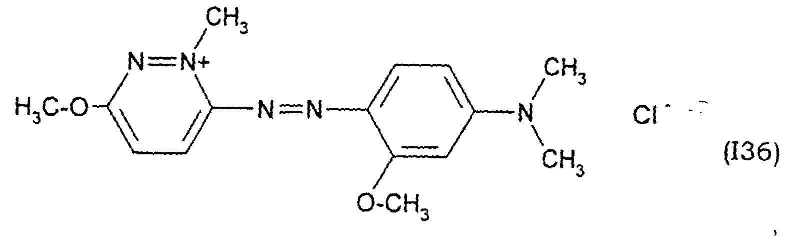

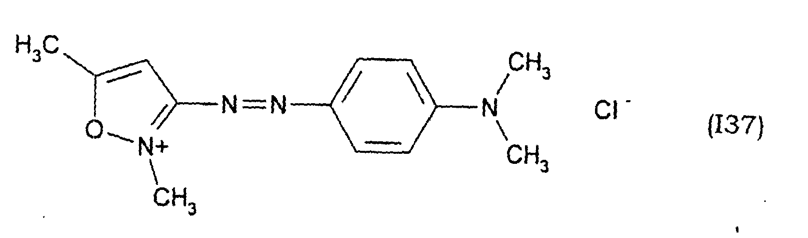

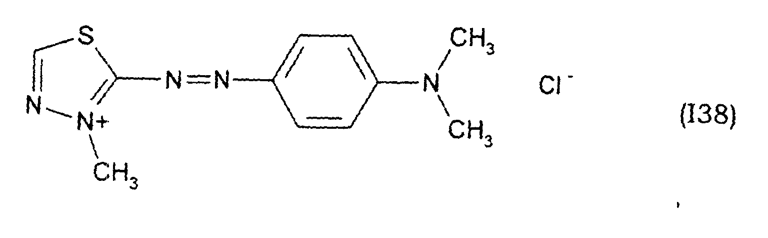

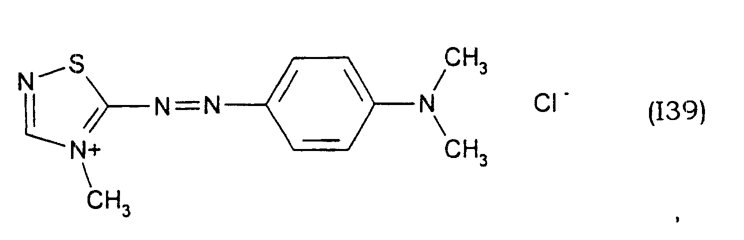

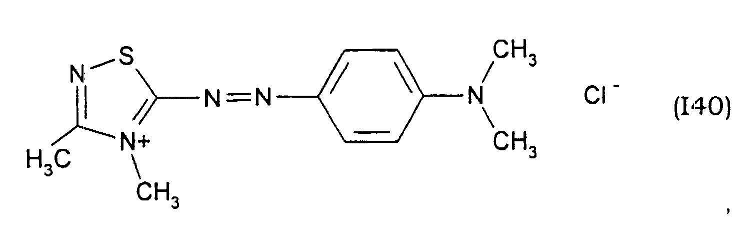

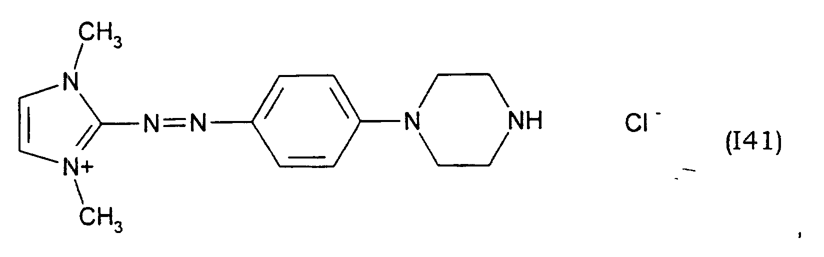

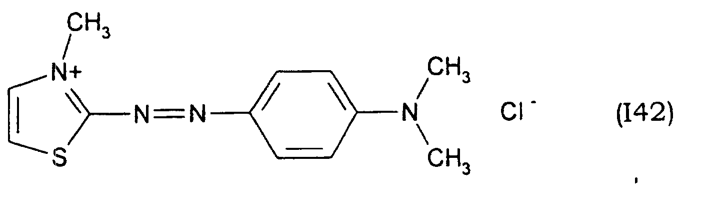

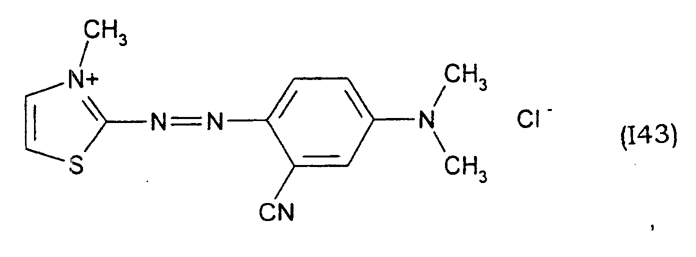

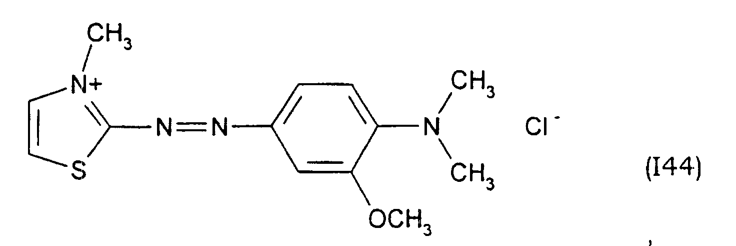

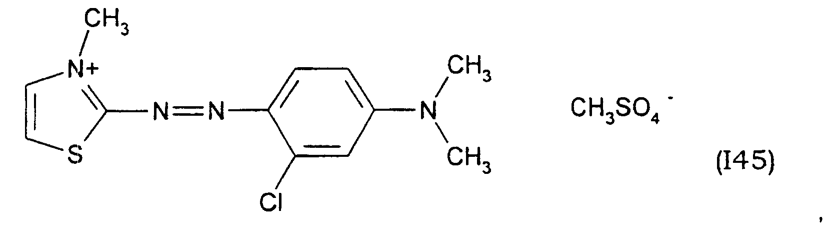

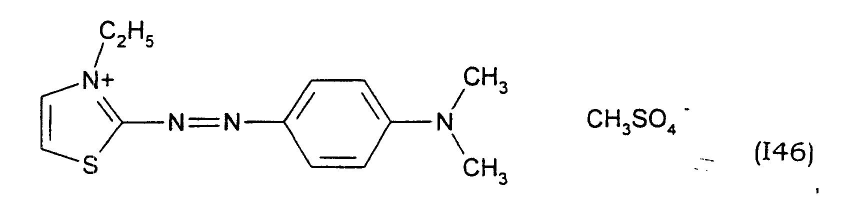

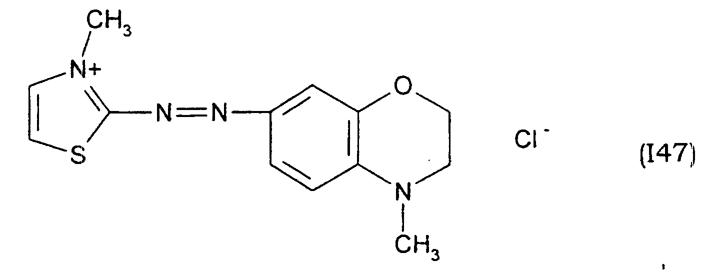

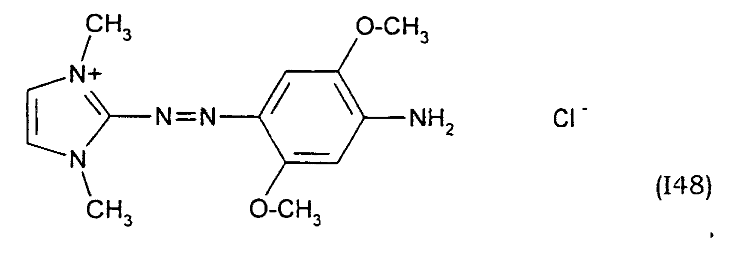

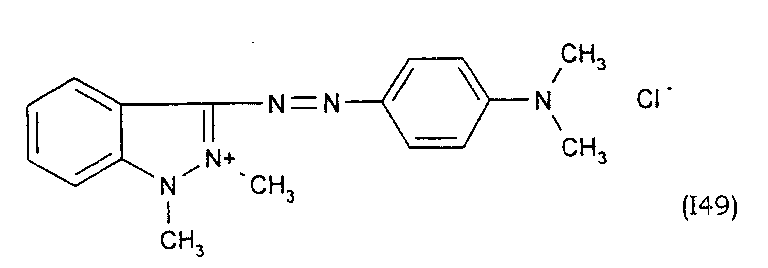

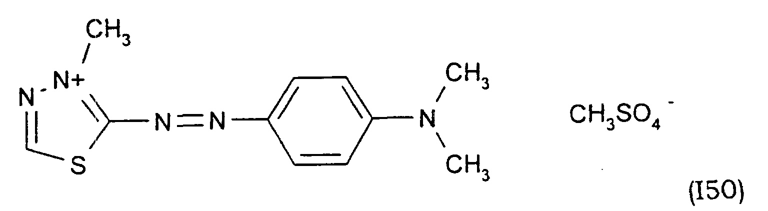

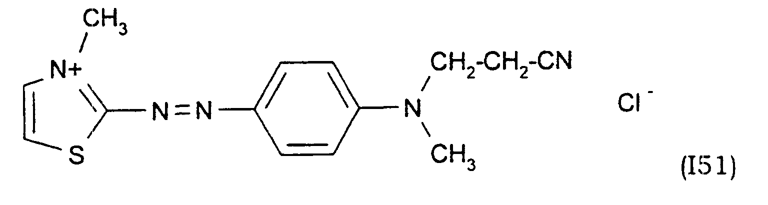

3. Zusammensetzung nach Anspruch 2, **dadurch gekennzeichnet, dass** die kationischen Direktfarbstoffe den Strukturen (I1), (I2), (I13) und (I29) entsprechen.

4. Zusammensetzung nach Anspruch 1, **dadurch gekennzeichnet, dass** die kationischen Direktfarbstoffe der Formel (II) unter den Verbindungen der folgenden Strukturen (II1) bis (II9) ausgewählt sind:

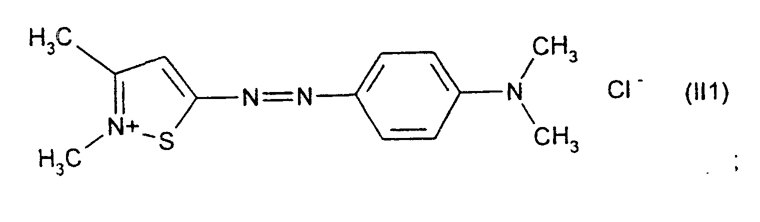

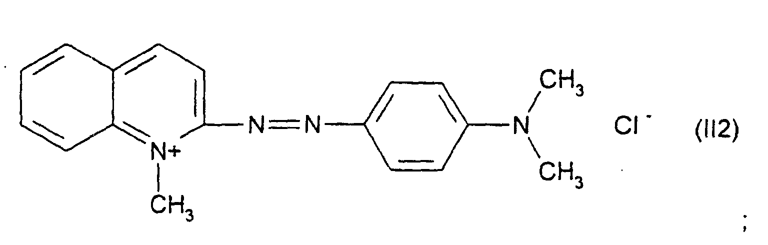

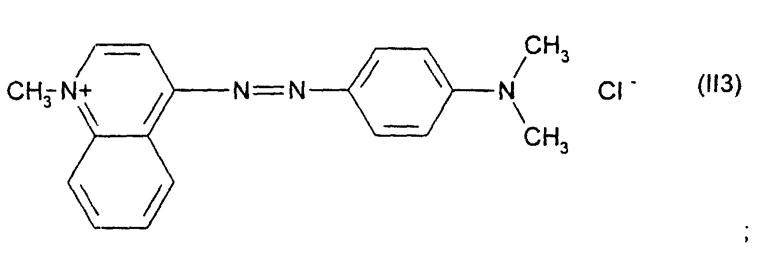

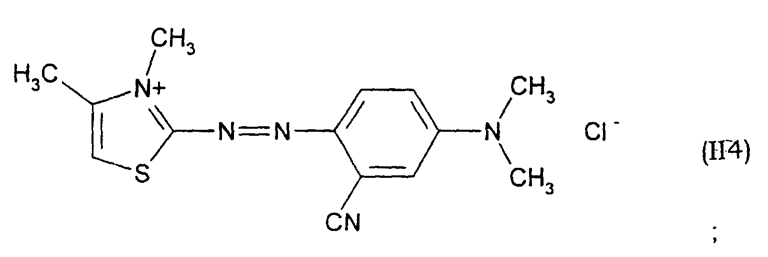

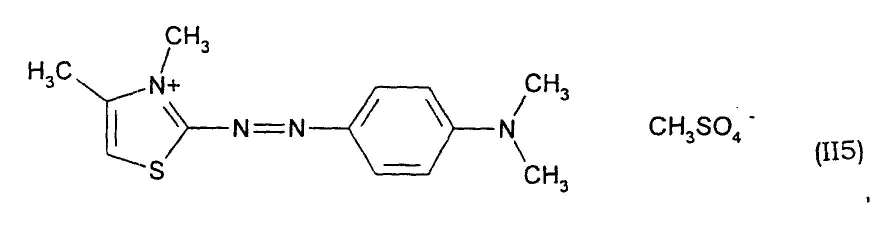

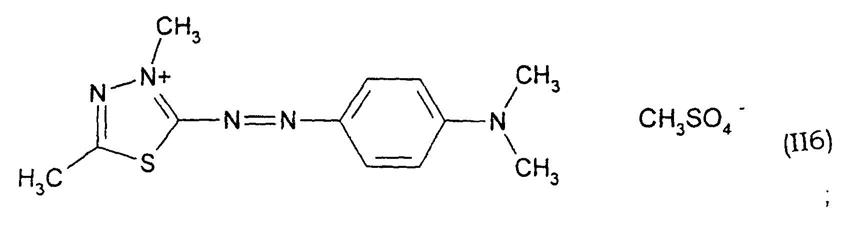

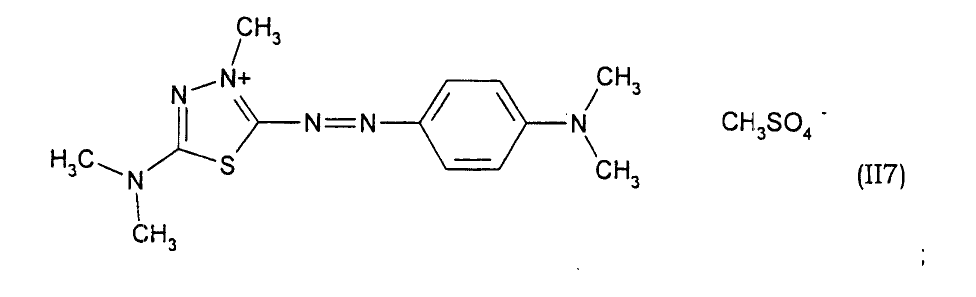

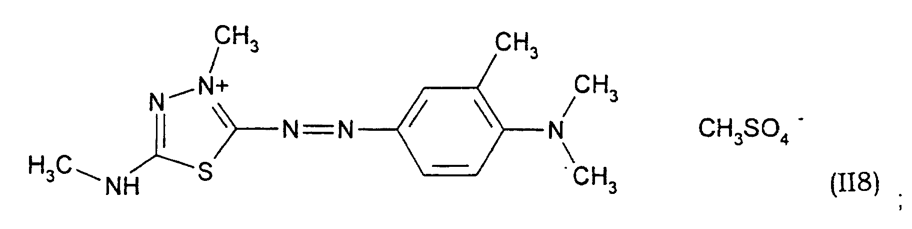

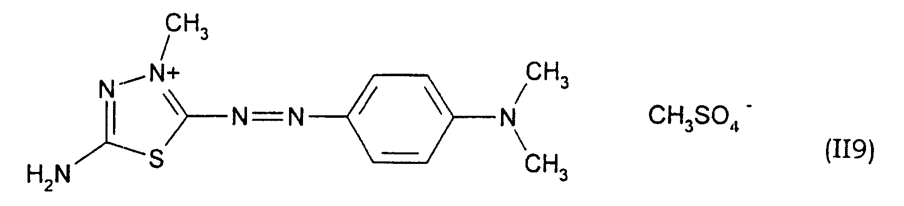

5. Zusammensetzung nach Anspruch 1, **dadurch gekennzeichnet, dass** die kationischen Direktfarbstoffe der Formel (III) unter den Verbindungen der folgenden Strukturen (III1) bis (III18) ausgewählt sind: 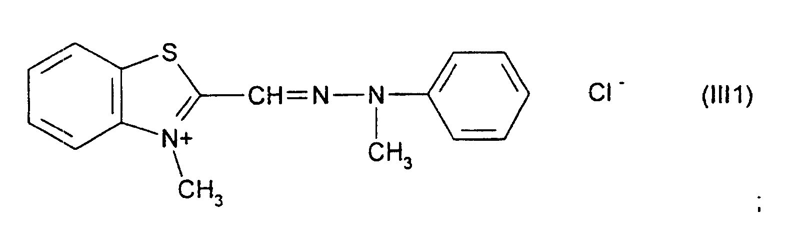 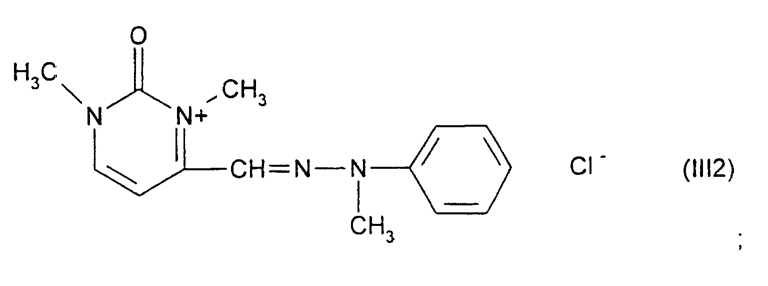 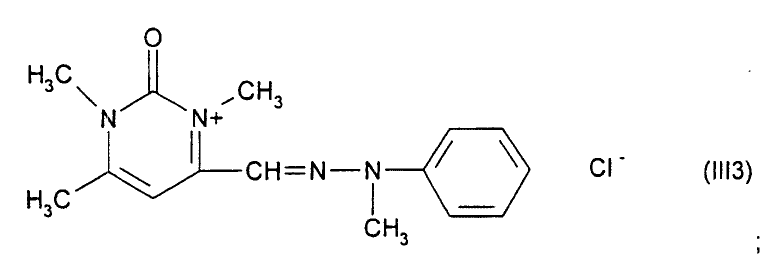 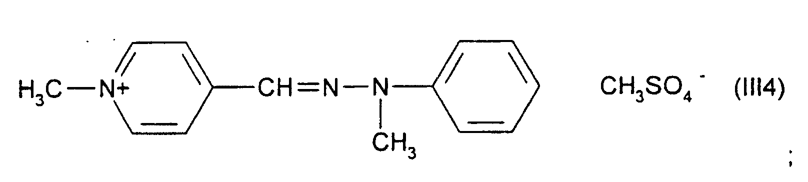 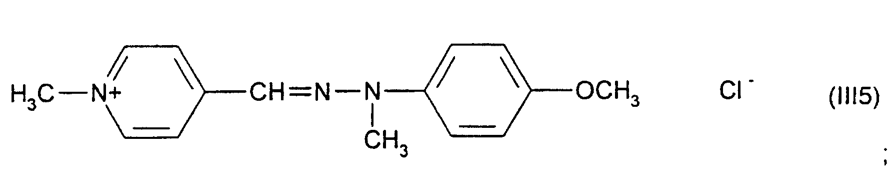 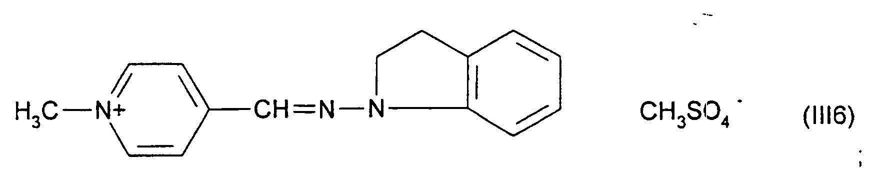 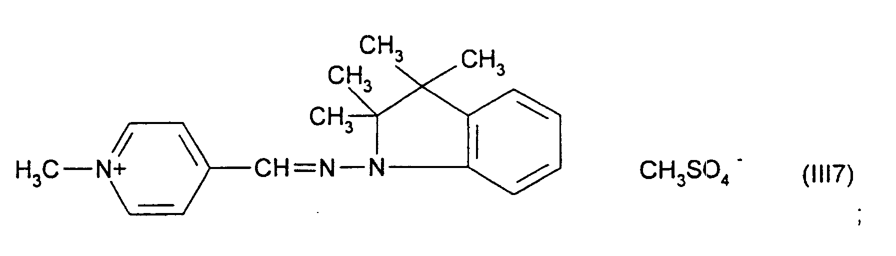 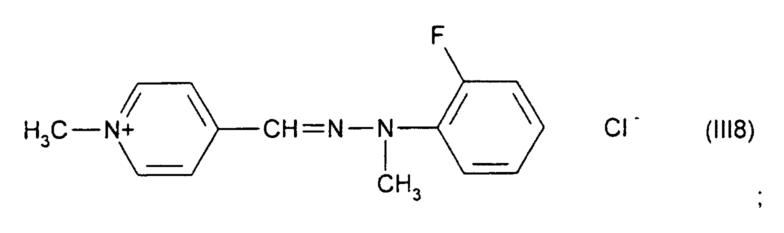 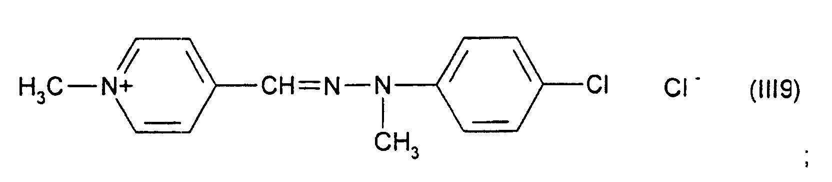 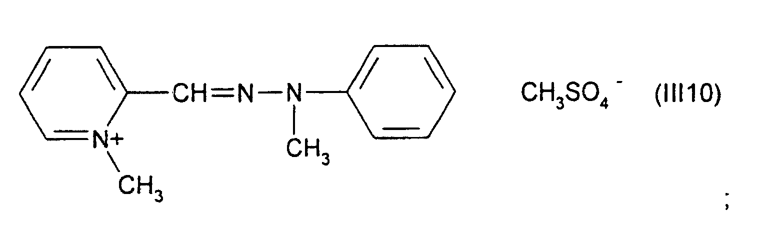 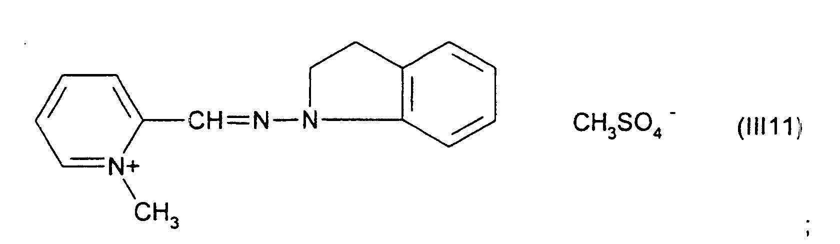 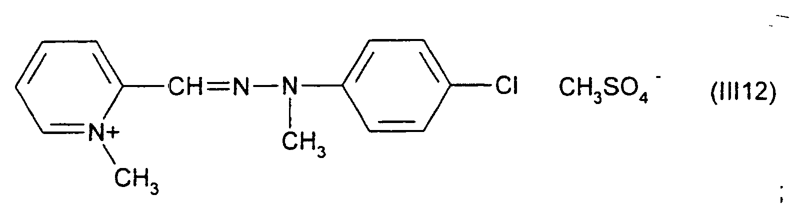 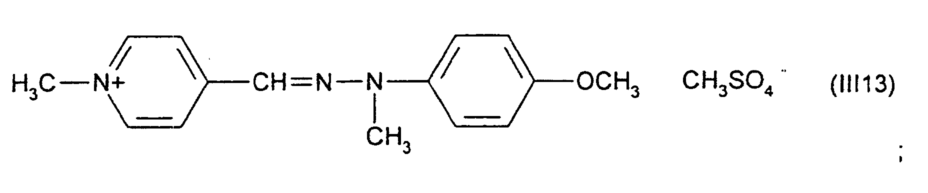 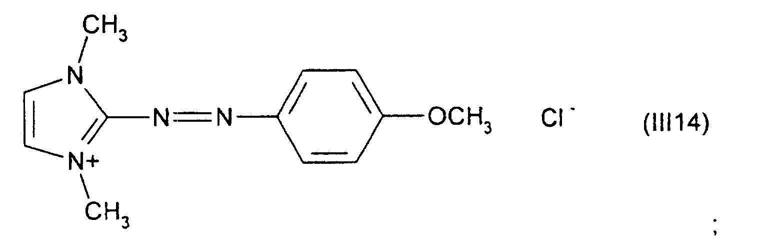 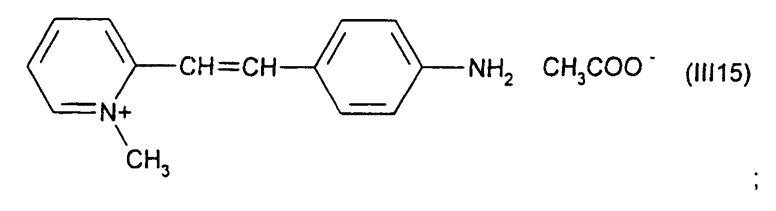 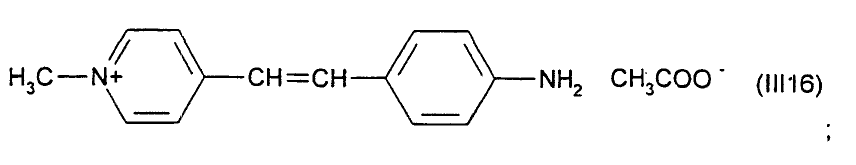 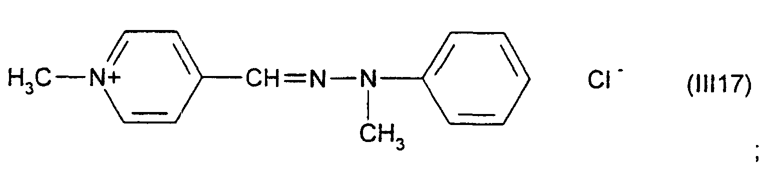 und

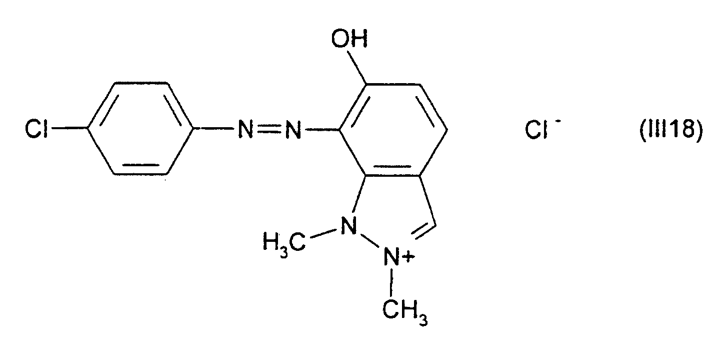

6. Zusammensetzung nach Anspruch 5, **dadurch gekennzeichnet, dass** die kationischen Direktfarbstoffe der Formel (III) den Strukturen (III4), (III5) und (III13) entsprechen.

7. Zusammensetzung nach Anspruch 1, **dadurch gekennzeichnet, dass** die kationischen Direktfarbstoffe der Formel (III') unter den Verbindungen der folgenden Strukturen (III'1) bis (III'3) ausgewählt sind: 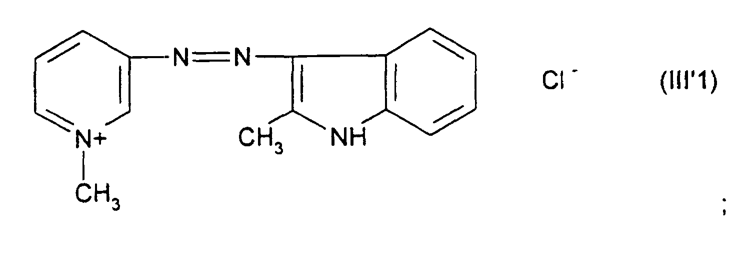 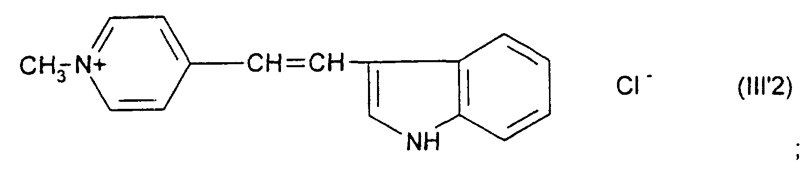 und

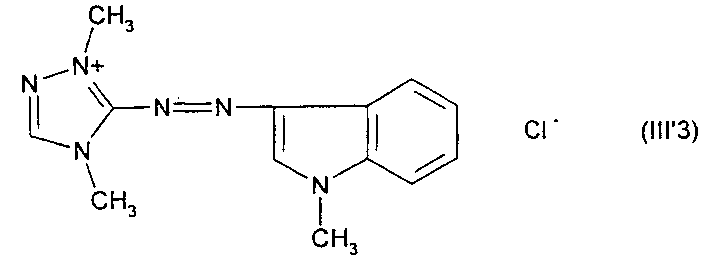

8. Zusammensetzung nach einem der vorhergehenden Ansprüche, **dadurch gekennzeichnet, dass** der oder die kationische(n) Direktfarbstoff(e) der Formeln (I), (II), (III) oder (III') 0,001 bis 10 Gew.-% des Gesamtgewichts der Zusammensetzung ausmachen.

9. Zusammensetzung nach Anspruch 8, **dadurch gekennzeichnet, dass** der oder die kationische(n) Direktfarbstoff(e) der Formeln (I), (II), (III) oder (III') 0,005 bis 5 Gew.-% des Gesamtgewichts der Zusammensetzung ausmachen.

10. Zusammensetzung nach einem der vorhergehenden Ansprüche, **dadurch gekennzeichnet, dass** das substantive Cellulosepolymer ein Polyquaternium 24 ist.

11. Zusammensetzung nach einem der Ansprüche 1 bis 9, **dadurch gekennzeichnet, dass** das substantive Polymer vom Typ der Dimethyldiallylammoniumhalogenid/ (Meth)acrylsäure-Copolymere ein Copolymer von Dimethyldiallylammoniumchlorid und Acrylsäure (80/20, auf das Gewicht bezogen) ist.

12. Zusammensetzung nach einem der Ansprüche 1 bis 9, **dadurch gekennzeichnet, dass** die substantiven Polymere vom. Typ der Homo- und Copolymere eines Methacryloyloxyethyltrimethylammoniumhalogenid unter dem vernetzten Poly(methacryloyloxyethyltrimethylammoniumchlorid)-Homopolymer in 50%iger Dispersion in Mineralöl, dem vernetzten Copolymer von Acrylamid und Methacryloyloxyethyltrimethylammoniumchlorid (20/80 Gew.-%) in 50%iger Dispersion in Mineralöl, dem Methacryloyloxyethyltrimethylammonium / Methacryloyloxyethyldimethylacetylammoniummethosulfat-Copolymer ausgewählt ist.

13. Zusammensetzung nach einem der Ansprüche 1 bis 9, **dadurch gekennzeichnet, dass** die substantiven Polymere vom Typ der Vinylpyrrolidonpolymere mit kationischen Einheiten ausgewählt sind unter:
a) Vinylpyrrolidonpolymeren mit Dimethylaminoethylmethacrylateinheiten;
b) Vinylpyrrolidonpolymeren mit Methacrylamidopropyltrimethylammoniumeinheiten; und
c) Vinylpyrrolidonpolymeren mit Methylvinylimidazoliumeinheiten.

14. Zusammensetzung nach einem der Ansprüche 1 und 10 bis 13, **dadurch gekennzeichnet, dass** das oder die substantive(n) Polymer(e) (ii) 0,01 bis 10 Gew.-% des Gesamtgewichts der Zusammensetzung ausmachen.

15. Zusammensetzung nach Anspruch 14, **dadurch gekennzeichnet, dass** das oder die substantive(n) Polymer(e) (ii) 0,1 bis 5 Gew.-% des Gesamtgewichts der Zusammensetzung ausmachen.

16. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

17. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

18. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

19. Zusammensetzung nach Anspruch 18, **dadurch gekennzeichnet, dass** die Oxidationsbase(n) 0,0005 bis 12 Gew.-% des Gesamtgewichts der Zusammensetzung ausmachen.

20. Zusammensetzung nach Anspruch 19, **dadurch gekennzeichnet, dass** die Oxidationsbase(n) 0,005 bis 6 Gew.-% des Gesamtgewichts der Zusammensetzung ausmachen.

21. Zusammensetzung nach einem der Ansprüche 18 bis 20, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

22. Zusammensetzung nach Anspruch 21, **dadurch gekennzeichnet, dass** der oder die Kuppler 0,0001 bis 10 Gew.-% des Gesamtgewichts der Farbmittelzusammensetzung ausmachen.

23. Zusammensetzung nach Anspruch 22, **dadurch gekennzeichnet, dass** der oder die Kuppler 0,005 bis 5 Gew.-% des Gesamtgewichts der Farbmittelzusammensetzung ausmachen.

24. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidati.onsmittel enthält.

25. Verfahren zum Färben von Keratinfasern und insbesondere menschlichen Keratinfasern, wie dem Haar, **dadurch gekennzeichnet, dass** auf die Fasern mindestens eine Farbm.ittelzusammensetzung nach einem der Ansprüche 1 bis 24 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

26. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 24 während einer Zeitspanne, die zur Bildung der gewünschten Färbung ausreichend ist, aufgebracht wird, ohne dass nochmals gespült wird.

27. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) das in den vorhergehenden Ansprüchen definierte substantive Polymer (ü) enthält.

28. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) das in den vorhergehenden Ansprüchen definierte substantive Polymer (ii) enthält.

29. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 27 oder 28 und eine andere Abteilung die Zusammensetzung (B1) oder (B2) nach Anspruch 27 oder 28 enthält.

## Claims

1. Composition for dyeing keratin fibres, and in particular human keratin fibres such as the hair, this composition containing, in a medium which is suitable for dyeing, (i) at least one cationic direct dye of formulae (I), (II), (III) and (III') below: 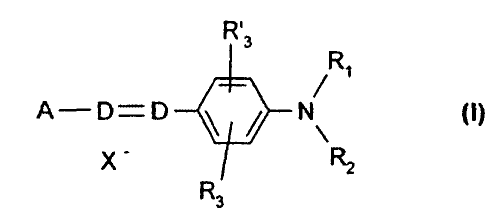 **in which formula (I):**
D represents a nitrogen atom or a -CH group,
R₁ and R₂, which may be identical or different, represent a hydrogen atom; a C₁-C₄ alkyl radical which can be substituted with a -CN, -OH or -NH₂ radical, or form, with a carbon atom of the benzene ring, an optionally oxygenated or nitrogenous heterocycle, which can be substituted with one or more C₁-C₄ alkyl radicals; a 4'-aminophenyl radical,
R₃ and R'₃, which may be identical or different, represent a hydrogen or halogen atom chosen from chlorine, bromine, iodine and fluorine, or a cyano, C₁-C₄ alkoxy or acetyloxy radical,
X⁻ represents an anion preferably chosen from chloride, methyl sulphate and acetate,
A represents a group chosen from structures A1 to A18 below: 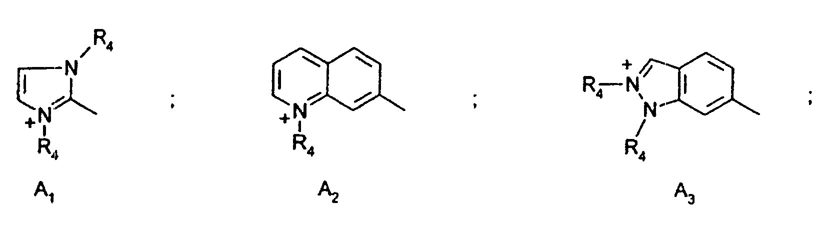 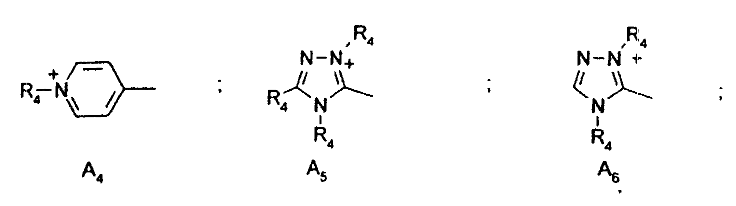 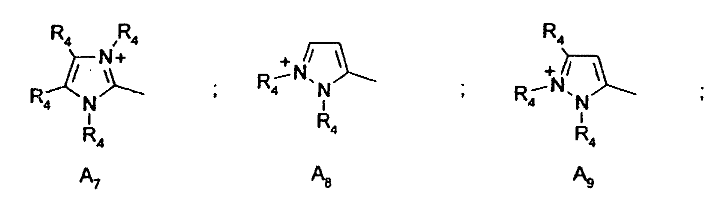 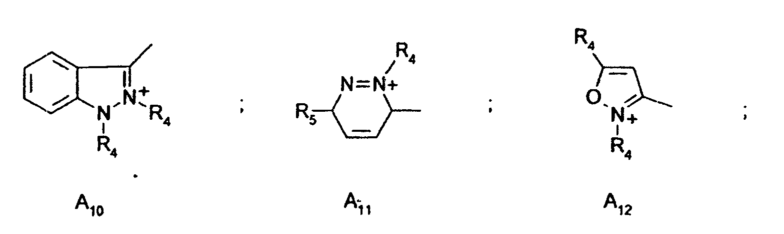 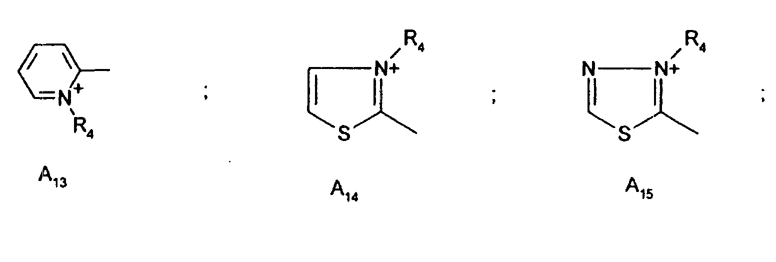 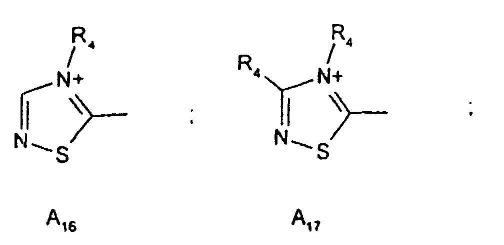 and 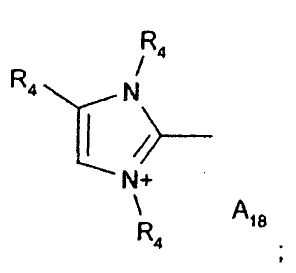 in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom;
and also the following compounds of formula (I): 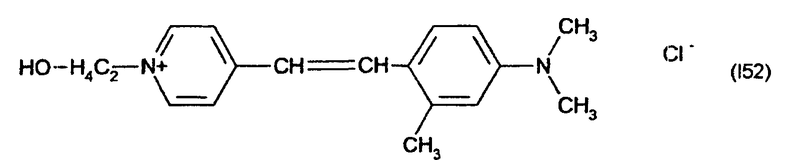 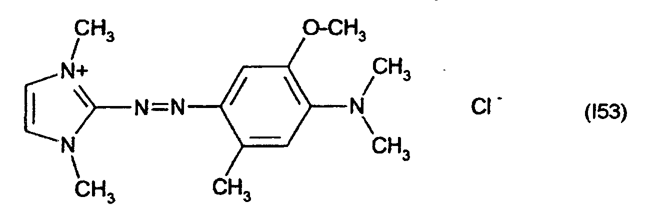 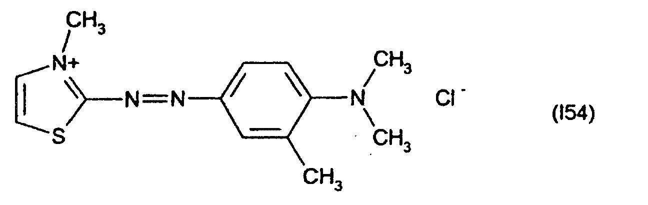 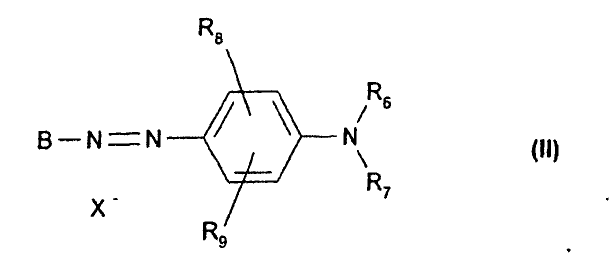 **in which formula (II):**
R₆ represents a hydrogen atom or a C₁-C₄ alkyl radical,
R₇ represents a hydrogen atom, an alkyl radical which can be substituted with a -CN radical or with an amino group, a 4'-aminophenyl radical, or forms, with R₆, an optionally oxygenated and/or nitrogenous heterocycle which can be substituted with a C₁-C₄ alkyl radical,
R₈ and R₉, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methyl sulphate and acetate,
B represents a group chosen from structures B1 to B6 below: 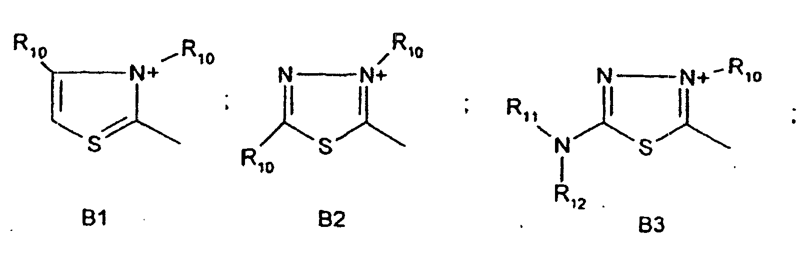 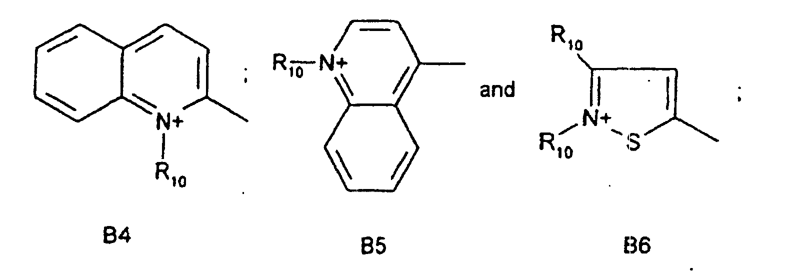 in which R₁₀ represents a C₁-C₄ alkyl radical, R₁₁ and R₁₂, which may be identical or different, represent a hydrogen atom or a C₁-C₄ alkyl radical; 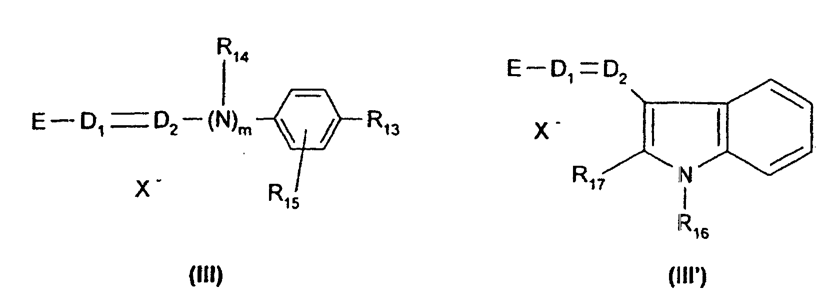
**in which formulae (III) and (III'):**
R₁₃ represents a hydrogen atom, a C₁-C₄ alkoxy radical, a halogen atom such as bromine, chlorine, iodine or fluorine, or an amino radical,
R₁₄ represents a hydrogen atom, a C₁-C₄ alkyl radical, or forms, with a carbon atom of the benzene ring, a heterocycle which is optionally oxygenated and/or substituted with one or more C₁-C₄ alkyl groups,
R₁₅ represents a hydrogen or halogen atom such as bromine, chlorine, iodine or fluorine,
R₁₆ and R₁₇, which may be identical or different, represent a hydrogen atom or a C₁-C₄ alkyl radical,
D₁ and D₂, which may be identical or different, represent a nitrogen atom or a -CH group,
m = 0 or 1,
it being understood that when R₁₃ represents an unsubstituted amino group, then D₁ and D₂ simultaneously represent a -CH group and m = 0,
X⁻ represents an anion preferably chosen from chloride, methyl sulphate and acetate,
E represents a group chosen from structures E1 to E8 below: 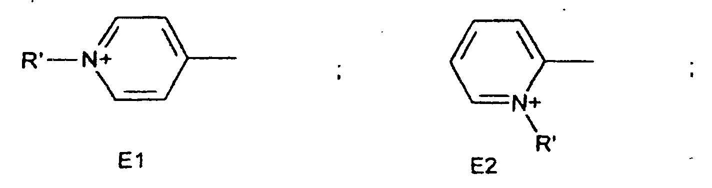 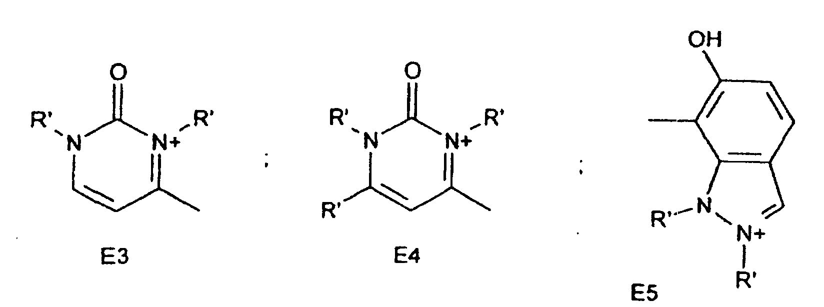 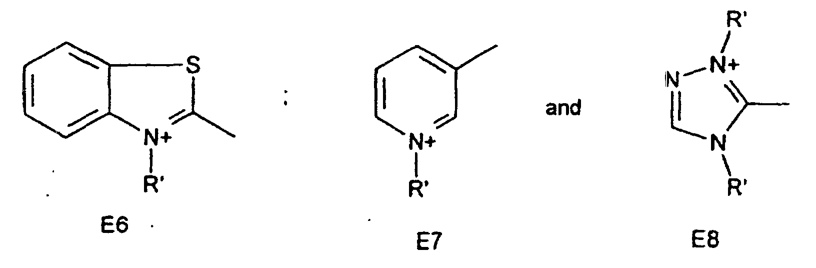 in which R' represents a C₁-C₄ alkyl radical;
when m = 0 and when D₁ represents a nitrogen atom, then E can also denote a group of structure E9 below: 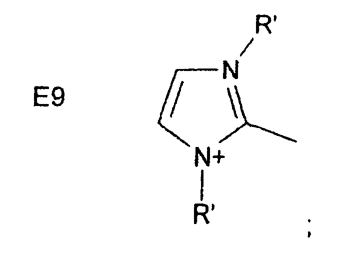 in which R' represents a C₁-C₄ alkyl radical;
the said composition being **characterized in that** it also contains **(ii)** at least one cationic or amphoteric substantive polymer chosen from the group formed by:
1/- cellulosic cationic derivatives with the exception of Polyquaternium 10;
2/- copolymers of dimethyldiallylammonium halide and of (meth)acrylic acid;
3/- methacryloyloxyethyltrimethylammonium halide homopolymers and copolymers;
4/- polyquaternary ammonium polymers chosen from:
- polymers consisting of repeating units corresponding to formula (IV) below: 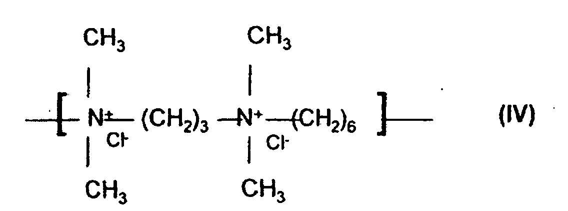
- polymers consisting of repeating units corresponding to formula (V) below: 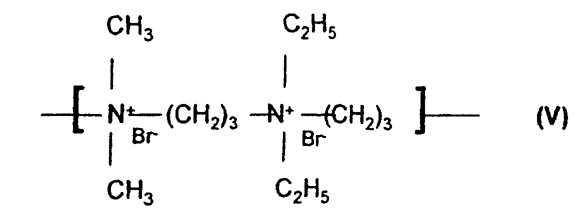
- polymers consisting of repeating units corresponding to formula (VI) below: 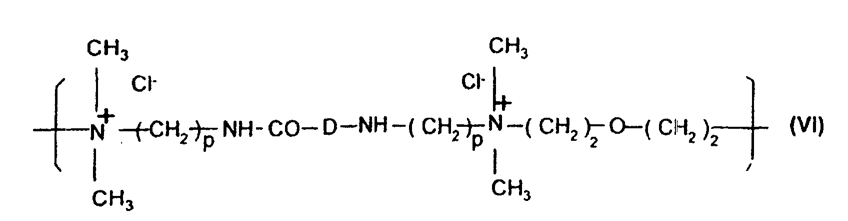 in which p denotes an integer ranging from 1 to 6 approximately, D can be zero or can represent a group - (CH₂)ᵣ-CO- in which r denotes a number equal to 4 or 7;
5/- vinylpyrrolidone copolymers containing cationic units;
6/- mixtures thereof.

2. Composition according to Claim 1, **characterized in that** the cationic direct dyes of formula (I) are chosen from the compounds corresponding to structures (I1) to (I51) below:

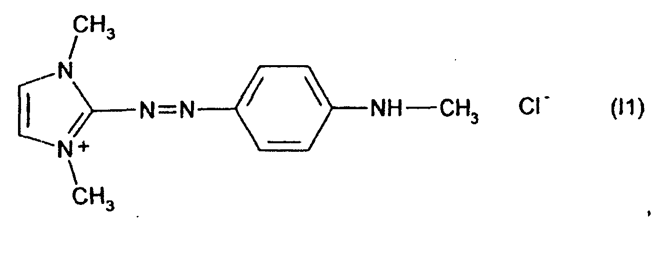

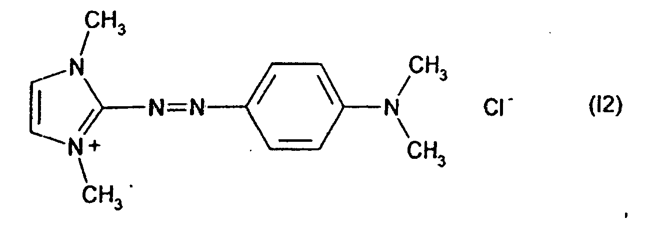

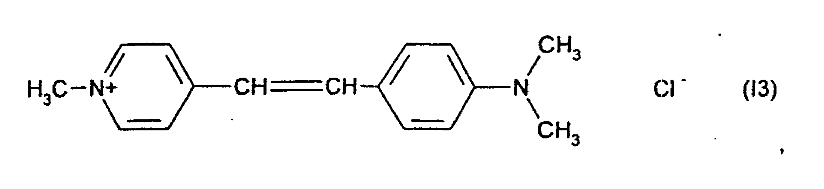

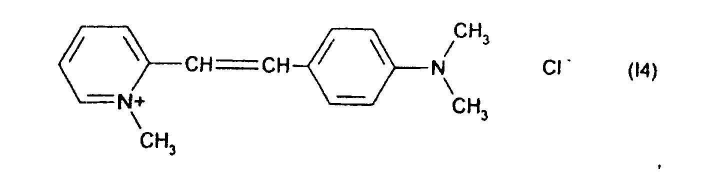

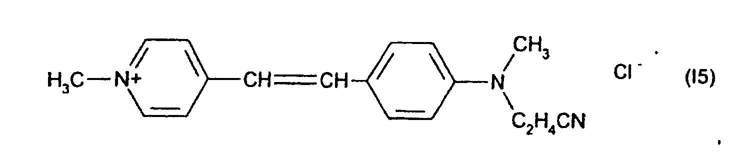

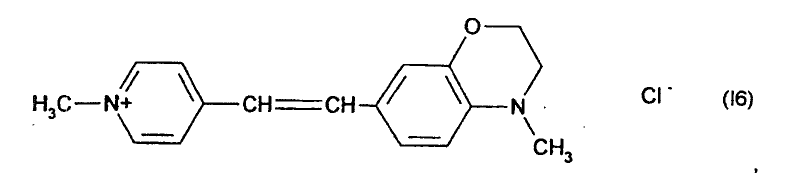

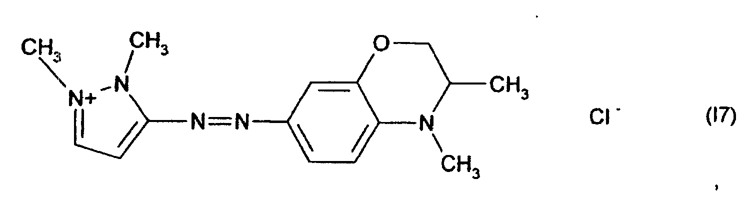

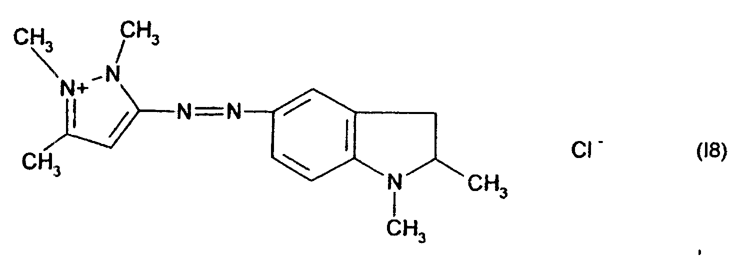

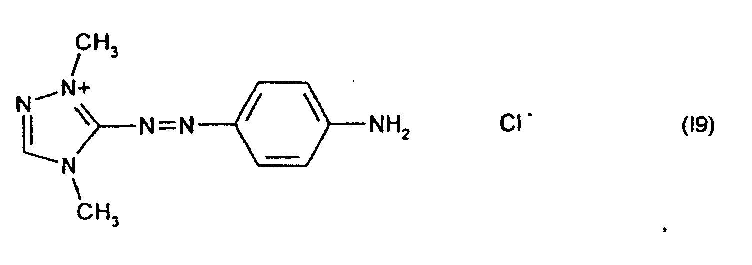

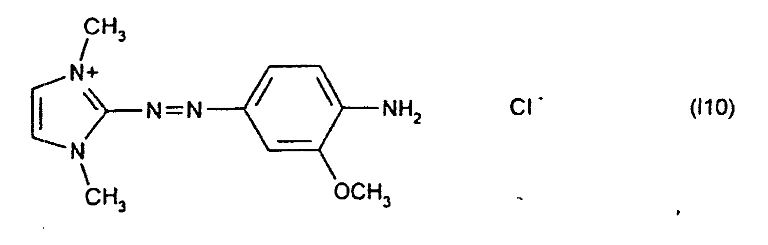

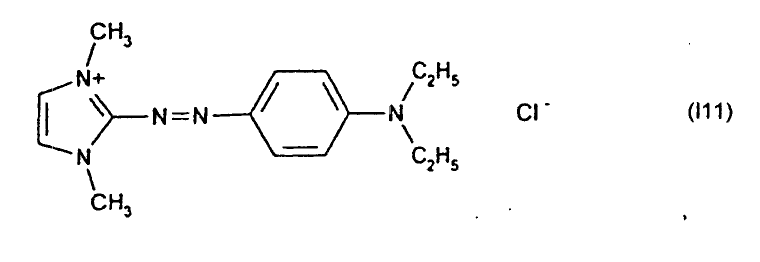

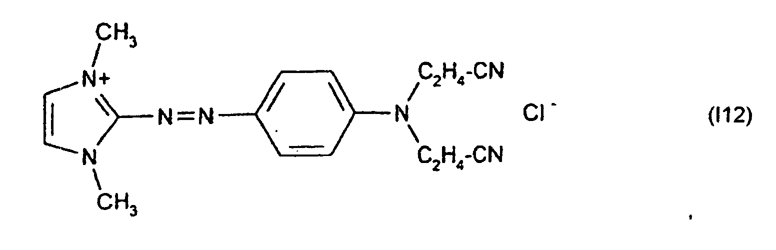

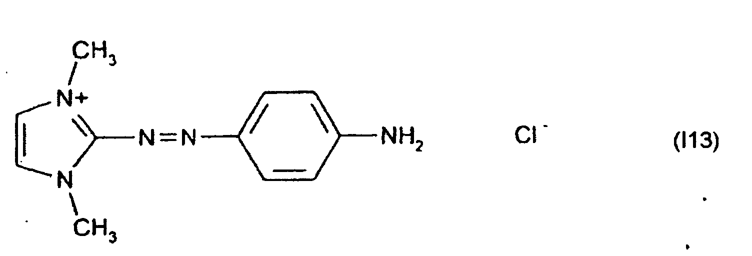

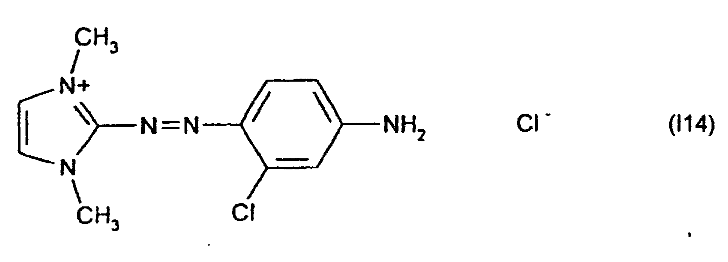

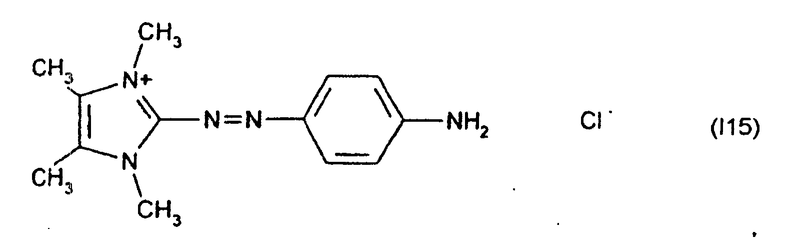

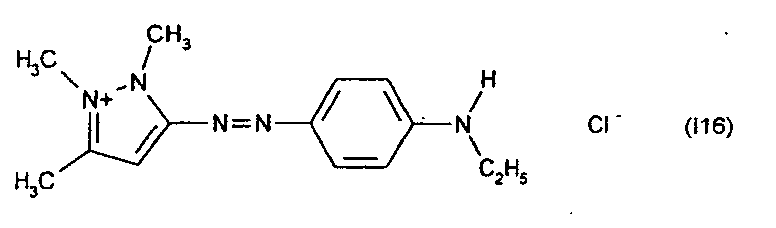

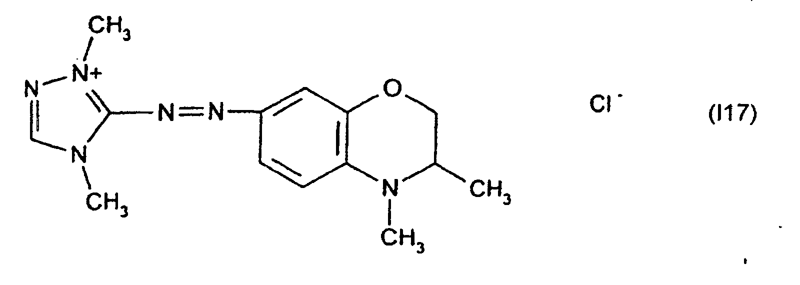

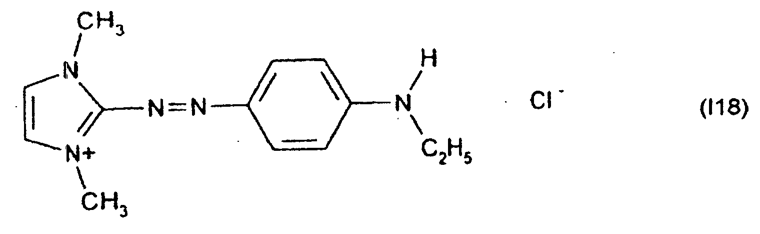

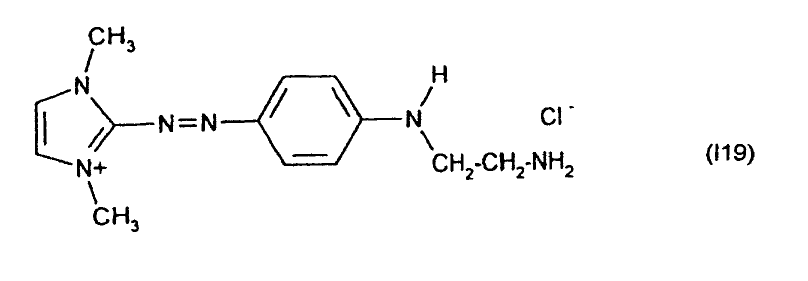

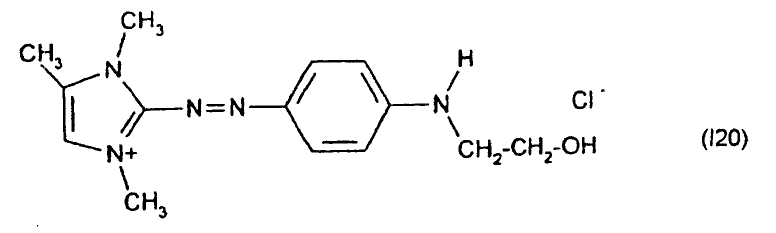

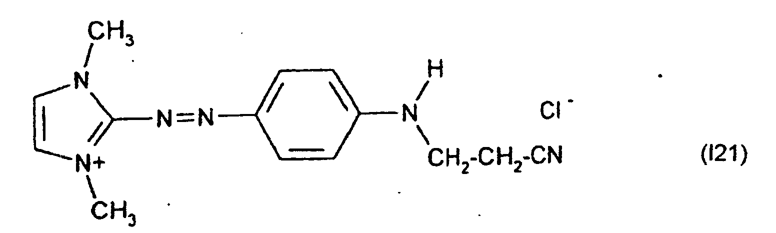

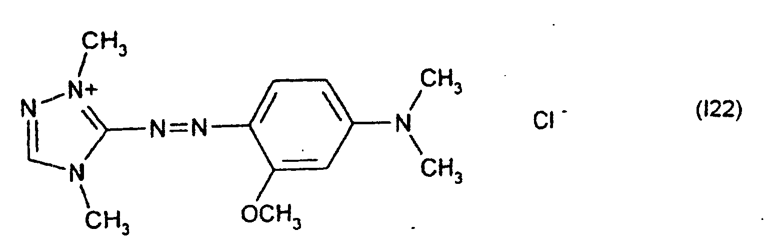

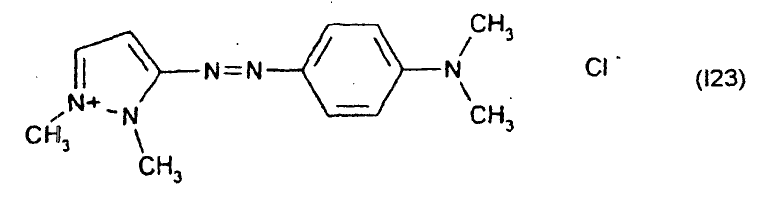

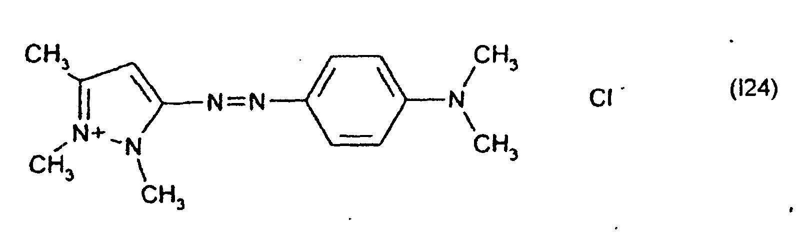

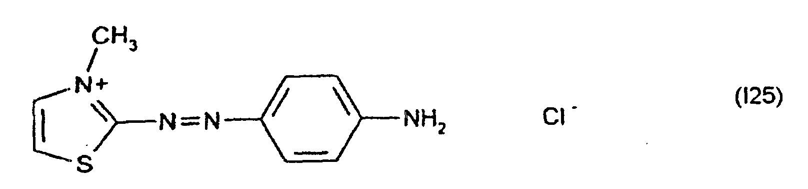

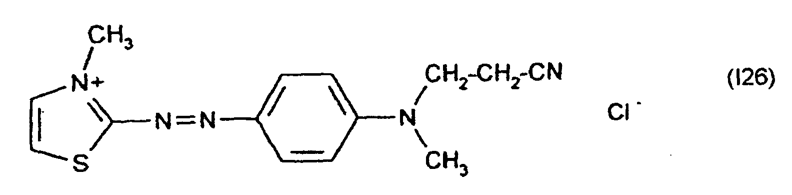

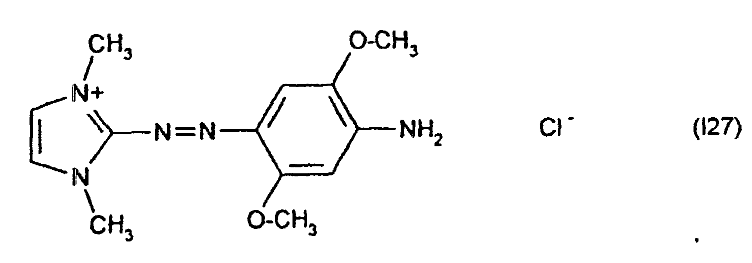

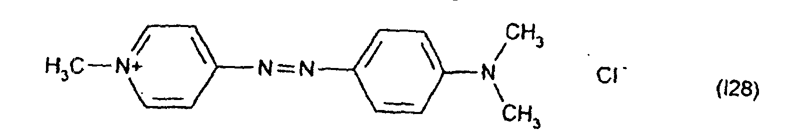

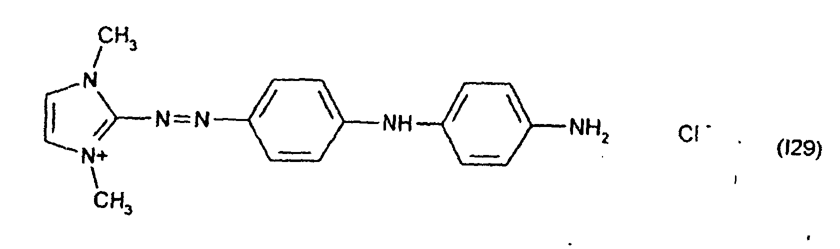

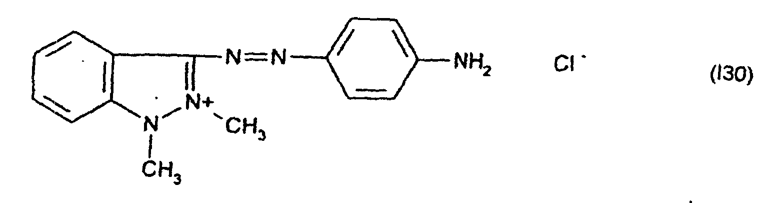

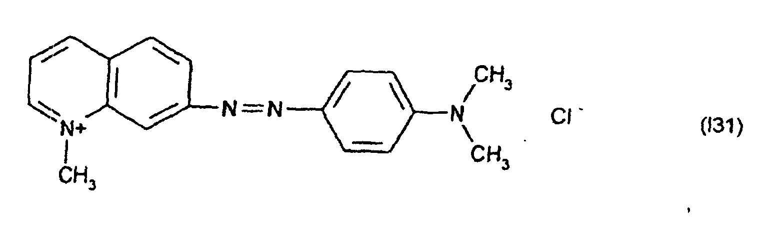

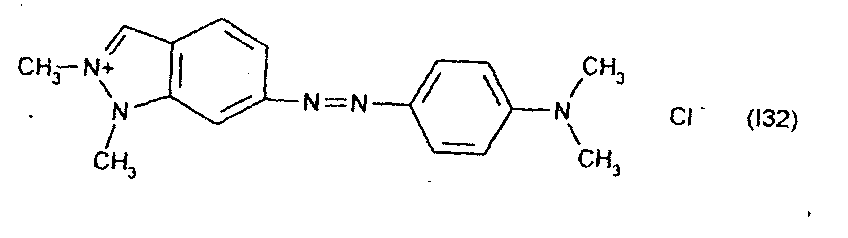

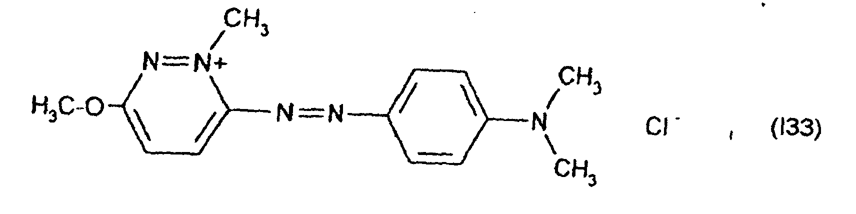

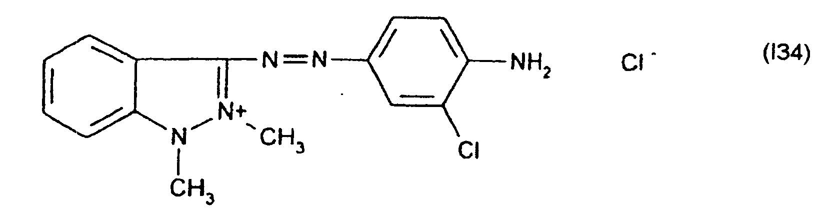

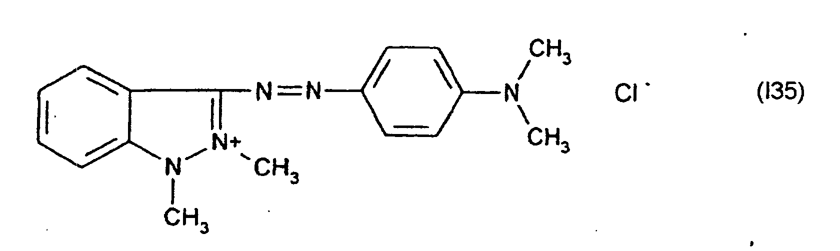

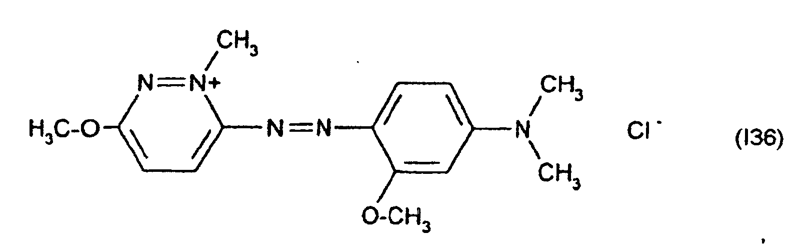

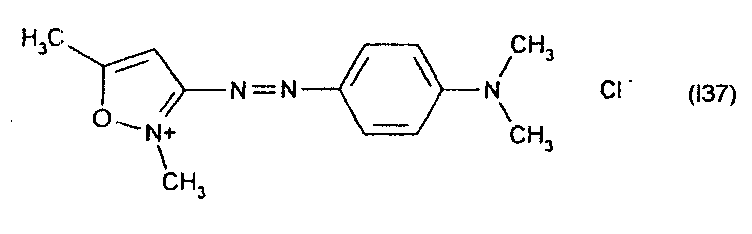

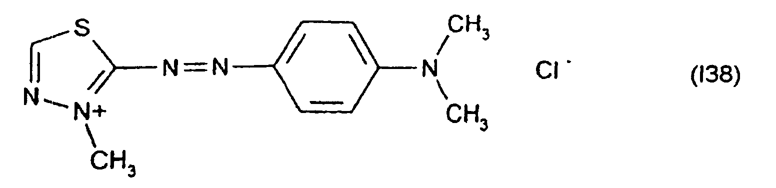

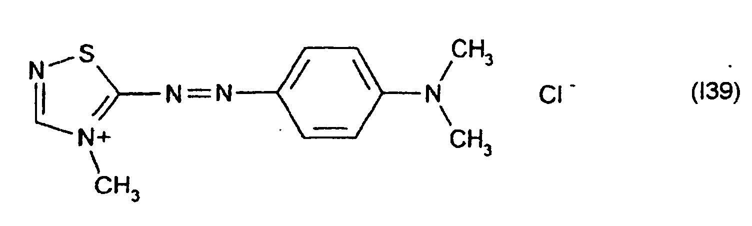

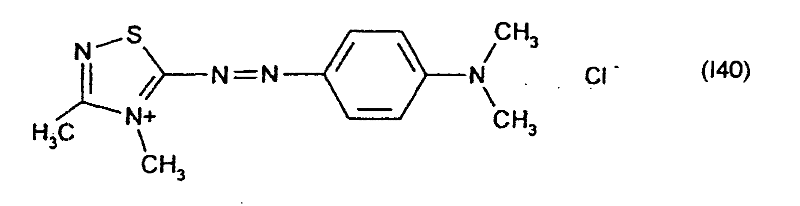

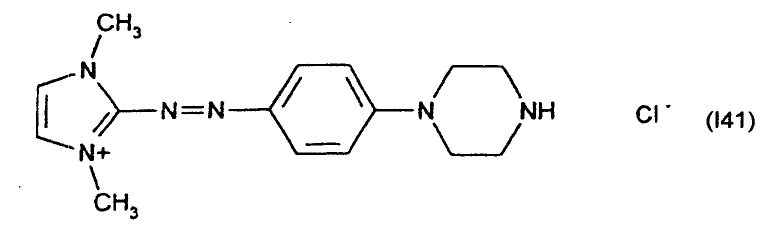

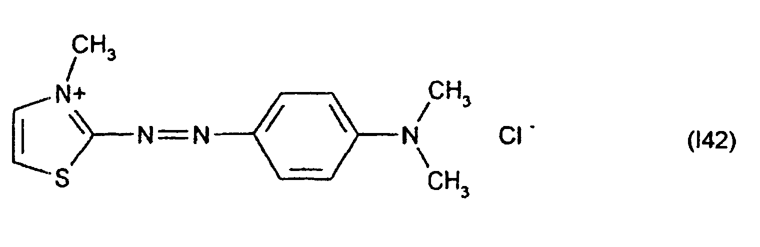

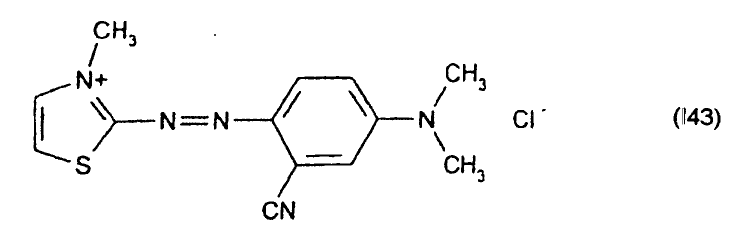

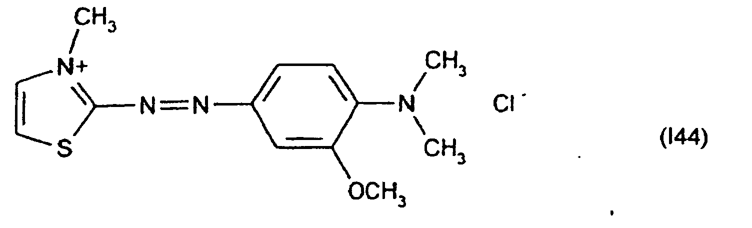

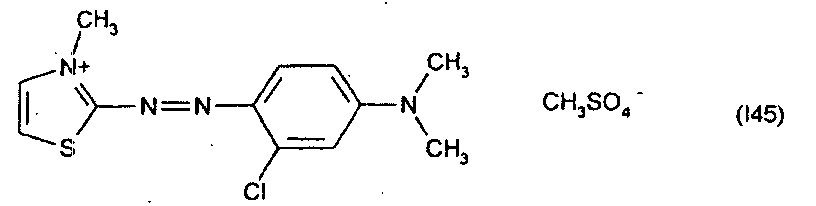

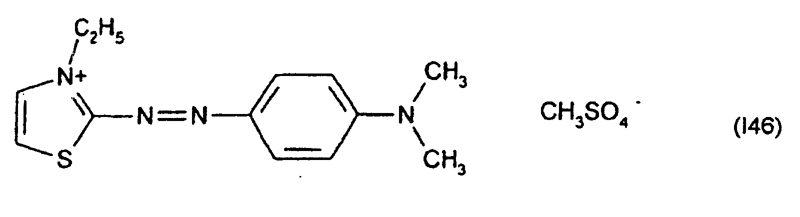

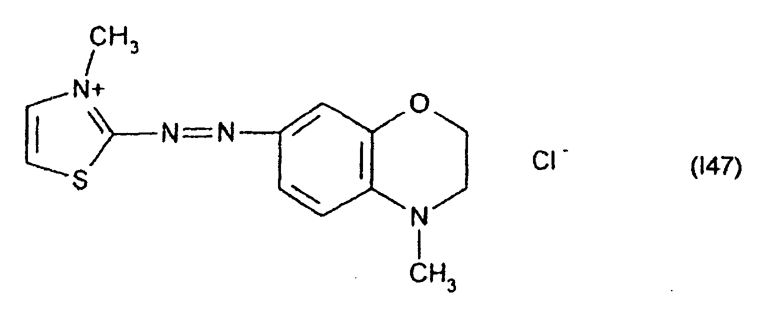

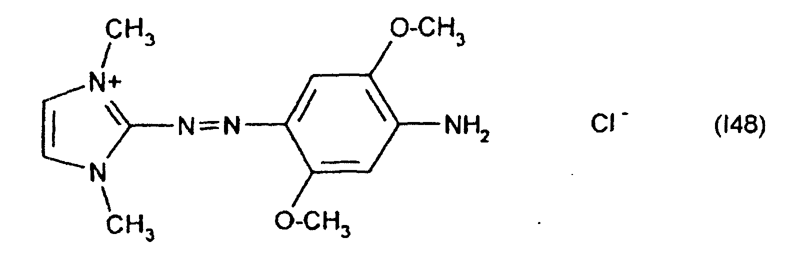

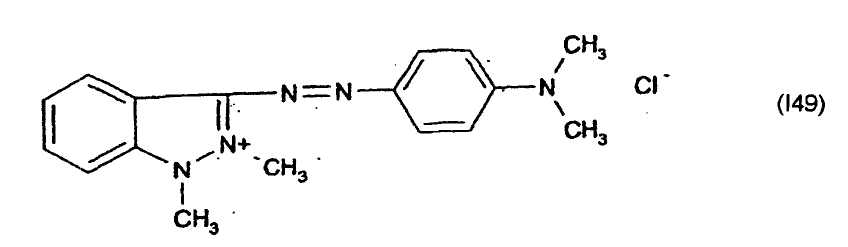

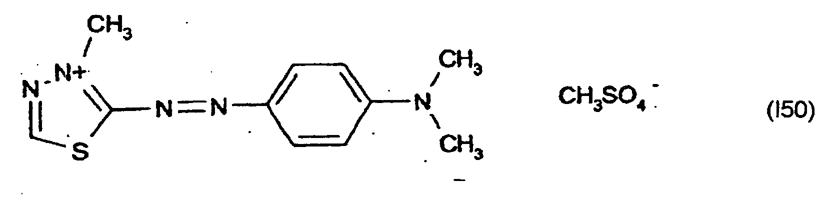

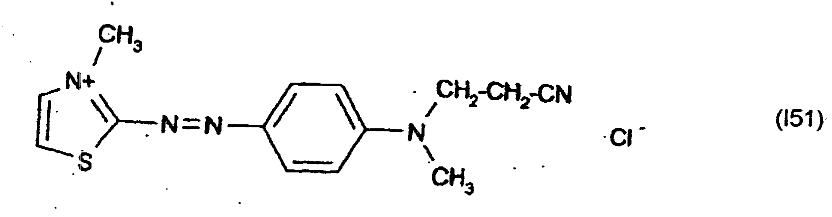

3. Composition according to Claim 2, cha.racterized in that the cationic direct dyes correspond to structures (I1), (I2), (I13) and (I29).

4. Composition according to Claim 1, **characterized in that** the cationic direct dyes of formula (II) are chosen from the compounds corresponding to structures (II1) to (II9) below: 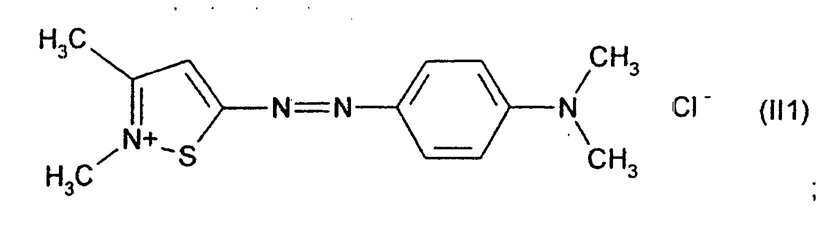 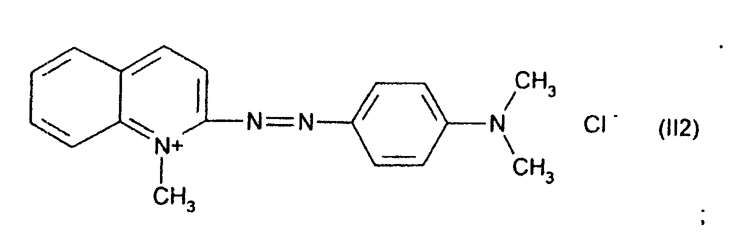 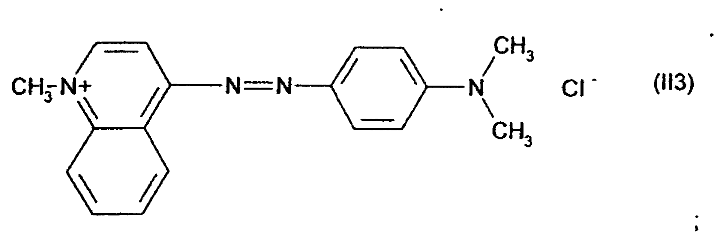 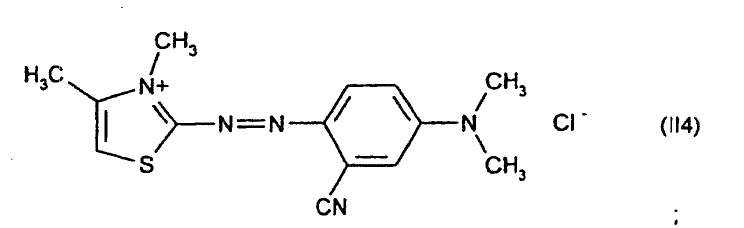 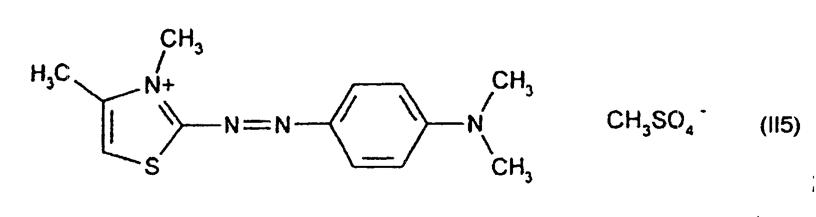 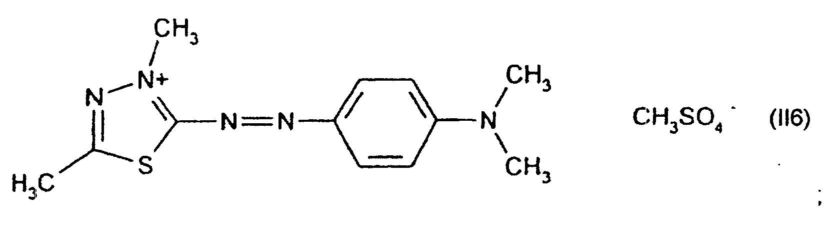 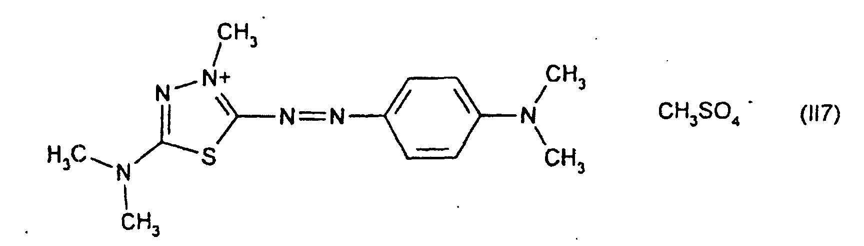 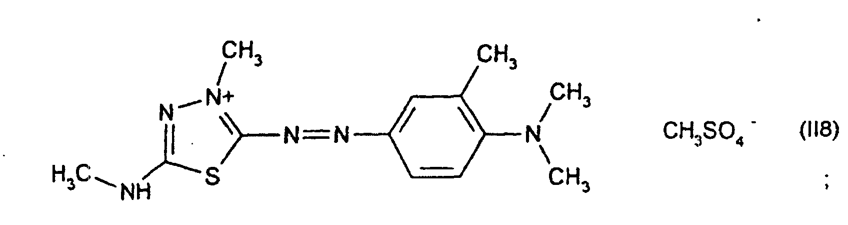 and

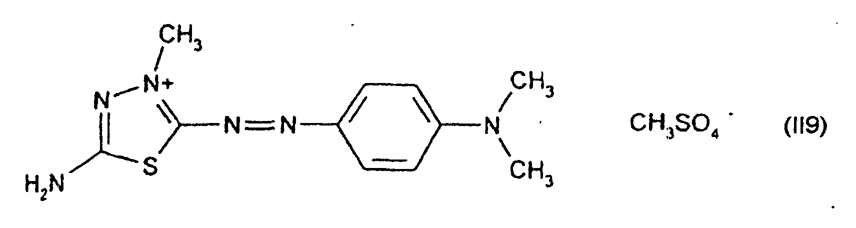

5. Composition according to Claim 1, **characterized in that** the cationic direct dyes of formula (III) are chosen from the compounds corresponding to structures (III1) to (III18) below: 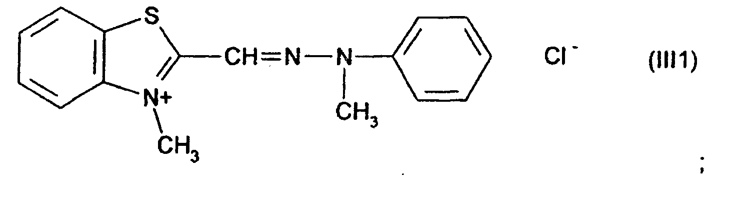 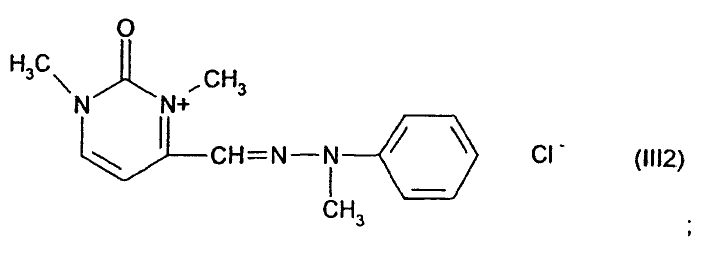 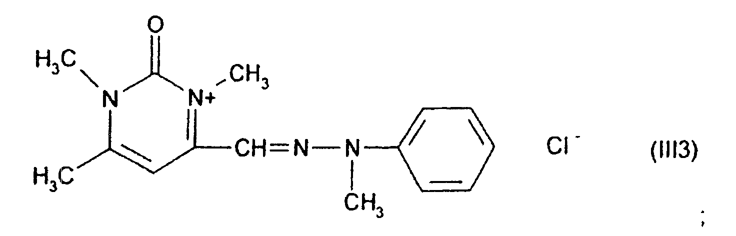 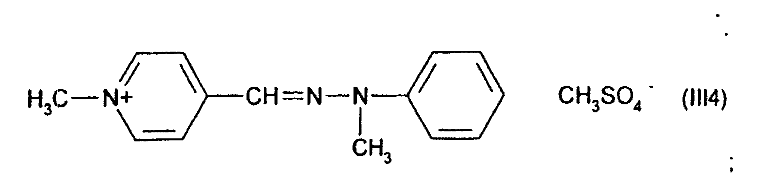 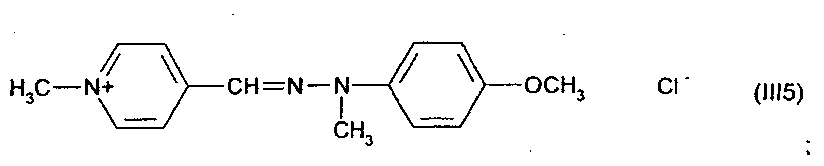 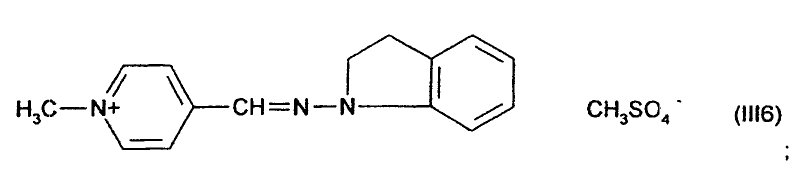 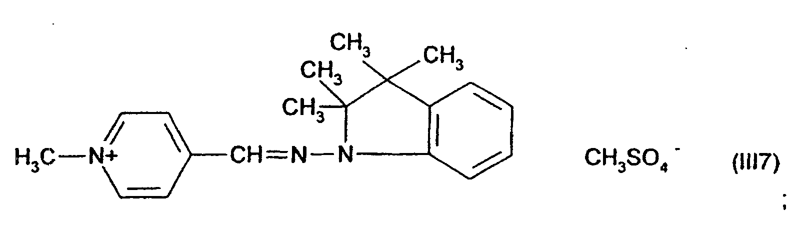 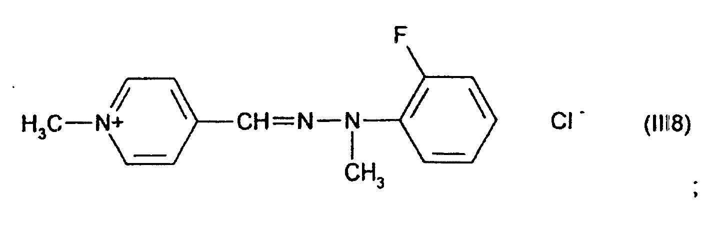 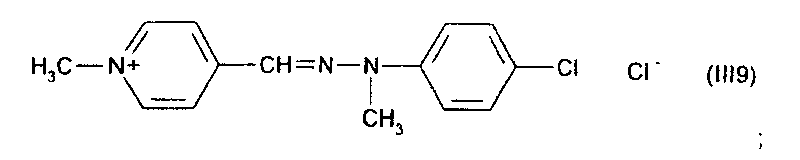 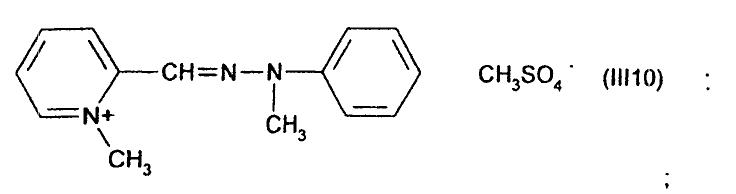 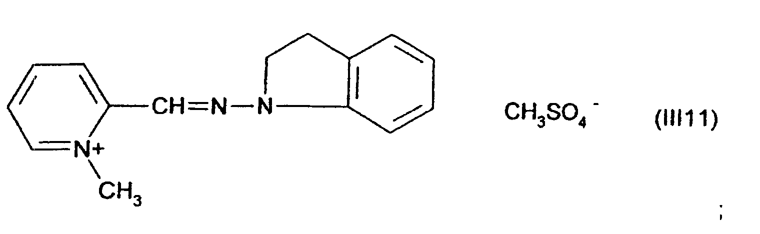 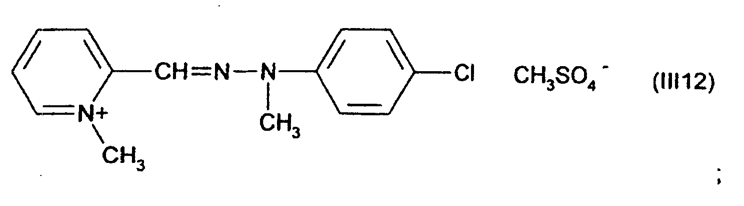 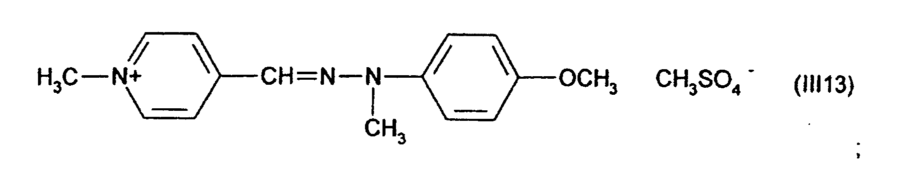 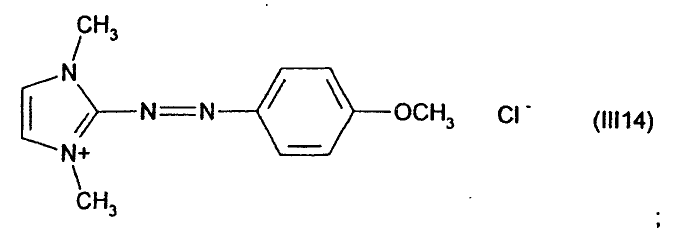 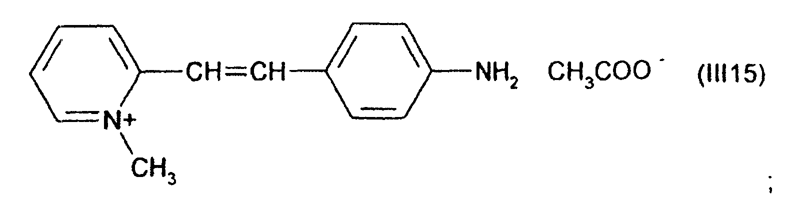 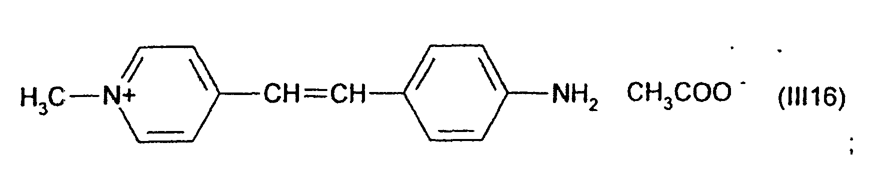 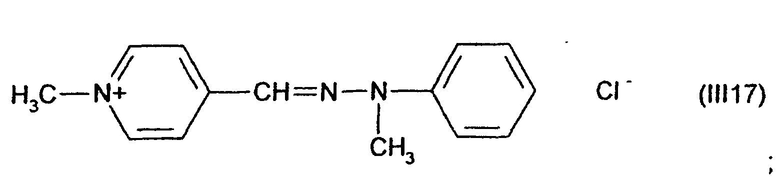 and

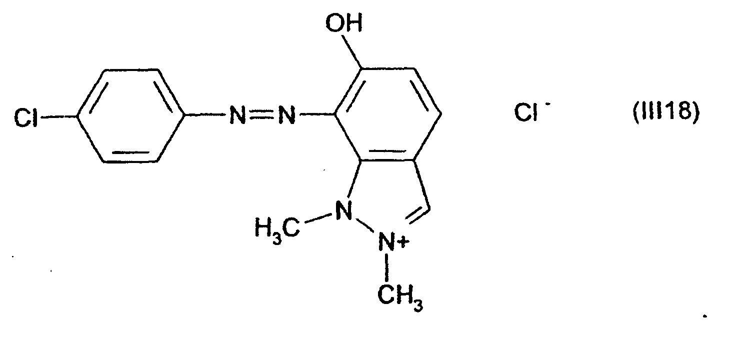

6. Composition according to Claim 5, **characterized in that** the cationic direct dyes of formula (III) are chosen from the compounds corresponding to structures (III4), (III5) and (III13).

7. Composition according to Claim 1, **characterized in that** the cationic direct dyes of formula (III') are chosen from the compounds corresponding to structures (III'1) to (III'3) below: 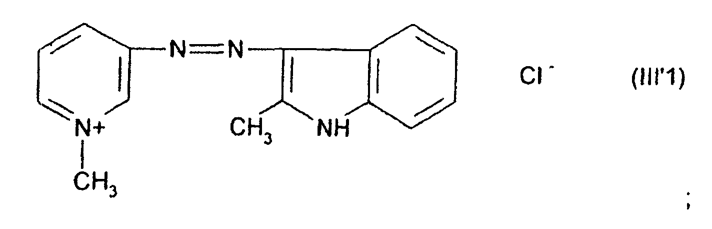 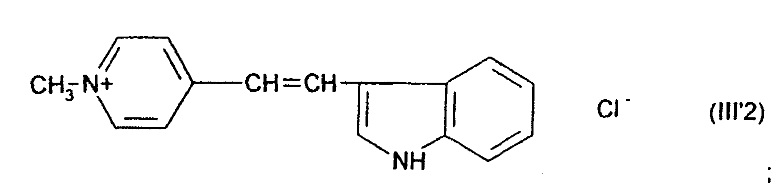 and

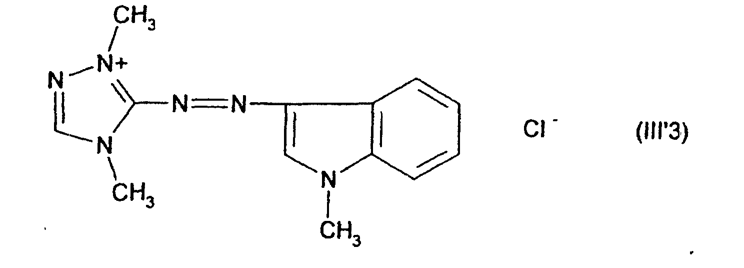

8. Composition according to any one of the preceding claims, **characterized in that** the cationic direct dye(s) of formulae (I), (II), (III) or (III') represent(s) from 0.001 to 10% by weight relative to the total weight of the composition.

9. Composition according to Claim 8, **characterized in that** the cationic direct dye(s) of formulae (I), (II), (III) or (III') represent(s) from 0.005 to 5% by weight relative to the total weight of the composition.

10. Composition according to any one of the preceding claims, **characterized in that** the cellulosic substantive polymer is a Polyquaternium 24.

11. Composition according to any one of Claims 1 to 9, **characterized in that** the substantive polymer of the copolymer of dimethyldiallylammonium halide and of (meth)acrylic acid type is a copolymer of dimethyldiallylammonium chloride and of acrylic acid (80/20 by weight).

12. Composition according to any one of Claims 1 to 9, **characterized in that** the substantive polymers of the methacryloyloxyethyltrimethylammonium halide homopolymer and copolymer type are chosen from crosslinked poly(methacryloyloxyethyltrimethylammonium chloride) homopolymer, as a 50% dispersion in mineral oil, the crosslinked copolymer of acrylamide and of methacryloyloxyethyltrimethylammonium chloride (20/80 by weight), as a 50% dispersion in mineral oil, and the methosulphate of the copolymer of methacryloyloxyethyltrimethylammonium and of methacryloyloxyethyldimethylacetylammonium.

13. Composition according to any one of Claims 1 to 9, **characterized in that** the substantive polymers of the vinylpyrrolidone polymer type containing cationic units are chosen from:
a) vinylpyrrolidone polymers containing dimethylaminoethyl methacrylate units;
b) vinylpyrrolidone polymers containing methacrylamidopropyltrimethylammonium units;
c) vinylpyrrolidone polymers containing methylvinylimidazolium units.

14. Composition according to any one of Claims 1 and 10 to 13, **characterized in that** the substantive polymer(s) (ii) represent(s) from 0.01 to 10% by weight relative to the total weight of the composition.

15. Composition according to Claim 14, **characterized in that** the substantive polymer(s) (ii) represent(s) from 0.1 to 5% by weight relative to the total weight of the composition.

16. Composition according to any one of the preceding claims, **characterized in that** the medium which is suitable for dyeing (or support) consists of water or of a mixture of water and at least one organic solvent.

17. Composition according to any one of the preceding claims, **characterized in that** it has a pH of between 2 and 11, and preferably between 5 and 10.

18. Composition according to any one of the preceding claims, **characterized in that** it is intended for oxidation dyeing and **in that** it contains one or more oxidation bases chosen from para-phenylenediamines, bis(phenyl)alkylenediamines, para-aminophenols, ortho-aminophenols and heterocyclic bases.

19. Composition according to Claim 18, **characterized in that** the oxidation base(s) represent(s) from 0.0005 to 12% by weight relative to the total weight of the dye composition.

20. Composition according to Claim 19, **characterized in that** the oxidation base(s) represent(s) from 0.005 to 6% by weight relative to the total weight of the dye composition.

21. Composition according to any one of Claims 18 to 20, **characterized in that** it contains one or more couplers chosen from meta-phenylenediamines, meta-aminophenols, meta-diphenols and heterocyclic couplers.

22. Composition according to Claim 21, **characterized in that** the coupler(s) represent(s) from 0.0001 to 10% by weight relative to the total weight of the dye composition.

23. Composition according to Claim 22, **characterized in that** the coupler(s) represent(s) from 0.005 to 5% by weight relative to the total weight of the dye composition.

24. Composition according to any one of the preceding claims, **characterized in that** it is intended for oxidation dyeing or for lightening direct dyeing and **in that** it contains at least one oxidizing agent.

25. Process for dyeing keratin fibres, and in particular human keratin fibres such as the hair, **characterized in that** at least one dye composition as defined in any one of Claims 1 to 24 is applied to the fibres, for a period which is sufficient to develop the desired coloration, after which the fibres are rinsed, optionally washed with shampoo, rinsed again and dried.

26. Process for dyeing keratin fibres, and in particular human keratin fibres such as the hair, **characterized in that** at least one dye composition as defined in any one of Claims 1 to 24 is applied to the fibres, for a period which is sufficient to develop the desired coloration, without final rinsing.

27. Process for dyeing keratin fibres, and in particular human keratin fibres such as the hair, **characterized in that** it includes a first step which consists in separately storing, on the one hand, a composition (A1) comprising, in a medium which is suitable for dyeing, at least one cationic direct dye (i) as defined in the preceding claims and at least one oxidation base, and, on the other hand, a composition (B1) containing, in a medium which is suitable for dyeing, at least one oxidizing agent, and then in mixing them together at the time of use, before applying this mixture to the keratin fibres, the composition (A1) or the composition (B1) containing the substantive polymer (ii) as defined in the preceding claims.

28. Process for dyeing keratin fibres, and in particular human keratin fibres such as the hair, **characterized in that** it includes a first step which consists in separately storing, on the one hand, a composition (A2) comprising, in a medium which is suitable for dyeing, at least one cationic direct dye (i) as defined in the preceding claims and, on the other hand, a composition (B2) containing, in a medium which is suitable for dyeing, at least one oxidizing agent, and then in mixing them together at the time of use, before applying this mixture to the keratin fibres, the composition (A2) or the composition (B2) containing the substantive polymer (ii) as defined in the preceding claims.

29. Multi-compartment dyeing device or multicompartment dyeing kit, **characterized in that** a first compartment contains composition (A1) or (A2) as defined in Claim 27 or 28, and a second compartment contains composition (B1) or (B2) as defined in Claim 27 or 28.
